# EUROPEAN PATENT SPECIFICATION

(11) **EP 4 408 978 B1**
(45) Date of publication and mention of the grant of the patent: **15.04.2026**
(21) Application number: 22741811.8
(22) Date of filing: 25.07.2022
(51) Int. Cl.: C12N 5/075, A61K 38/17

(54) **STABILISING THE HUMAN SPINDLE BY KIFC1/HSET**
STABILISIERUNG DER MENSCHLICHEN SPINDEL DURCH KIFC1/HSET
STABILISATION DU FUSEAU HUMAIN PAR KIFC1/HSET

(30) Priority: 27.09.2021 EP 21199120
(43) Date of publication of application: 07.08.2024
(73) Proprietor: Max-Planck-Gesellschaft zur Förderung der Wissenschaften e.V., 80539 München (DE)
(72) Inventor: SCHUH, Melina, 37085 Göttingen (DE); SO, Chun, 37073 Göttingen (DE)
(74) Representative: Wichmann, Hendrik
(86) International application number: PCT/EP2022/070801
(87) International publication number: WO 2023/046335

(56) References cited:
- WO-A1-2014/064187
- US-A1- 2010 242 125
- RAWE ET AL: "Mammalian Oocyte Maturation and Microtubule-Associated Proteins Dynamics", FERTILITY AND STERILITY, ELSEVIER, AMSTERDAM, NL, vol. 84, 1 September 2005 (2005-09-01), pages S143, XP005085022, ISSN: 0015-0282, DOI: 10.1016/J.FERTNSTERT.2005.07.349
- BENNABI ISMA ET AL: "Shifting meiotic to mitotic spindle assembly in oocytes disrupts chromosome alignment", vol. 19, no. 2, 12 January 2018 (2018-01-12), GB, pages 368 - 381, XP055906742, ISSN: 1469-221X, Retrieved from the Internet <URL:https://onlinelibrary.wiley.com/doi/full-xml/10.15252/embr.201745225> DOI: 10.15252/embr.201745225
- HALL V.J. ET AL: "Developmental competence of human in vitro aged oocytes as host cells for nuclear transfer", vol. 22, no. 1, 1 January 2007 (2007-01-01), GB, pages 52 - 62, XP055902440, ISSN: 0268-1161, Retrieved from the Internet <URL:https://academic.oup.com/humrep/article-pdf/22/1/52/9682956/del345.pdf> DOI: 10.1093/humrep/del345

## Description

### Background of the Invention

Aneuploidy in human eggs is a leading cause of aberrant embryonic development, resulting in miscarriages and genetic disorders such as Down syndrome. It is estimated that around 25 - 50% of human eggs are aneuploid, and the aneuploidy rate increases exponentially with maternal age (1). Until recently, it has remained unclear why human eggs are so prone to aneuploidy, even in young women. The inventors' previous study of meiosis and chromosome segregation in live human oocytes for the first time revealed that human oocytes often assemble highly unstable spindles during meiosis I (2). Bipolar spindles drive chromosome segregation in mitosis and meiosis, and their correct assembly is a prerequisite for accurate chromosome segregation. In human oocytes, the two spindle poles frequently widen or fragment (2), forming apolar or multipolar spindles (2, 3). These unstable spindles often misalign and missegregate chromosomes (2), and have been linked to tri-directional division at anaphase I (3). However, the cause of spindle instability in human oocytes is still unknown. More recently, it has been shown that not only triploid human fertilized eggs (zygotes) (4, 5), but also the diploid ones are prone to assemble multipolar spindles and undergo multipolar divisions (6, 7). Therefore, identifying the cause of spindle instability may lead to therapeutic strategies that not only reduce aneuploidy in human eggs, but also improve the overall outcomes of assisted reproductive technology.

Spindle instability is extremely rare in normal mitotic cells, whose aneuploidy rates are much lower. Mitotic cells assemble spindles differently from those in oocytes. In mitosis, two centrosomes act as the main microtubule-organizing centers (MTOCs) and form the two spindle poles (8, 9). Canonical centrosomes consist of a pair of centrioles surrounded by pericentriolar material, which contains proteins responsible for microtubule nucleation and anchoring (10). The number of centrosomes is tightly regulated in mitotic cells. Normally, the centrosome duplicates once per cell cycle, ensuring the formation of only two centrosomes, and thereby the assembly of bipolar spindles (11). However, when centrosomes are hyperamplified or centrosome integrity is lost, cells are predisposed to assemble multipolar spindles (12).

In oocytes of most species, including mammals, centrioles degenerate during oocyte development, resulting in the loss of centrosomes (13-15). How acentrosomal spindle poles are organized has mostly been studied in non-mammalian oocytes and in mouse oocytes. Early in vitro work in Xenopus egg extracts showed that the minus-end-directed motor dynein organizes the poles by crosslinking and sliding microtubules in spindles assembled on chromatin beads (16, 17). In C. elegans oocytes, the katanin MEI-1 recruits the spindle pole-associated protein ASPM-1 (18), which may further recruit dynein and/or NUMA-related LIN-5 for pole focusing (19, 20). In Drosophila oocytes, Asp and NUMA-related Mud are dispensable for spindle pole organization (*21*, *22*), but the minus-end-directed motor kinesin-14 Ncd bundles microtubules for pole focusing (*23*, *24*). In mouse oocytes, canonical centrosomes are functionally replaced by acentriolar MTOCs (aMTOCs; microtubule-organizing center) (*25*). aMTOCs contain many of the components of pericentriolar material (*26*), cluster as a ring at the two spindle poles and represent major sites of microtubule nucleation and anchoring in mouse oocytes (*25, 27-30*).

However, the mechanism of spindle pole organization in non-rodent mammalian oocytes including humans, cows and pigs remains unclear. In these species, the pericentriolar material does not aggregate into distinct aMTOCs but remains dispersed in the cytoplasm throughout meiosis (*2*, *31*). Although earlier studies of these oocytes reported the spindle pole localization of a few proteins such as γ-tubulin and NUMA (*2, 32-35*), how these oocytes assemble their spindle poles and control their spindle polarity without centrosomes or aMTOCs remains poorly understood.

US 2010/242125 A1 is concerned with somatic nuclear transfer in animals in order to change mitotic spindle defects. The result of somatic nuclei transfer is fundamentally different from assisted reproductive technologies such as in vitro fertilization or intracytoplasmic sperm injection. Whereas somatic nuclear transfer results in a genetically identical human being (i.e. cloning), assisted reproductive technologies result in a genetically different human being. Thus, somatic nuclear transfer for human is not considered as fertility treatment and is thus not an option for assisted reproductive technology.

Rawe et al. 2005 entitled "Mammalian oocyte maturation and microtubule-associated Proteins dynamics" is a conference abstract concerned with preliminary observations regarding the presence of proteins in human and bovine oocytes.

WO 2014/064187A1 is a patent application pertaining to treating proliferative diseases, wherein protein and mRNA sequences of KIFC1/HSET are listed.

Therefore, there is a need in the art to reveal the mechanism behind spindle assembly in humans and, in particular, to improve human spindle stability during meiosis in human oocytes and mitosis in human zygotes. Deciphering said mechanism and improving spindle stability would be a great advance for assisted reproductive technology.

This invention shows that human oocytes have unstable spindles because they lack KIFC1/HSET, which stabilizes the spindles in other oocytes such as mouse oocytes and in cancer cells. The presented data demonstrates that the absence of KIFC1/HSET, that stabilizes the spindles in other mammalian oocytes such as mouse oocytes, is a major contributor to spindle instability in human oocytes. Thus, the introduction of KIFC1/HSET into human oocytes and/or human zygotes stabilizes the spindles, and thereby the human oocytes and/or zygotes have a lower probability of suffering from aneuploidy. The inventors have surprisingly demonstrated that human oocytes lack KIFC1/HSET and thus the probability of unstable human spindles can be lowered by introducing human KIFC1/HSET into human oocytes. The inventors have shown that unstable spindles in human oocytes can be stabilized by the introduction of human KIFC1/HSET.

### Summary of the Invention

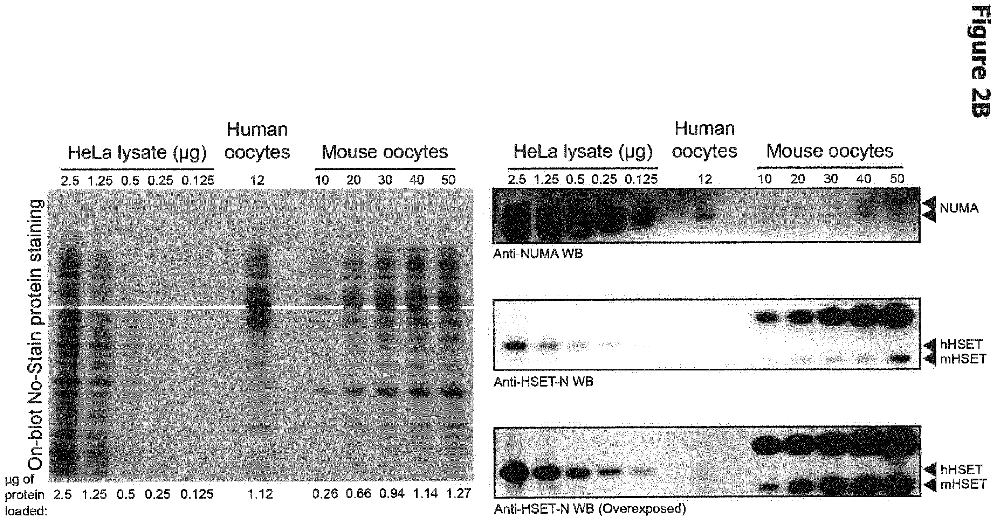

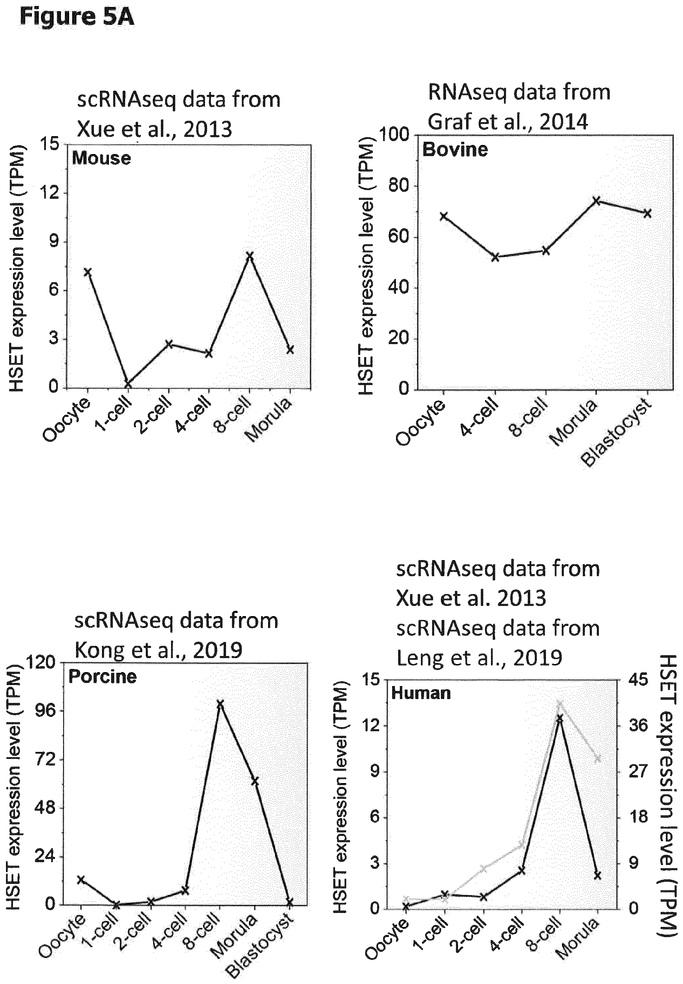

It has been repeatedly demonstrated in the art that mouse oocytes express KIFC1/HSET (*83*, *84*). Thus, it has also been expected that human oocytes also express KIFC1/HSET. However, it has been unexpectedly demonstrated that human oocytes lack KIFC1/HSET (see **Fig.** 2B). To determine whether KIFC1/HSET is expressed in human oocytes, the inventors examined data from previous proteomics studies on mouse and human oocytes (*77, 78*)*.* The inventors noticed that KIFC1/HSET could only be detected in the mouse dataset. However, due to the differences in proteome coverage for these two studies, the inventors additionally analyzed KIFC1/HSET expression using data from previous RNA-seq studies of mammalian oocytes and embryos (79-82). Mouse, bovine, and porcine oocytes had a prominent pool of maternal KIFC1/HSET mRNA, which was depleted upon fertilization, and embryonic KIFC1/HSET mRNA was expressed from the 2- to 4-cell stage onwards (**Fig.** 5A). In contrast, KIFC1/HSET mRNA was barely detectable in human oocytes and zygotes, but was readily expressed from the 2- or 4-cell stage onwards in embryos based on the data presented in references 79-82 (**Fig.** 5A).

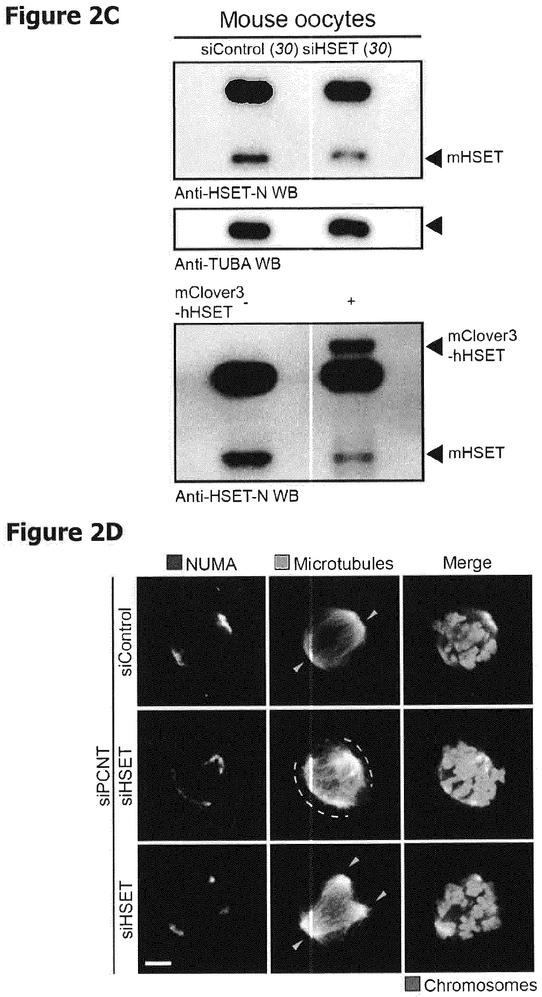

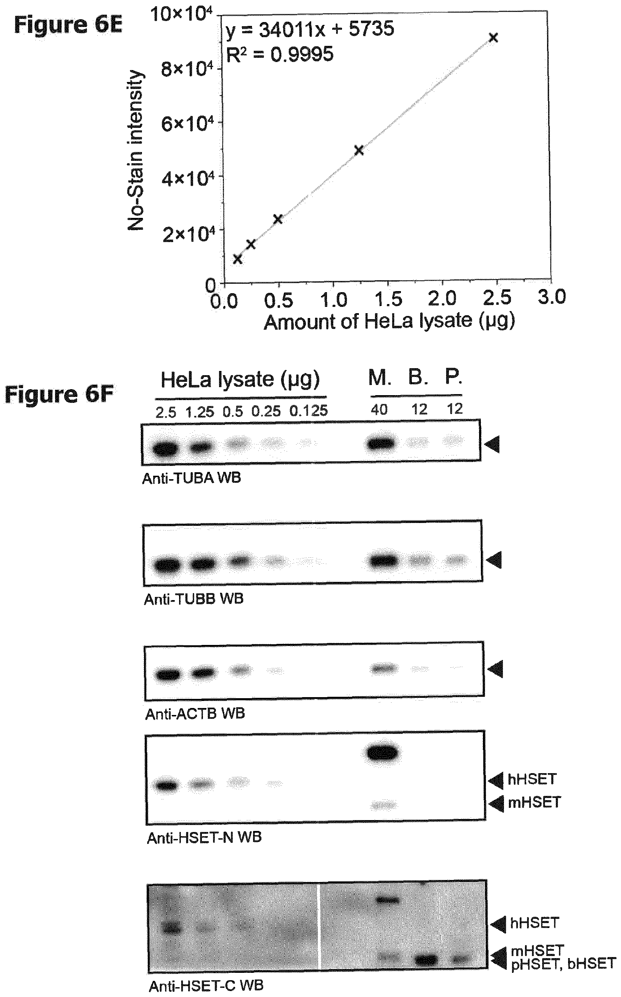

The inventors subsequently examined KIFC1/HSET protein levels in oocytes and in asynchronized HeLa cells as a positive control (**Fig.** 2B and C). Although the inventors could readily detect KIFC1/HSET in HeLa cell, mouse oocyte, bovine oocyte and porcine oocyte lysates (**Fig.** 2B, and **Fig.** 6F), the inventors could not detect KIFC1/HSET in comparable amounts of human oocyte lysate, even after overexposure (**Fig.** 2B). Thus, the inventors conclude that human oocytes lack KIFC1/HSET.

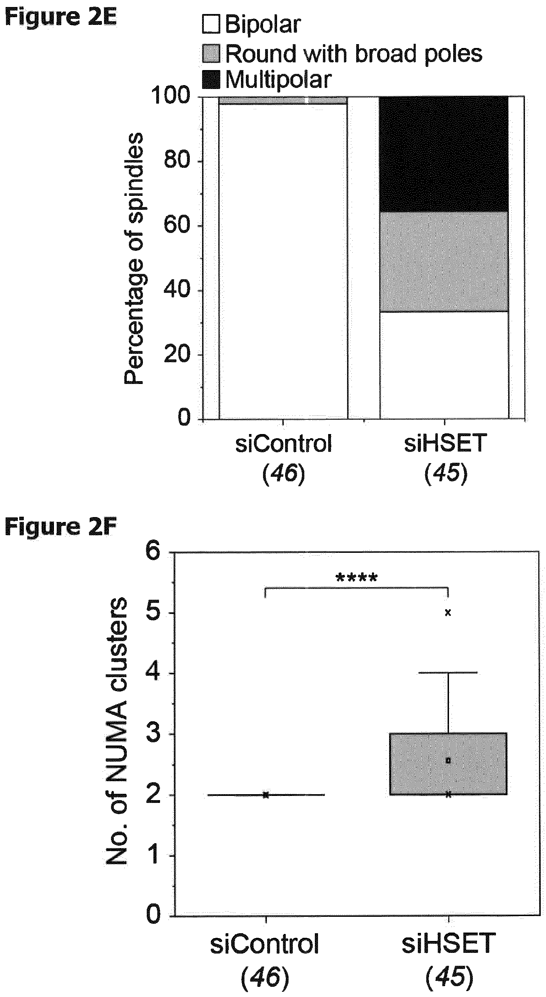

To mimic the lack of KIFC1/HSET in human oocytes, the inventors depleted KIFC1/HSET in humanized mouse oocytes that do not contain aMTOCs and in bovine oocytes using follicle RNAi and Trim-Away, respectively. Strikingly, around 35% of KIFC1/HSET-depleted aMTOC-free mouse oocytes assembled a multipolar spindle (**Fig.** 2D and E). Moreover, around 30% of KIFC1/HSET-depleted aMTOC-free mouse oocytes assembled a round spindle with broad poles (**Fig.** 2D and E). These spindles closely resembled the "apolar" spindles that were previously observed in live human oocytes (2). Thus, the depletion of KIFC1/HSET in aMTOC-free mouse oocytes and in bovine oocytes fully recapitulates the spindle instability of human oocytes. Thus, depletion of KIFC1/HSET specifically recapitulates the spindle instability of human oocytes, strongly suggesting that the lack of KIFC1/HSET is a major contributor to spindle instability in human oocytes.

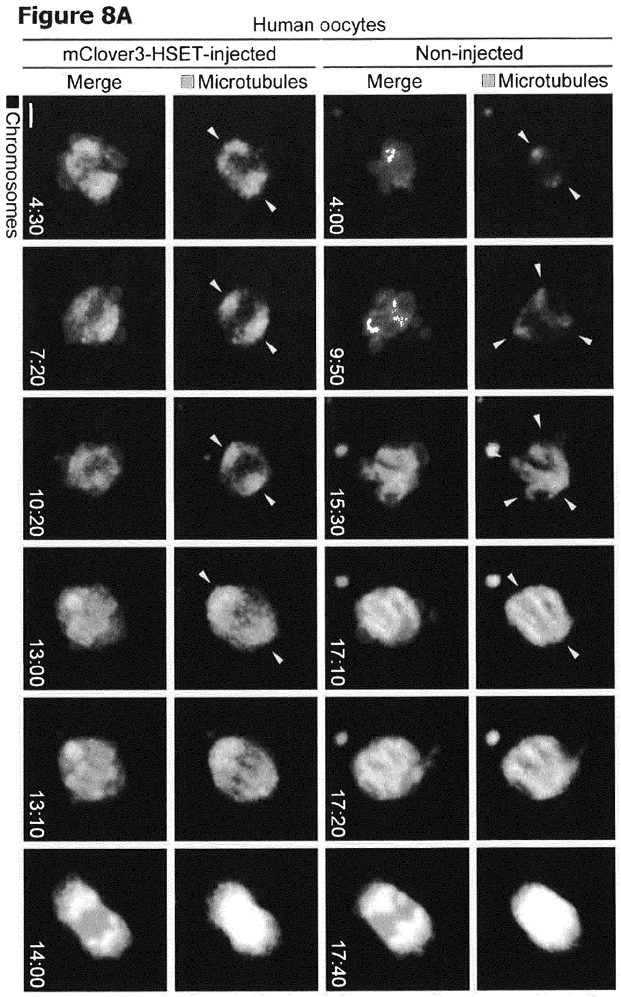

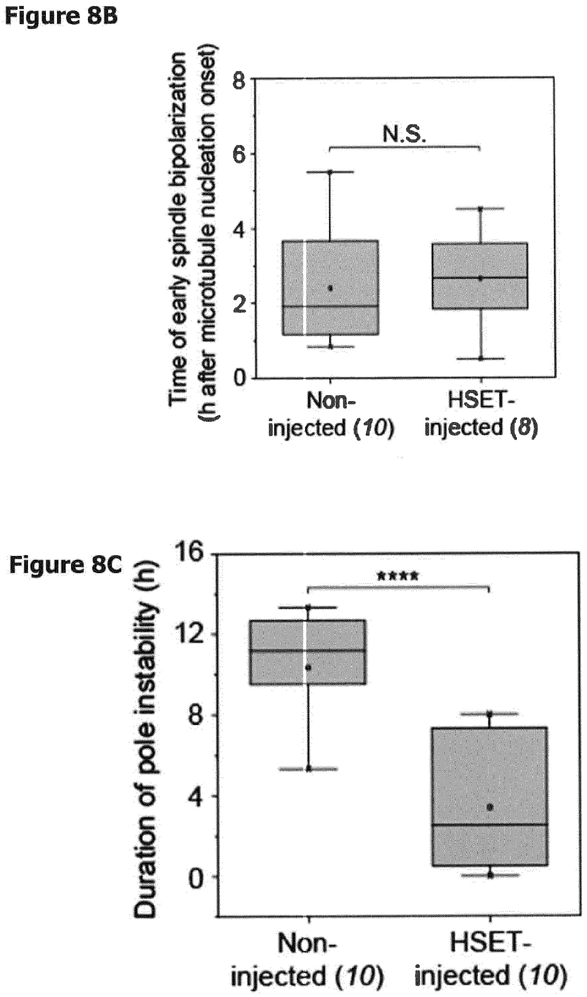

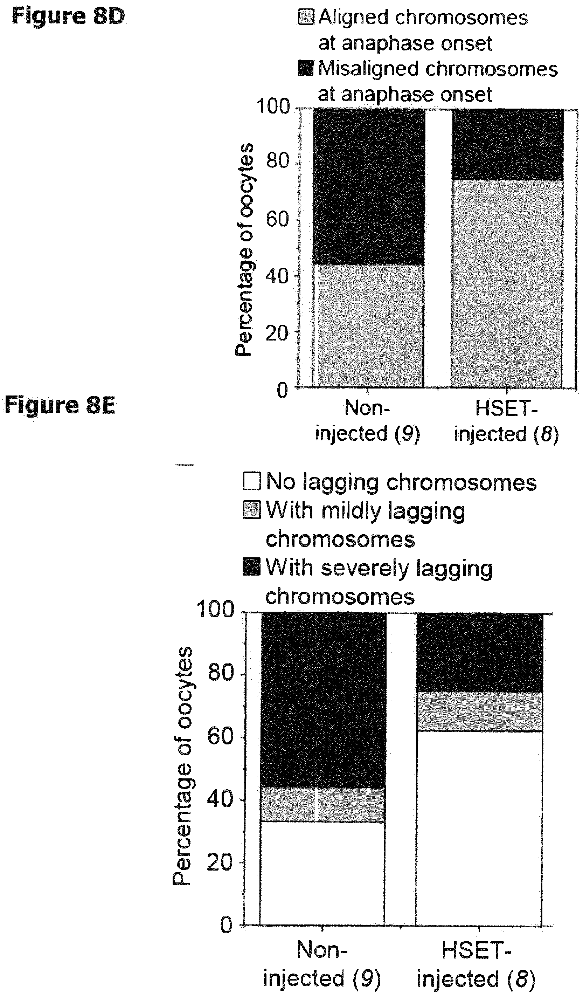

To confirm that the lack of KIFC1/HSET is a major contributor to spindle instability in human oocytes, the inventors introduced KIFC1/HSET into human oocytes in **Fig.** 8 and demonstrate that the introduction of KIFC1/HSET significantly reduced the duration of spindle pole instability (**Fig.** 8C). The inventors also quantified the frequency of misaligned chromosomes at anaphase onset (**Fig.** 8D) and lagging chromosomes (**Fig.** 8E) in human oocytes non-injected and injected with KIFC1/HSET. Together, the inventors infer that the introduction of KIFC1/HSET stabilized the meiotic spindle and reduced the risk of aneuploidy in human oocytes.

The presented data uncover the long-sought cause of spindle instability in human oocytes: Human oocytes lack kinesin-14 KIFC1/HSET, a key spindle-stabilizing factor that is present in oocytes of other species. As shown in this and previous studies (23, 24, 83, 88-90), most mammalian and non-mammalian oocytes express KIFC1/HSET to promote proper spindle assembly. As human oocytes do not express KIFC1/HSET, the inventors propose that the absence of these activities renders their spindles unstable.

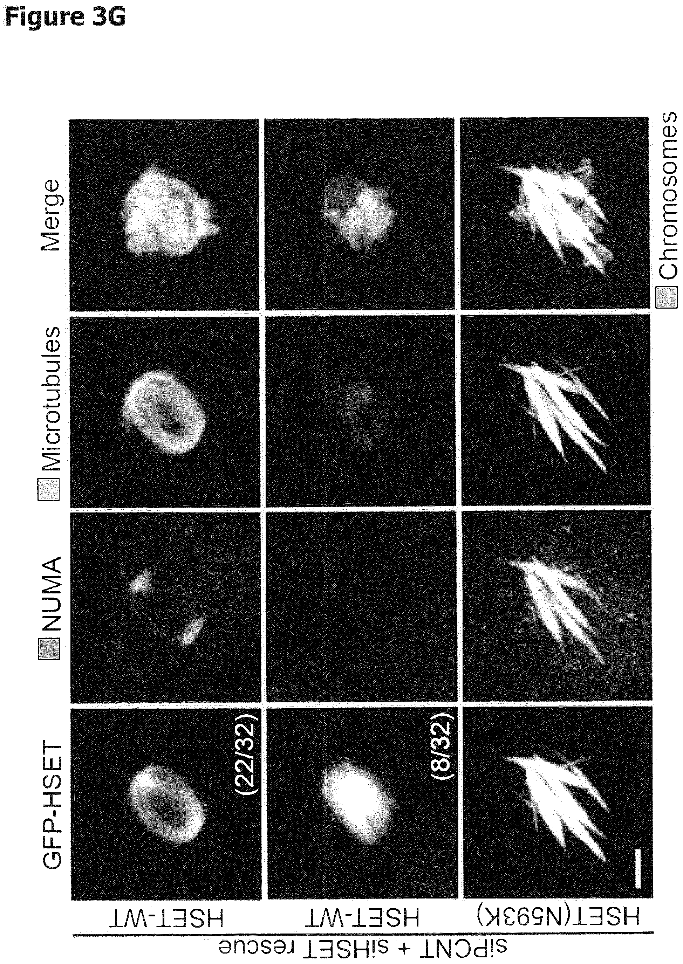

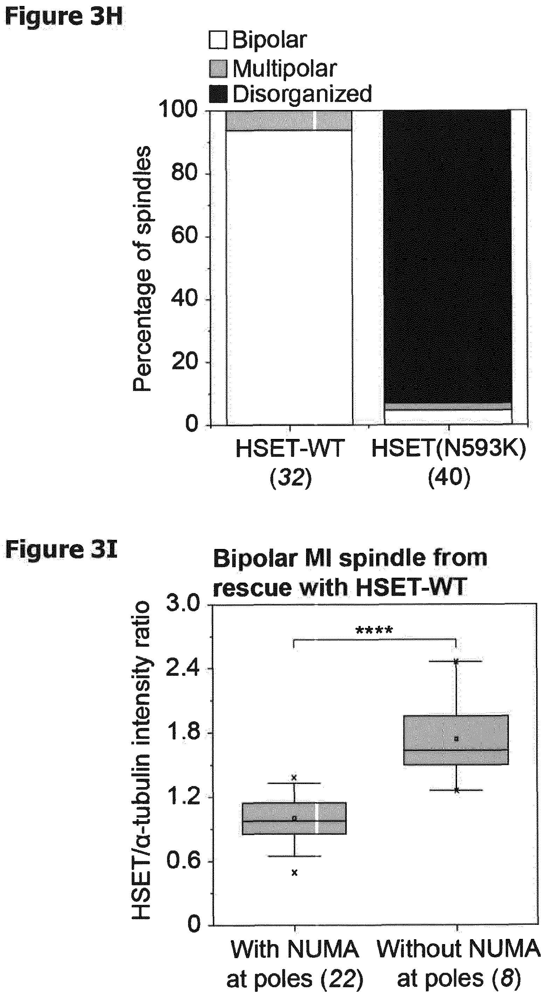

This lack of KIFC1/HSET in human oocytes can explain the surprisingly high rate of aneuploidy occurring in human oocytes. The high percentage of spindle defects can be rescued by the introduction of KIFC1/HSET, e.g. by microinjection, as shown in **Fig.** 3G and H. Hence, the present invention is concerned with stabilizing the human spindle by the *in vitro* supplementation with KIFC1/HSET.

To the knowledge of the inventors, this is the first study to show that human oocytes lack KIFC1/HSET. Mainly based on datasets mouse oocytes and zygotes, it has been expected in the art that KIFC1/HSET is present in human oocytes as well. Thus, based on the art discussed above, the skilled person had no incentive to introduce KIFC1/HSET into human oocytes or a zygote.

Importantly, the introduction of KIFC1/HSET into a human oocyte is contemplated, as further defined by the claims. Upon introduction, KIFC1/HSET stabilizes the human meiotic or mitotic spindle. Thereby, the probability of having unstable spindles is lowered and the risk of aneuploidy is lowered. One particular advantage of the present invention is that KIFC1/HSET is present in human somatic cells. Hence, KIFC1/HSET is well tolerated by the oocytes or zygotes. The invention is thus concerned with KIFC1/HSET protein or RNA for assisted reproductive technology.

The methods described herein are *in vitro* methods for assisted reproductive technology. Hence, the KIFC1/HSET is introduced in *ex vivo.* In other words, the KIFC1/HSET is introduced into a naturally occurring oocyte and thereby a non-naturally occurring oocyte is obtained.

KIFC1/HSET does not alter the germline or integrates into the genome. Instead, KIFC1/HSET supports the stability of the human meiotic or mitotic spindle. Thereby supports the correct segregation of human chromosomes, i.e. avoiding aneuploidy.

The scope of the invention is defined by the claims.

### Detailed description of the invention

In a first aspect, an *in vitro* method for assisted reproductive technology comprising introducing (i) KIFC1/HSET protein or (ii) mRNA encoding KIFC1/HSET into a human oocyte is disclosed. Specifically, the KIFC1/HSET may be introduced into the oocyte, for example by microinjection. The term 'introducing' as used herein refers to bringing a biological molecule, e.g. the KIFC1/HSET, into a cell, e.g. an oocyte or zygote. A particularly preferred biological molecule, as used herein, is a protein or mRNA. Similarly, an in vitro method of supplementing a human oocyte with (i) KIFC1/HSET protein or (ii) mRNA encoding KIFC1/HSET is also disclosed. The term 'supplementing' as used herein refers adding a biological molecule to something, e.g. adding KIFC1/HSET to the oocyte. Upon introduction, the oocyte comprises KIFC1/HSET, preferably to a detectable amount by e.g. Western Blot. Hence, an oocyte comprising (i) KIFC1/HSET protein or (ii) mRNA encoding KIFC1/HSET, which has been introduced into the oocyte is also disclosed herein. Of course, the skilled person is aware of suitable methods to introduce a biological molecule into a cell or to supplement a cell with a biological molecule. For example, the skilled person is aware of microinjection methods, which are standard in the art. Furthermore, microinjection has been used by the inventors and described in Example 2 (see Methods and Materials). Alternatively, the skilled person may use electroporation, which has been described in the art (*114*).

The method may also be referred to as an *in vitro* method comprising the step of
(I) providing (i) KIFC1/HSET protein or (ii) mRNA encoding KIFC1/HSET and
(II) introducing (i) KIFC1/HSET protein or (ii) mRNA encoding KIFC1/HSET into a human oocyte, is disclosed.

Before performing the method, the human oocyte may be, preferably is, a naturally occurring oocyte. After performing the method, the human oocyte may be, preferably is, a non-naturally oocyte. This is because human oocytes lack (i) KIFC1/HSET protein or (ii) mRNA encoding KIFC1/HSET, as suggested by the data shown herein. The term 'naturally occurring', as used herein, means stemming from a natural origin, preferably is obtainable from nature, including the human body. Hence, a naturally occurring oocyte, as used herein, is an oocyte to which no KIFC1/HSET has been introduced into. *Vice versa,* a non-naturally occurring oocyte as used herein is a oocyte to which KIFC1/HSET has been introduced into. Hence, the term 'naturally occurring' does not mean that the oocyte is in its natural environment, such as the ovaries, fallopian tubes or the uterus. In general, oocytes are obtainable by oocyte retrieval, wherein oocyte retrieval as such is not part of the invention. The method of introducing KIFC1/HSET into an oocyte is an *in vitro* method for assisted reproductive technology.

In general, the term *'in vitro'* as used herein is means outside of a living organism such a human body. For example, the method may be performed after having obtained an oocyte from a woman and by microinjection of the KIFC1/HSET into the oocyte. In other words, the methods as described herein are performed *ex vivo.* This means that the methods are not performed on a living subject, such as a human being.

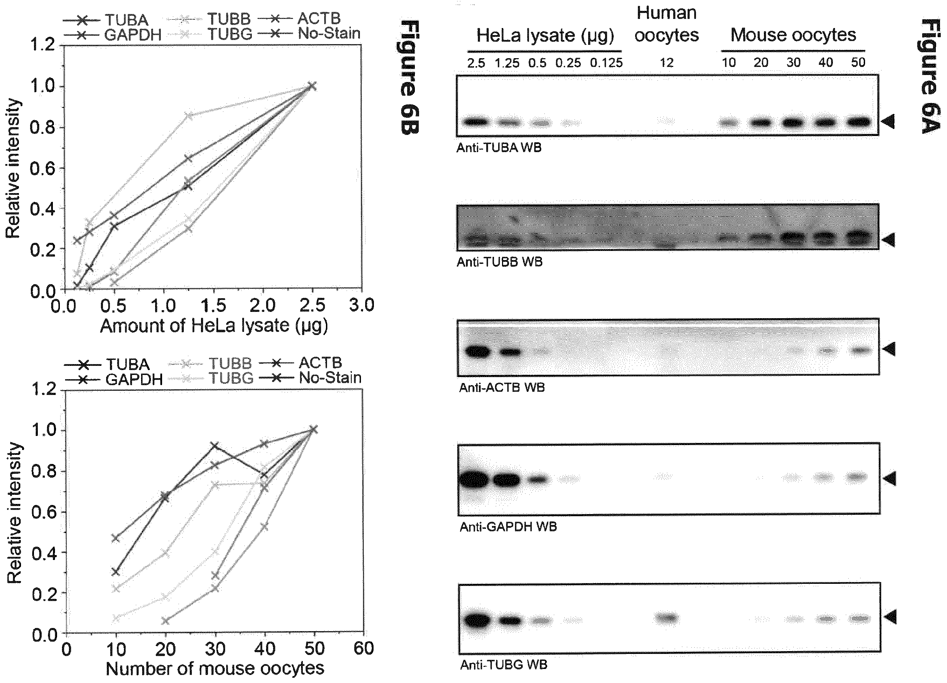

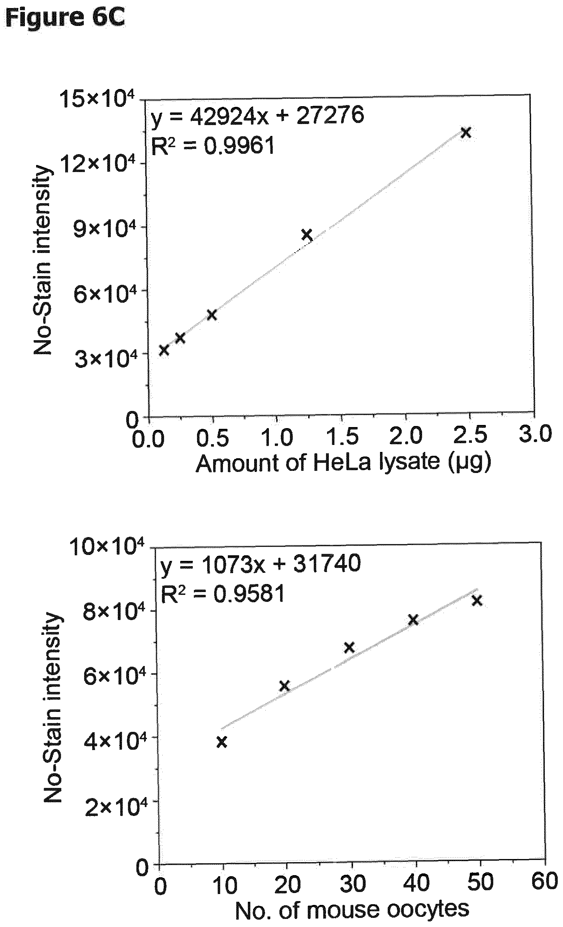

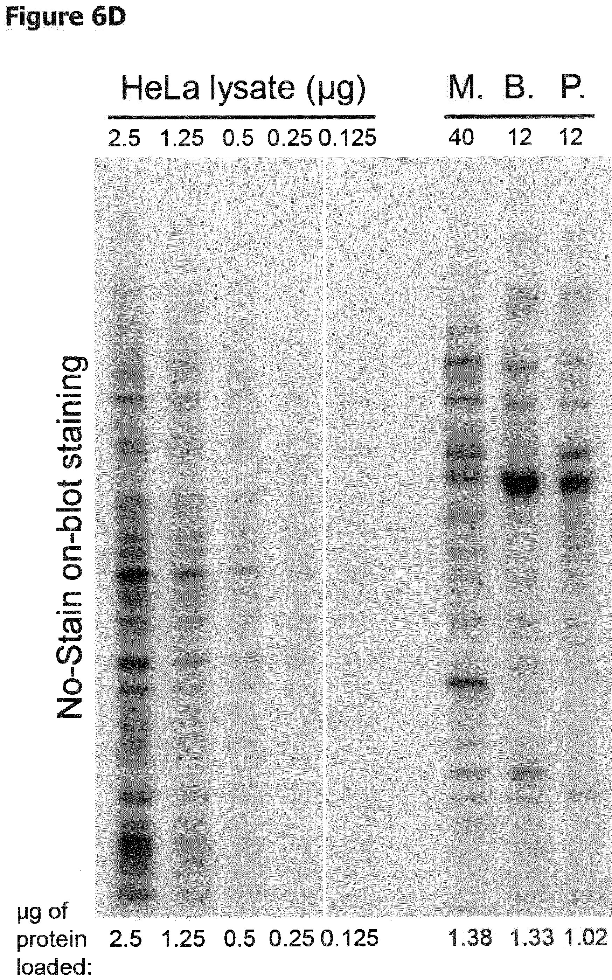

In an embodiment, the human oocyte expresses the KIFC1/HSET less than a mouse oocyte and/or a HeLa cell. Regarding the expression of KIFC1/HSET in human oocytes, mouse oocytes and HeLa cells, particular reference is made to **Fig.** 2B herein. The term 'expression' as used herein refers to the concentration of KIFC1/HSET (amount per volume) in a cell. For example, in **Fig. 2B** 10 - 50 mouse, 12 human, bovine or porcine oocytes per lane were used. More preferably, the human oocyte may express the KIFC1/HSET protein less than a mouse oocyte and/or a HeLa cell based on the concentration of KIFC1/HSET protein. Hence, the expression of KIFC1/HSET protein is not assessed based on the total amount per cell. This concentration based approach is also reflected in the data as shown herein: To ensure comparable loading of oocyte lysate from different species (**Fig. 2B**), the inventors performed on-blot total protein normalization, which outperformed the sensitivity and linearity of all canonical housekeeping proteins (**Fig.** 6, A to E). However, the expression of KIFC1/HSET is in practice typically not assessed in every oocyte as this would require sacrificing the oocyte for analysis. The significantly lower expression of KIFC1/HSET represents a characteristic of human oocytes.

Specifically, the human oocyte may express the KIFC1/HSET at least 2-fold less than a mouse oocyte, preferably at least 4-fold less, more preferably at least 6-fold less, even more preferably at least 10-fold less, and even more preferably at least 20-fold less, even more preferably at least 30-fold less, even more preferably at least 40-fold less, even more preferably at least 50-fold less, and even more preferably at least 60-fold less. Preferably, the human oocyte may express the KIFC1/HSET protein at least 2-fold less than a mouse oocyte, more preferably at least 4-fold less, more preferably at least 6-fold less, even more preferably at least 10-fold less, and even more preferably at least 20-fold less even more preferably at least 30-fold less, even more preferably at least 40-fold less, even more preferably at least 50-fold less, and even more preferably at least 60-fold less. Of course, the mouse oocyte, as referred herein, is a wild-type mouse oocyte. In particular, the wild-type mouse oocyte is a naturally occurring oocyte, i.e. an oocyte stemming from a natural origin. In other words, a wild-type mouse oocyte is not depleted of specific proteins by e.g. siRNA or Trim-away. Specifically, a mouse oocyte is not depleted of KIFC1/HSET. Based on the experiments of the inventors, such as the data presented in **Fig.** 2B, the inventors could detect a 74-fold lower expression of KIFC1/HSET protein in human oocytes than in mouse oocytes.

In addition, the human oocyte human oocyte may express the KIFC1/HSET at least 2-fold less than a HeLa cell, preferably at least 4-fold less, more preferably at least 6-fold less, even more preferably at least 10-fold less, and even more preferably at least 20-fold less. Preferably, the human oocyte may express the KIFC1/HSET protein at least 2-fold less than a Hela cell, more preferably at least 4-fold less, more preferably at least 6-fold less, even more preferably at least 10-fold less, and even more preferably at least 20-fold less. Based on the experiments of the inventors, such as the data presented in **Fig.** 2B, the inventors could detect a 28-fold lower expression of KIFC1/HSET protein in human oocytes than in HeLa cells.

As reflected e.g. in Fig. 2B, the expression of the KIFC1/HSET may be assessed by the band intensity on Western Blot. Synonymously, the term 'Immunoblot' may be used. Of course, the skilled person is aware of standard procedures to perform a Western Blot, wherein proteins that have been separated by electrophoresis are transferred onto e.g. PVDF or nitrocellulose membranes, and are identified by their reaction with labeled antibodies. The band intensity on Western Blot may be assessed by using the same KIF1/HSET antibody as primary antibody, preferably wherein the band intensity is assessed using a rabbit anti-HSET antibody, more preferably the rabbit anti-HSET-N antibody ab172620 (Abcam). Of course the skilled person is aware that primary antibodies towards KIFC1/HSET, as well as secondary antibodies, can be commercially obtained from many providers. Moreover, the skilled person is able to ensure that the primary antibody used has the same or a similar affinity to human and mouse KIFC1/HSET. For example, the skilled person could confirm the affinity in somatic mouse and human cells by blotting different ranges of total protein and comparing the band intensity of KIFC1/HSET at comparable total protein levels.

In a preferred embodiment, the Western Blot is performed using the same secondary antibody, the same blocking solution, the same incubation times, same lysis buffer, and/or the same reaction buffer. An exemplary secondary antibody may be anti-rabbit, which can be commercially obtained, e.g. from Sigma-Aldrich, such as the antibody A9169. A suitable blocking solution may be Tris-buffered saline (TBS) with 5% skim milk and 0.1% tween-20. Suitable incubation times with the primary and secondary antibody can be easily determined by the skilled person. For example, the incubation with the primary antibody may be performed at e.g. 4°C overnight or for shorter time periods at room temperature, and/or the incubation with the secondary antibody may be performed at room temperature for 1 hour. A preferred lysis buffer is 1.333× NuPAGE LDS sample buffer (Thermo Fisher Scientific) with 100 mM DTT. It is especially preferred that the Western Blot of the human oocytes, the mouse oocytes and the HeLa cells are performed under the same conditions.

The expression of KIFC1/HSET may be assessed by alternative methods in the art such as mass spectrometry. The skilled person is aware of standard procedures to determine the expression of a protein in two samples quantitatively. Furthermore, the expression mRNA encoding KIFC1/HSET may also be assessed by standard methods in the art, such as RNA sequencing, Northern Blot analysis, in situ hybridization and/or reverse transcription-polymerase chain reaction (RT-PCR) (see also ref. *119*).

Kinesin-like protein KIFC1 is referred to herein as 'KIFC1/HSET' is a protein that in humans is encoded by the KIFC1 gene and the protein as such is well known to the skilled person (*87*, 88). Briefly, the protein KIFC1/HSET is a member of kinesin-14 family. KIFC1/HSET consists of C-terminal motor domain, superhelical stalk and N-terminal tail domain. As known in the art, tail and motor domains contain microtubule-binding sites. This kinesin moves towards the minus-end of microtubule and has an ability to slide or crosslink microtubules. Upon translation of the mRNA encoding KIFC1/HSET, is functional KIFC1/HSET. The general function of KIFC1/HSET in the context of mitosis of somatic cells is known in the art. Hence, it is known that KIFC1/HSET protein has microtubule-binding activity, ATP hydrolysis activity and/or microtubule sliding activity, which can be tested *in vitro.* For example, the microtubule-binding activity is detectable by microtubule co-pelleting assay, the ATP hydrolysis is detectable by ATPase Assays and/or the microtubule sliding activity is detectable by microtubule gliding assay, more preferably live-cell imaging or immunofluorescence imaging. Hence, the skilled person is aware of numerous ways to assess the functionality of KIFC1/HSET. In a preferred embodiment, the KIFC1/HSET protein or translated mRNA encoding KIFC1/HSET has microtubule binding activity which is detectable by immunofluorescence imaging as shown in (87) Cai S, Weaver LN, Ems-McClung SC, Walczak CE. Kinesin-14 family proteins HSET/XCTK2 control spindle length by cross-linking and sliding microtubules. Mol Biol Cell. 2009 Mar;20(5):1348-59.

In another preferred embodiment, the KIFC1/HSET is recombinantly produced. Alternatively, KIFC1/HSET may be extracted from mammalian species. Recombinant KIFC1/HSET may be produced by a standard procedure in the art. For example, 293 cells, e.g. from ECACC, may be used for recombinant expression of KIFC1/HSET. Alternatively, other human host cells such as CHO cells, insect cells, yeast cells or bacterial cells may be used for the expression of KIFC1/HSET. Furthermore, the skilled person is aware of standard methods of protein purification such as affinity-purification followed by size exclusion chromatography e.g. via an ÄKTA pure (GE Healthcare) using HisTrap FF (GE Healthcare), followed by size exclusion chromatography using HiLoad 26/600 Superdex 200 pg (GE Healthcare). Furthermore, the skilled person is aware of the recombinant protein expression protocol as described in Hua, K. Jiang, Expression and Purification of Microtubule-Associated Proteins from HEK293T Cells for In Vitro Reconstitution. Methods Mol Biol 2101, 19-26 (2020).

In particular, the KIFC1/HSET protein may be human KIFC1/HSET protein or non-human KIFC1/HSET protein. It is preferred that the KIFC1/HSET protein is human protein. It is also preferred that the KIFC1/HSET **protein** comprises, and preferably consists of, a sequence being at least 70% identical to SEQ ID NO:**1** (human KIFC1/HSET), preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: **1.** In particular, the KIFC1/HSET protein has microtubule-binding activity, ATP hydrolysis activity and/or microtubule sliding activity, which optionally can be tested by the methods as described above.

The mRNA encoding KIFC1/HSET may comprise, preferably consist of, a sequence being at least 70% identical to SEQ ID NO: **2** (human KIFC1/HSET), preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: **2.** In particular, the translated KIFC1/HSET mRNA has microtubule-binding activity, ATP hydrolysis activity and/or microtubule sliding activity, which optionally can be tested by the methods as described above.

In another embodiment, the mRNA encoding KIFC1/HSET translates to an amino acid sequence comprising, preferably consisting of, a sequence being at least 70% identical to SEQ ID NO:**1** (human KIFC1/HSET), preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: **1.** In particular, the KIFC1/HSET protein has microtubule-binding activity, ATP hydrolysis activity and/or microtubule sliding activity, which optionally can be tested by the methods as described above.

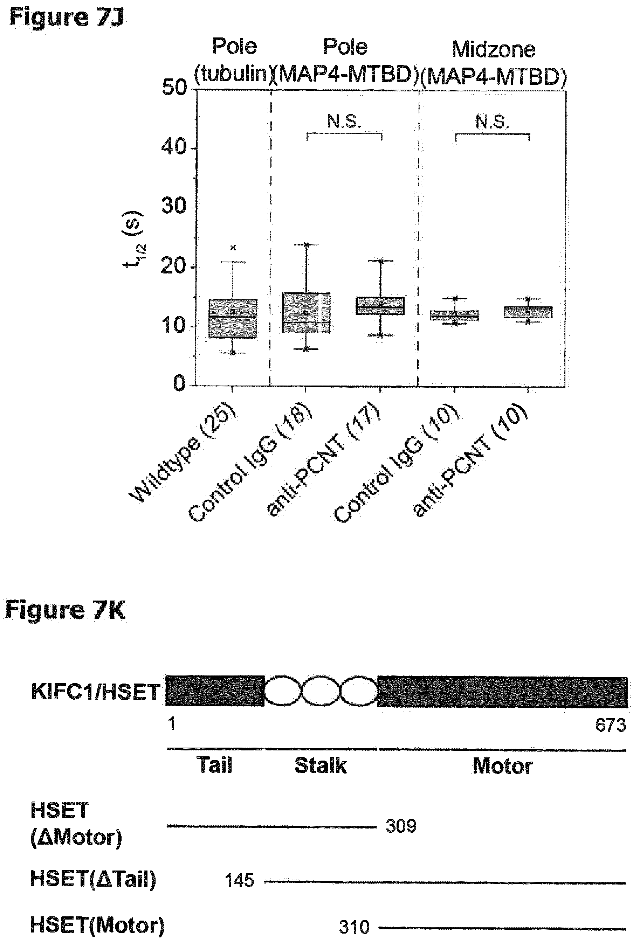

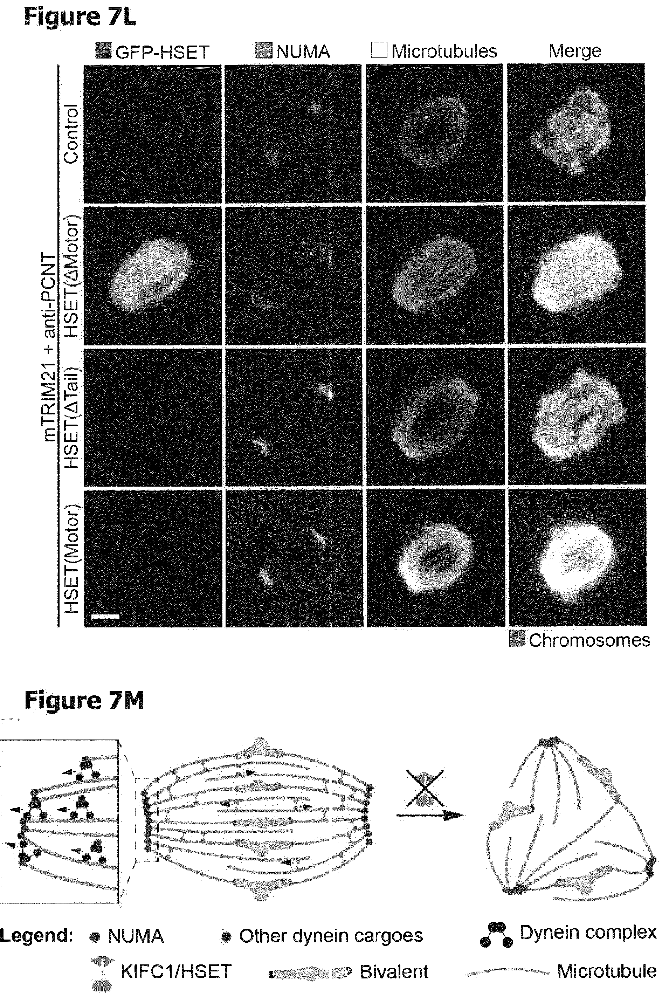

The KIFC1/HSET protein or the mRNA encoding the KIFC1/HSET may also be truncated, preferably wherein the KIFC1/HSET protein or the mRNA encoding the KIFC1/HSET may be N-terminally truncated, more preferably wherein the N-terminal tail of the KIFC1/HSET protein or the mRNA encoding the KIFC1/HSET may truncated. The skilled person is able to determine suitable truncations of the protein so that the protein retains its function. Furthermore, the skilled person can determine the function of a truncated version of KIFC1/HSET by methods as described above. For possible truncations, reference is made to **Fig.** 7K and L. Said Figures show that e.g. an N-terminal truncation of residues 1-144 of SEQ ID No: **1.** For example, the KIFC1/HSET protein may also be a fusion protein, which comprises, preferably consists of, the sequence of amino acids 1-144 and 310-673 of SEQ ID No: **1**. Said fusion protein is likely fully functional based on the observations made by the inventors e.g. shown in Fig. 7K and L.

Analogously, the mRNA encoding KIFC1/HSET may comprise, preferably may consist of the sequence of amino acids 438-2019 of SEQ ID No: **2,** and more preferably the sequence of amino acids 930-2019 of SEQ ID No: **2**.

In case the KIFC1/HSET protein is non-human KIFC1/HSET protein, the non-human KIFC1/HSET protein may be a mammalian KIFC1/HSET protein. Optionally, the KIFC1/HSET protein may be selected from a group consisting of primate, bovine, porcine and rodent KIFC1/HSET protein, more preferably wherein the KIFC1/HSET protein is selected from a group consisting of primate, bovine, porcine KIFC1/HSET protein, even more preferably wherein the KIFC1/HSET protein is primate KIFC1/HSET protein. The skilled person is aware of publicly available databases in order to obtain mammalian KIFC1/HSET sequences, which can e.g. be found on https://www.uniprot.org/. Furthermore, the amino acid sequence of bovine, porcine and mouse KIF1/HSET can be found in SEQ ID NO: **3** (bovine KIFC1/HSET), SEQ ID NO: **4** (porcine KIFC1/HSET), and SEQ ID NO: **5** (mouse KIFC1/HSET). In addition, the amino acid sequence of a primate KIFC1/HSET can be found in SEQ ID NO: **9** (Rhesus macaque KIFC1/HSET). The skilled person is aware of standard tools in the art to detect and/or determine sequence identity of any of these sequences with another sequence. Sequence alignment tools are for example freely available on https://www.ebi.ac.uk/Tools/msa/clustalo/, which also provide a percentage of sequence identity when the sequences are aligned. As known in the art, 'percent identity' refers to the percentage of amino acids or nucleic acids, which are identical between two aligned sequences.

In a preferred embodiment, the KIFC1/HSET protein is at least 70% identical to SEQ ID NO: **9** (Rhesus macaque KIFC1/HSET),preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NOs: **9.**

In another preferred embodiment, the KIFC1/HSET protein is at least 70% identical to any of SEQ ID NO: **3** (bovine KIFC1/HSET), SEQ ID NO: **4** (porcine KIFC1/HSET), and SEQ ID NO: **5** (mouse KIFC1/HSET), preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to any of SEQ ID NOs: **3-5.**

In still another preferred embodiment, the mRNA encoding KIFC1/HSET is at least 70% identical to any of, SEQ ID NO: **6** (bovine KIFC1/HSET), SEQ ID NO: **7** (porcine KIFC1/HSET) and SEQ ID NO: **8** (mouse KIFC1/HSET), preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to any of SEQ ID NOs: **6-8.**

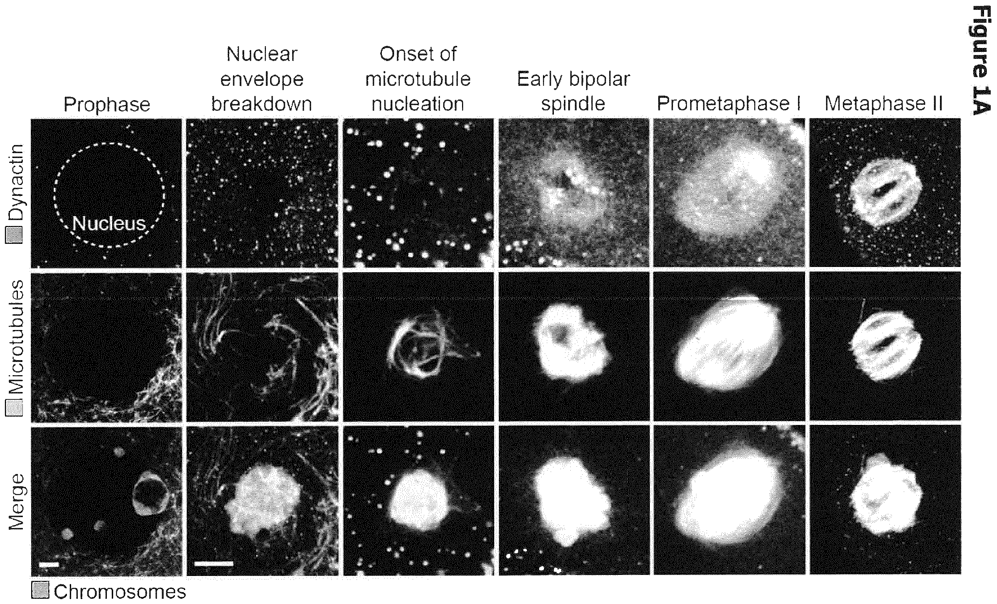

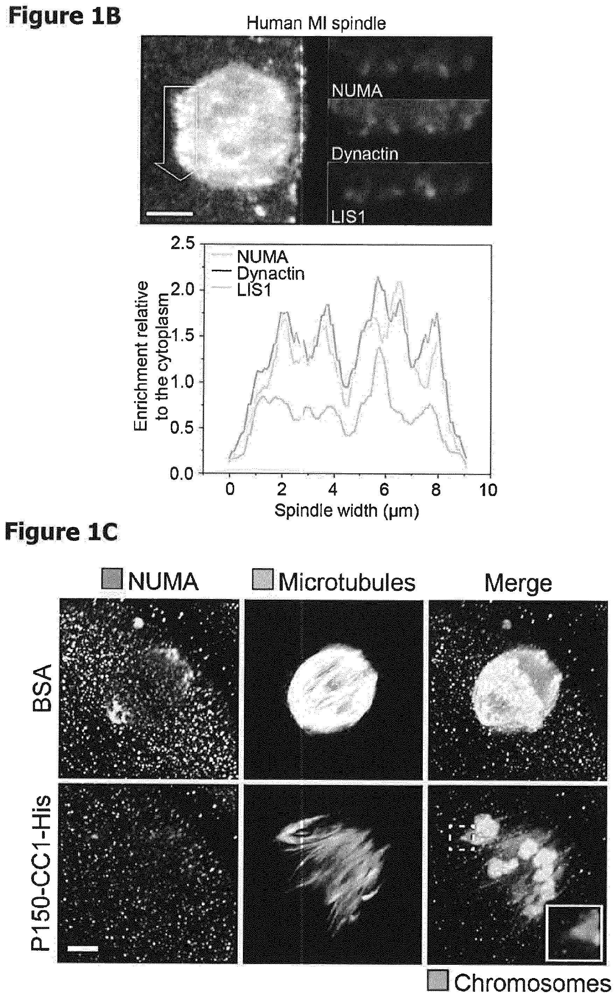

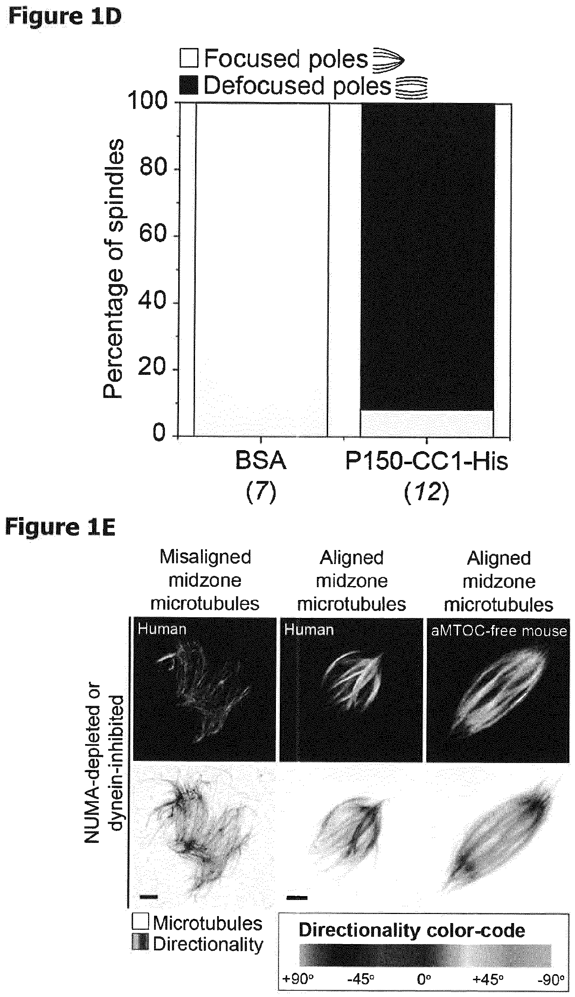

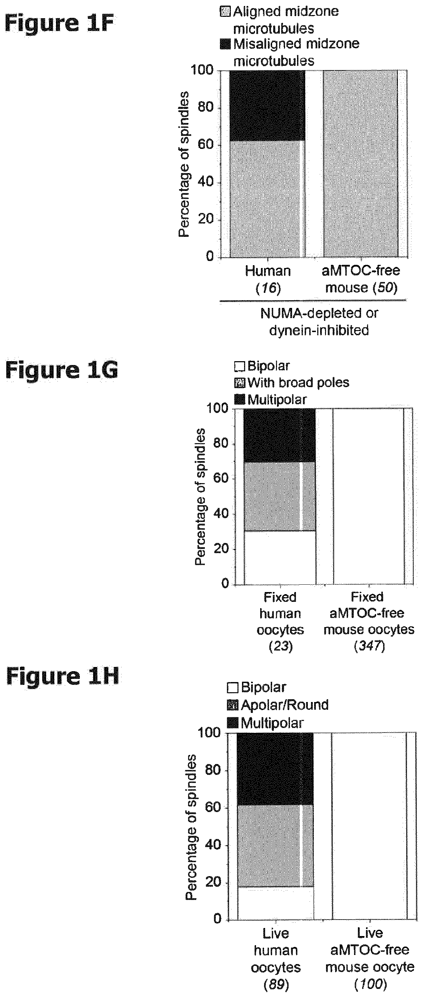

For correct chromosome segregation during meiosis and mitoses, a bipolar spindle is required to be formed, as known in the art. The skilled person is aware of suitable detection methods in order to determine a bipolar spindle, i.e. a spindle with two poles. Furthermore, the skilled person is provided with detailed data and protocols of how to detect a bipolar spindle as shown in Example 2 herein, in particular **Fig.** 1, 2D and 3, 4B and C, 7B. Hence, the skilled person can distinguish between bipolar spindles, disorganized spindles or multipolar spindles. A multipolar spindle as used herein refers to a spindle with at least 3 poles.

In particular, the human oocyte with introduced KIFC1/HSET may have, preferably has, a higher probability of having a bipolar meiotic spindle compared to the human oocyte without introduced KIFC1/HSET. An increase in probability is assumed when a higher percentage of human oocytes to which KIFC1/HSET has been introduced show a bipolar spindle compared human oocytes to which no KIFC1/HSET has been introduced. For example, around 30% of human oocytes in **Fig.** 1G show a bipolar spindle. To mimic the human oocytes as closely as possible, aMTOC-free mouse oocytes have been generated and HSET has been depleted by siRNA. As shown in **Fig.** 2E, said aMTOC-free and KIFC1/HSET depleted mouse oocytes closely mimic human oocytes (around 30% bipolar spindles). In **Fig.** 3H, it is demonstrated that the re-introduction of KIFC1/HSET (rescue experiment) in human oocyte mimics leads to the detection of around 96% bipolar spindles. Hence, the percentage of bipolar spindles changed from around 30 % to 96 %, i.e. a difference of 66% based on the starting percentage of 30%. In other words, the human oocyte mimics of **Fig.** 3H (left bar) to which KIFC1/HSET has been introduced have a 3.2 fold higher probability of having a bipolar spindle than the human oocyte mimics to which no KIFC1/HSET has been introduced. In practice, the percent probability is typically not assessed, when the oocyte is used for *in vitro* fertilization at a later stage as this would require sacrificing the oocytes for analysis. The change in probability represents a characteristic of human oocytes when KIFC1/HSET is introduced into the human oocyte.

Following the data presented, the human oocyte with introduced KIFC1/HSET may have at least a 5% higher probability of having a bipolar meiotic spindle compared to the human oocyte without introduced KIFC1/HSET, preferably at least 10%, more preferably at least 15%, more preferably at least 20%, more preferably at least 25%, and even more preferably at least 35%. The human oocyte with introduced KIFC1/HSET may have at least a 1.05 fold higher probability of having a bipolar meiotic spindle compared to the human oocyte without introduced KIFC1/HSET, preferably at least 1.1 fold, more preferably at least 1.15 fold, more preferably at least 1.2 fold, more preferably at least 1.25 fold, and even more preferably at least 1.35 fold.

*Vice versa,* the human oocyte without introduced KIFC1/HSET may have a lower probability of having a bipolar meiotic spindle compared to the human oocyte with introduced KIFC1/HSET. Specifically, the human oocyte without introduced KIFC1/HSET may have at least a 5% lower probability of having a bipolar meiotic spindle compared to the human oocyte with introduced KIFC1/HSET, preferably at least 10%, more preferably at least 15%, more preferably at least 20%, more preferably at least 25%, and even more preferably at least 35%. In particular, the human oocyte with introduced KIFC1/HSET may have a lower probability of having a multipolar or disorganized meiotic spindle compared to the human oocyte without introduced KIFC1/HSET. More preferably, the human oocyte with introduced KIFC1/HSET may have at least a 5% lower probability of having a multipolar or disorganized meiotic spindle compared to the human oocyte without introduced KIFC1/HSET, preferably at least 10%, more preferably at least 15%, more preferably at least 20%, more preferably at least 25%, and even more preferably at least 35%.

Exemplary data is provided in Example 2. In other words, the human oocyte without introduced KIFC1/HSET may have a higher probability of having a multipolar or disorganized meiotic spindle compared to the human oocyte with introduced KIFC1/HSET. For example, the human oocyte without introduced KIFC1/HSET may have at least a 5% higher probability of having a multipolar or disorganized meiotic spindle compared to the human oocyte with introduced KIFC1/HSET, preferably at least 10%, more preferably at least 15%, more preferably at least 20%, more preferably at least 25%, and even more preferably at least 35%. It is further contemplated that the human oocyte without introduced KIFC1/HSET may have at least a 1.05 fold higher probability of having a multipolar or disorganized meiotic spindle compared to the human oocyte with introduced KIFC1/HSET, preferably at least 1.1 fold, more preferably at least 1.15 fold, more preferably at least 1.2 fold, more preferably at least 1.25 fold, and even more preferably at least 1.35 fold. With regard to the percentage probabilities provided, as used in this disclosure, the percentage probability may be at most 400 %. In other words, the percentage probabilities provided, as used in this disclosure, the percentage probability may be at most 4 fold.

As shown in various Figures herein and specifically referred in Example 2, the bipolar meiotic spindle is assessed or detected by fluorescence microscopy or polarized light microscopy. In practice, the oocyte is not is typically not assessed by fluorescence microscopy, when the oocyte is used for *in vitro* fertilization at a later stage as this would require sacrificing the oocytes for analysis. The change in probability represents a characteristic of human oocytes when KIFC1/HSET is introduced into the human oocyte. Particular reference is made to **Fig.** 8, wherein KIFC1/HSET is introduced into the human oocyte and wherein the introduction of KIFC1/HSET significantly reduced the duration of spindle pole instability, as well as the frequency of misaligned chromosomes and lagging chromosomes. Based on the significantly lower duration of spindle pole instability, the data as shown herein demonstrate that KIFC1/HSET stabilizes the meiotic spindle in human oocytes. Similarly, it is also expected that KIFC1/HSET stabilizes the mitotic spindle in human zygotes. The skilled person is aware of how to determine the duration of spindle pole instability and is provided with further guidance in Example 2 herein. Briefly, the duration of spindle pole instability refers to the duration of excessive spindle pole remodeling after early spindle bipolarization. In the case of human oocytes, the remodeling is largely the widening or fragmentation of spindle poles, which results in apolar or multipolar spindle. The start and end points are determined from the videos based on the first and last time points in which the spindle was not bipolar.

Specifically, **Fig.** 8D demonstrates that more oocytes with introduced KIFC1/HSET had aligned their chromosomes at anaphase onset than oocytes without introduced KIFC1/HSET. Anaphase onset refers to the time point before chromosome separation was first observed, as defined in the inventors' previous study (*2*). More specifically, only 44.4% of oocytes without introduced KIFC1/HSET showed aligned chromosomes at anaphase onset, whereas 75% of oocytes with introduced KIFC1/HSET showed aligned chromosomes as anaphase onset. Thus, there was a difference of 30.6% relative to the starting percentage of 44%. In other words, the human oocytes in **Fig.** 8D into which KIFC1/HSET has been introduced have a 1.7 fold (75%/44%) higher probability of aligned chromosomes at anaphase onset in comparison to an oocyte without introduced KIFC1/HSET.

In an embodiment, the introduction of KIFC1/HSET into the oocyte increases the probability of aligned chromosomes at anaphase onset in comparison to an oocyte without introduced KIFC1/HSET, preferably wherein the probability is increased by at least 5%, more preferably 10%, even more preferably 20%, even more preferably 30%, and even more preferably 40% in comparison to an oocyte without introduced KIFC1/HSET. It is also contemplated that the introduction of KIFC1/HSET into the oocyte increases the probability of aligned chromosomes at anaphase onset in comparison to an oocyte without introduced KIFC1/HSET, preferably wherein the probability is increased by at least 1.05 fold, more preferably at least 1.1 fold, even more preferably at least 1.2 fold, even more preferably at least 1.3 fold, and even more preferably at least 1.4 fold in comparison to an oocyte without introduced KIFC1/HSET.

Additionally, **Fig.** 8E demonstrates that more oocytes with introduced KIFC1/HSET correctly segregated chromosomes than oocytes without introduced KIFC1/HSET. In general, lagging chromosomes during anaphase are known in the art and are a consequence of chromosomes or chromatids not properly separating from each other. When the chromosomes or chromatids do not properly separate during anaphase, the daughter cells are more likely to inherit incorrect numbers of chromosomes. Lagging chromosomes are therefore a common cause of aneuploidy. Hence, the higher the probability of lagging chromosomes during anaphase, the higher the probability of aneuploidy. Chromosomes are classified as mildly and severely lagging (see **Fig.** 8E) when they failed to clear the central spindle region within 10 or 20 min after anaphase onset, respectively, which has been previously described in the art (121). Specifically, 33.3% of oocytes without introduced KIFC1/HSET had no lagging chromosomes, while 62.5% of oocytes with introduced KIFC1/HSET had no lagging chromosomes. Thus, there was a difference of 29.5% based on the starting percentage of 33%. In other words, the human oocytes in **Fig.** 8E into which KIFC1/HSET has been introduced have a 1.9 fold (62.5%/33.3%) higher probability of aligned chromosomes at anaphase onset in comparison to an oocyte without introduced KIFC1/HSET.

In an embodiment, the introduction of KIFC1/HSET into the oocyte increases the probability of no lagging chromosomes in comparison to an oocyte without introduced KIFC1/HSET, preferably wherein the probability is increased by at least 5%, more preferably at least 10%, even more preferably at least 20%, even more preferably at least 30%, and even more preferably at least 40% in comparison to an oocyte without introduced KIFC1/HSET. It is also contemplated that the introduction of KIFC1/HSET into the oocyte increases the probability of no lagging chromosomes in comparison to an oocyte without introduced KIFC1/HSET, preferably wherein the probability is increased by at least 1.05 fold, more preferably at least 1.1 fold, even more preferably at least 1.2 fold, even more preferably at least 1.3 fold, and even more preferably at least 1.4 fold in comparison to an oocyte without introduced KIFC1/HSET.

Ideally, the introduction of KIFC1/HSET into the oocyte may increase the formation of a bipolar spindle in comparison to an oocyte without introduced KIFC1/HSET. In other words, the introduction of KIFC1/HSET into the oocyte may increase the probability of the formation of a bipolar spindle in comparison to an oocyte without introduced KIFC1/HSET. In particular, after the introduction of KIFC1/HSET into the oocyte, the probability of the formation of a bipolar spindle is increased in comparison to the naturally occurring oocyte without introduced KIFC1/HSET. Said increase in probability may be assessed by comparing the percentages of the formation of a bipolar spindle in (a) naturally occurring human oocytes or human oocytes to which no KIFC1/HSET has been introduced with (b) non-naturally occurring human oocyte or human oocytes to which KIFC1/HSET has been introduced. An increase in probability is obtained when a higher percentage of (b) non-naturally occurring human oocytes or human oocytes to which KIFC1/HSET has been introduced show a bipolar spindle than (a) naturally occurring human oocytes or human oocytes to which no KIFC1/HSET has been introduced. In general and as used herein, the higher the probability number of an event, the more likely is it that the event will occur. The KIFC1/HSET may stabilize the meiotic spindle of the human oocyte. For example, the human oocyte with the stabilized meiotic spindle may have a higher probability of having a bipolar spindle than a non-stabilized meiotic spindle. In particular, the non-stabilized spindle may be a multipolar or a disorganized spindle. For example, the stabilization of the meiotic spindle may be assessed or detected by spindle polarity morphology, preferably wherein the spindle polarity morphology of a stabilized meiotic spindle has higher probability of being a bipolar spindle than a non-stabilized meiotic spindle, more preferably wherein the non-stabilized spindle is a multipolar or a disorganized. As also noted above and shown herein, meiotic spindle may be assessed by fluorescence microscopy or polarized light microscopy.

The (i) KIFC1/HSET protein or the (ii) mRNA encoding KIFC1/HSET may be introduced during various stages. In particular, KIFC1/HSET may be introduced during germinal vesicle stage, meiosis I or meiosis II. As known in the art, the germinal vesicle (GV) is the nucleus of the oocyte. All oocytes within the ovary are arrested in the dictyate stage of prophase of meiosis I (MI), and this meiotic stage is characterized by the presence of an intact GV, which can be identified morphologically by histology and light microscopy. Oocytes maintain this meiotic arrest throughout oogenesis as they grow in size. As oocytes begin to mature, their nuclei-germinal vesicles (GV) break down and chromosomes condense, the germinal vesicle breakdown (GVBD). Specifically, (i) the KIFC1/HSET protein or (ii) the mRNA encoding KIFC1/HSET may be introduced during germinal vesicle stage. In an embodiment, (i) the KIFC1/HSET protein or (ii) the mRNA encoding KIFC1/HSET may be introduced during prophase dictyate arrest, prometaphase I meiotic spindle formation, metaphase I, anaphase I, or telophase I. Preferably, (i) the KIFC1/HSET protein or (ii) the mRNA encoding KIFC1/HSET may be introduced during germinal vesicle stage, prophase, before nuclear envelope breakdown, and/or after nuclear envelope breakdown but before fertilization. Of course, the skilled person is aware of the various stages and is able to distinguish between said stages *in vitro,* e.g. by light microscopy. In particular, (i) the KIFC1/HSET protein or (ii) the mRNA encoding KIFC1/HSET may be introduced during prophase I dictyate arrest. Also preferred is that (i) the KIFC1/HSET protein or (ii) the mRNA encoding KIFC1/HSET is introduced during prometaphase II, or metaphase II. It is even more preferred that (i) the KIFC1/HSET protein or (ii) the mRNA encoding KIFC1/HSET is introduced during metaphase II. In one embodiment, the (i) the KIFC1/HSET protein or (ii) the mRNA encoding KIFC1/HSET is introduced together with sperm into the oocyte.

Moreover, a suitable amount of (i) the KIFC1/HSET protein or (ii) mRNA encoding the human KIFC1/HSET is introduced into the human oocyte. The skilled person is able to determine a suitable amount of (i) the KIFC1/HSET protein or (ii) mRNA encoding the human KIFC1/HSET. For example, the skilled person may perform a titration experiment, which guides the skilled person. Furthermore, the titration experiment may detect spindle morphology disturbances, when too much KIFC1/HSET is introduced into the oocyte, which can be assessed by fluorescence microscopy or polarized light microscopy. One sign of spindle morphology disturbance is the excessive bundling of spindle microtubules, as for example shown in **Fig.** 3G and as described in Example 2 herein. At the same time, introducing too little KIFC1/HSET does not improve the probability of showing bipolar spindles.

Exemplary suitable amount of KIFC1/HSET protein may be between 1-250 pg per oocyte, preferably 2-150 pg, more preferably 3-100 pg, more preferably 4-80 pg, more preferably 5-50 pg, even more preferably 6-30 pg, even more preferably 7-20 pg, even more preferably 8-15 pg, and most preferably around 10 pg.

Exemplary suitable amount of mRNA encoding the human KIFC1/HSET may be between 1-4000 fg per oocyte, preferably 5-3000 fg, more preferably 20-2500 fg, more preferably 35-2000 fg, more preferably 50-1900 fg, even more preferably 60-1700 fg, even more preferably 70-1500 fg, even more preferably 100-1000 fg, and most preferably 140-750 fg.

The methods as described herein do not modify the germline identity of human beings.

Also disclosed is an *in vitro* method for stabilizing the meiotic spindle of a human oocyte by
a. providing the human oocyte *in vitro* and
b. delivering (i) the KIFC1/HSET protein or (ii) mRNA encoding the KIFC1/HSET into the human oocyte,
whereby the human KIFC1/HSET protein stabilizes the meiotic spindle in the human oocyte. In this method, the delivery of (i) the KIFC1/HSET protein or (ii) mRNA encoding the human KIFC1/HSET is synonymous to the introduction of (i) the KIFC1/HSET protein or (ii) mRNA encoding the human KIFC1/HSET. Furthermore, the introduction is further defined above, the human oocyte is further defined above, (i) the KIFC1/HSET protein or (ii) mRNA encoding the KIFC1/HSET is further defined above, the stabilization of the meiotic spindle is further defined above, and/or a suitable amount of (i) KIFC1/HSET protein or (ii) mRNA encoding the KIFC1/HSET is further above. In other words, one or more of the above embodiments may be combined with said method.

Also disclosed is a method of stabilizing the meiotic spindle of a human oocyte by (i) KIFC1/HSET protein or (ii) mRNA encoding the KIFC1/HSET. Preferably, (i) the KIFC1/HSET protein or (ii) mRNA encoding the KIFC1/HSET is introduced into the human oocyte, preferably wherein the KIFC1/HSET protein is recombinantly produced KIFC1/HSET protein. Preferred embodiments regarding the introduction, the human oocyte, the stabilization of the meiotic spindle, and/or the suitable amount of (i) KIFC1/HSET protein or (ii) mRNA encoding the KIFC1/HSET are detailed above and apply equally to this aspect of the invention.

A particular advantage of the invention is that the human spindle is stabilized. This stabilization reduces the risk for aneuploidy. As also known in the art, aneuploidy is the presence of an abnormal number of chromosomes in a cell, for example a human cell having 45 or 47 chromosomes instead of the usual 46. In contrast, a cell with any number of complete chromosome sets is called a euploid cell. Hence, aneuploidy is a result of an improper chromosome segregation. Errors in chromosome segregation lead to aneuploidy, a state where the number of chromosomes in a cell or organism deviates from multiples of the haploid genome. Aneuploidy arising through chromosome mis-segregation during meiosis is a major cause of infertility and inherited birth defects. Hence, based on the data disclosed herein, it is expected that the stabilized human spindle reduces the risk of aneuploidy. Also based on the data, the introduction of KIFC1/HSET is expected to reduce the risk of aneuploidy.

Disclosed, but not part of the invention is a non-naturally occurring human oocyte, wherein a (i) KIFC1/HSET protein or (ii) KIFC1/HSET mRNA may have been introduced into a naturally occurring human oocyte thereby obtaining the non-naturally occurring oocyte.

Furthermore, a non-naturally occurring human oocyte may comprise a (i) KIFC1/HSET protein or (ii) KIFC1/HSET mRNA, wherein the (i) KIFC1/HSET protein or (ii) KIFC1/HSET mRNA has been introduced into the oocyte. Moreover, a non-naturally occurring human oocyte is contemplated herein, wherein the non-naturally occurring human oocyte has been supplemented with a (i) KIFC1/HSET protein or (ii) KIFC1/HSET mRNA thereby obtaining the non-naturally occurring oocyte.

Preferably, the KIFC1/HSET protein is a recombinantly produced KIFC1/HSET protein. Exemplary methods for recombinant expression and/or testing the functionality of KIFC1/HSET protein are described above. It is also preferred that (i) the KIFC1/HSET protein or (ii) mRNA encoding the KIFC1/HSET stabilizes the meiotic spindle of the oocyte. As detailed above, the naturally occurring human oocyte expresses the KIFC1/HSET at least 2-fold less than a mouse oocyte and/or a HeLa cell. The information provided above on the naturally occurring human oocyte as well as the expression of KIFC1/HSET and the detection of KIFC1/HSET apply equally to this aspect of the invention. Similarly, the functional and structural information on KIFC1/HSET protein or mRNA encoding the KIFC1/HSET also apply equally to this aspect. Aspects regarding the introduction, the human oocyte, the stabilization of the meiotic spindle, and/or the suitable amount of (i) KIFC1/HSET protein or (ii) mRNA encoding the KIFC1/HSET are detailed above and apply equally to this aspect of the disclosure.

In addition, a device for injection comprising (i) KIFC1/HSET protein or (ii) mRNA encoding the KIFC1/HSET suitable for introduction into a human oocyte *in vitro,* wherein the device is a microinjection needle is disclosed herein. It is preferred that (i) the KIFC1/HSET protein or (ii) mRNA encoding the KIFC1/HSET stabilizes the meiotic spindle of the oocyte. Preferred embodiments regarding the introduction, the human oocyte, the stabilization of the meiotic spindle, and/or the suitable amount of (i) KIFC1/HSET protein or (ii) mRNA encoding the KIFC1/HSET are detailed above and apply equally to this part of the invention.

Moreover, a device for injection comprising KIFC1/HSET protein or KIFC1/HSET mRNA suitable for stabilizing the meiotic spindle human oocyte *in vitro* is disclosed herein. It is preferred that (i) the KIFC1/HSET protein or (ii) mRNA encoding the KIFC1/HSET stabilizes the meiotic spindle of the oocyte. Preferred embodiments regarding the introduction, the human oocyte, the stabilization of the meiotic spindle, and/or the suitable amount of (i) KIFC1/HSET protein or (ii) mRNA encoding the KIFC1/HSET are detailed above and apply equally to this aspect of the invention.

In general, a method of introducing (i) KIFC1/HSET protein or (ii) mRNA encoding the KIFC1/HSET into a human oocyte is disclosed. It is preferred that (i) the KIFC1/HSET protein or (ii) mRNA encoding the KIFC1/HSET stabilizes the meiotic spindle of the oocyte. Preferred embodiments regarding the introduction, the human oocyte, the stabilization of the meiotic spindle, and/or the suitable amount of (i) KIFC1/HSET protein or (ii) mRNA encoding the KIFC1/HSET are detailed above and apply equally to this part of the invention.

In another aspect, a (i) KIFC1/HSET protein or (ii) mRNA encoding the KIFC1/HSET for use in a method of lowering the probability of having a disorganized and/or a multipolar spindle during mitosis in a human zygote by introducing (i) KIFC1/HSET protein or (ii) mRNA encoding the KIFC1/HSET into a human zygote is disclosed. Additionally, a (i) KIFC1/HSET protein or (ii) mRNA encoding the KIFC1/HSET for use in a method of lowering the probability of having a disorganized and/or a multipolar spindle during mitosis in a human zygote by supplementing the human zygote with (i) KIFC1/HSET protein or (ii) mRNA encoding the KIFC1/HSET is disclosed.

Furthermore, a (i) KIFC1/HSET protein or (ii) mRNA encoding the KIFC1/HSET for use in a method of increasing the probability of having a bipolar spindle during mitosis in a human zygote by introducing (i) KIFC1/HSET protein or (ii) mRNA encoding the KIFC1/HSET into a human zygote is disclosed. In addition, a (i) KIFC1/HSET protein or (ii) mRNA encoding the KIFC1/HSET for use in a method of increasing the probability of having a bipolar spindle during mitosis in a human zygote by supplementing the human zygote with (i) KIFC1/HSET protein or (ii) mRNA encoding the KIFC1/HSET is disclosed.

Ideally, the method prevents disorganized and/or a multipolar spindle during mitosis in a human zygote. Furthermore, a (i) KIFC1/HSET protein or (ii) mRNA encoding the KIFC1/HSET for use in a method of lowering the probability aneuploidy by introducing (i) KIFC1/HSET protein or (ii) mRNA encoding the KIFC1/HSET into a human zygote is disclosed. Ideally, the method prevents aneuploidy upon mitotic division. As known in the art, a zygote is a fertilized egg cell that results from the union of a female gamete. Hence, a zygote is a single cell.

In an instance, (i) the KIFC1/HSET protein or (ii) mRNA encoding the KIFC1/HSET stabilizes the mitotic spindle of the human zygote, preferably wherein the KIFC1/HSET protein is recombinantly produced KIFC1/HSET protein. Similarly to the experiments known from the art and as disclosed herein the stabilization of the human mitotic spindle can be assessed by polarized light microscopy.

Preferably, the human zygote is provided and is not part of the invention. For example, in practice the zygote may be obtainable by *in vitro* fertilization, e.g. by intracytoplasmic sperm injection, wherein the *in vitro* fertilization is not form part of the invention.

In an instance, the human zygote expresses the KIFC1/HSET at least 2-fold less than a mouse zygote, preferably at least 4-fold less, more preferably at least 6-fold less, even more preferably at least 10-fold less, and even more preferably at least 20-fold less, even more preferably at least 30-fold less, even more preferably at least 40-fold less, even more preferably at least 50-fold less, and even more preferably at least 60-fold less. Also described is an aspect, wherein the human zygote expresses the KIFC1/HSET at least 2-fold less than a HeLa cells, preferably at least 4-fold less, more preferably at least 6-fold less, even more preferably at least 10-fold less, and even more preferably at least 20-fold less. In practice, the expression of KIFC1/HSET is typically not assessed in zygotes, as this would require sacrificing the zygote for analysis. Human parthenotes can be used as mimics and the expression of KIFC1/HSET can be assessed therein. The expression of the KIFC1/HSET may be assessed by the band intensity on Western Blot. Synonymously, the term 'Immunoblot' may be used. Of course, the skilled person is aware of standard procedures to perform a Western Blot. Furthermore, the details provided regarding the Western Blot for detecting KIFC1/HSET have been described above and apply equally to KIFC1/HSET in zygotes.

The expression of KIFC1/HSET may be assessed by alternative methods in the art such as mass spectrometry. The skilled person is aware of standard procedures to determine the expression of a protein quantitatively. Furthermore, the expression mRNA encoding KIFC1/HSET may also be assessed by standard methods in the art, such as Northern Blot analysis, in situ hybridization and/or reverse transcription-polymerase chain reaction (RT-PCR). In addition, aspects disclosed regarding the function and structure of the (i) KIFC1/HSET protein or (ii) mRNA encoding the KIFC1/HSET also apply to this aspect of the disclosure.

In another aspect, the introduction of KIFC1/HSET into the zygote increases the probability of having a bipolar spindle in comparison to an zygote without introduced KIFC1/HSET. In other words, there is an increased probability for the formation of a bipolar spindle. The presence of the bipolar spindle may be assessed or detected by fluorescence microscopy or polarized light microscopy. As used herein, assessing or detecting refers to the skilled person action of determining the presence of absence of something, for example by using the indicated method.

KIFC1/HSET may be introduced by microinjection or electroporation. For example, the skilled person is aware of microinjection methods, which are standard in the art. Furthermore, microinjection has been used by the inventors and described in Example 2 (see Methods and Materials). Alternatively, electroporation may be used, which is also known in the art for zygotes (*114*)*.*

In yet another aspect, wherein (i) the KIFC1/HSET protein or (ii) the mRNA encoding KIFC1/HSET is introduced during mitosis, preferably during mitotic prophase, metaphase, anaphase, telophase, or S phase, and more preferably during mitotic prophase. Of course the skilled person is aware of these cell cycle stages from common general knowledge and is also able to distinguish between these stages (*120*). Specifically, (i) the KIFC1/HSET protein or (ii) the mRNA encoding KIFC1/HSET may be introduced before the assembly of pronuclei and/or during the assembly or presence of pronuclei. As known in the art, the parental chromosomes of the zygote first become enclosed in two separate pronuclei near the surface of the fertilized egg. Hence, the skilled person is able to determine the stage of the assembly of the pronuclei as well as the presence of the pronuclei of a zygote by e.g. polarized light microscopy.

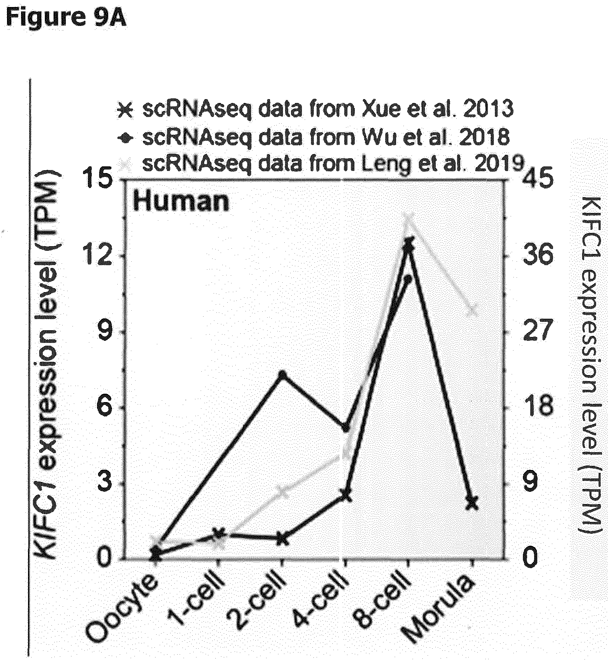

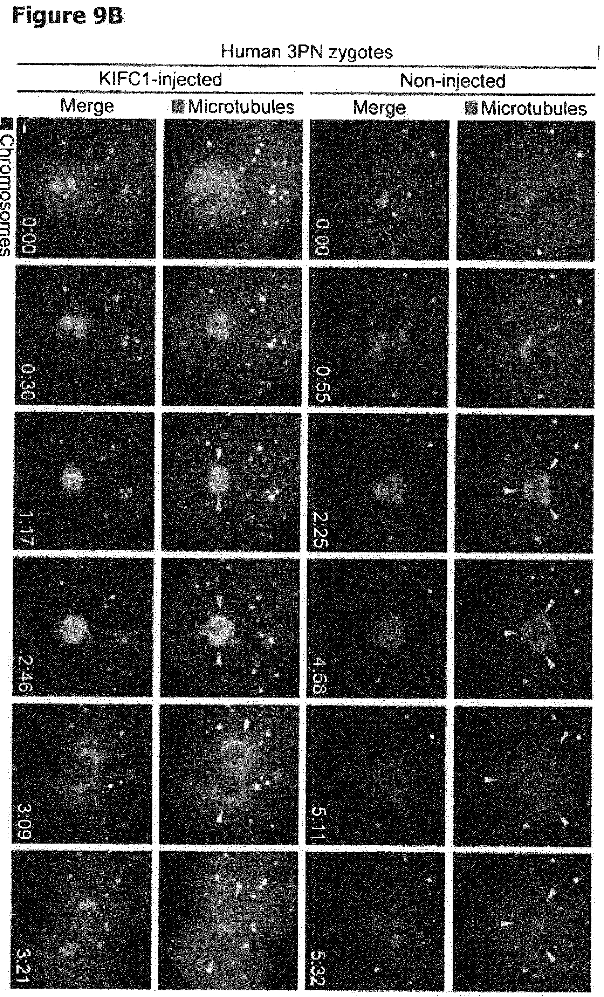

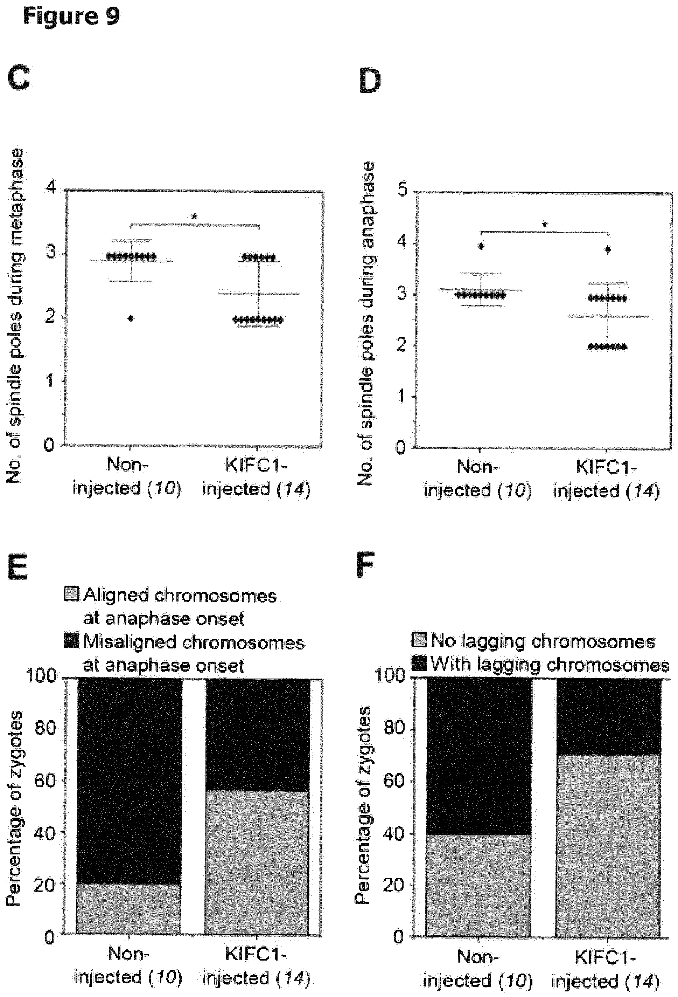

Importantly, the KIFC1/HSET may stabilize the mitotic spindle of the human zygote. Data for such a stabilization is provided in **Fig.** 9 and discussed in detail below. For example, the human zygote with the stabilized mitotic spindle has a higher probability of having a bipolar spindle than a non-stabilized mitotic spindle, preferably wherein the non-stabilized spindle is a multipolar or a disorganized spindle. The skilled person may assess or detect the stabilization of the mitotic spindle by spindle polarity morphology using fluorescence microscopy or polarized light microscopy. In other words, the human zygote with introduced KIFC1/HSET has a higher probability of forming a bipolar mitotic spindle compared to the human zygote without introduced KIFC1/HSET. Specifically, the human zygote with introduced KIFC1/HSET has at least a 5% higher probability of having a bipolar mitotic spindle compared to the human zygote without introduced KIFC1/HSET, preferably at least 10%, more preferably at least 15%, more preferably at least 20%, more preferably at least 25%, and even more preferably at least 35%. The human zygote with introduced KIFC1/HSET has at least 1.05 fold higher probability of having a bipolar mitotic spindle compared to the human zygote without introduced KIFC1/HSET, preferably at least 1.1 fold, more preferably at least 1.15 fold, more preferably at least 1.2 fold, more preferably at least 1.25 fold, and even more preferably at least 1.35 fold.

The information provided above regarding the probability of having a bipolar spindle, applies equally to this aspect of the disclosure. As disclosed herein, the skilled person is aware how to determine the presence of a bipolar spindle. Vice versa, the human zygote without introduced KIFC1/HSET may have a lower probability of having a bipolar mitotic spindle compared to the human zygote with introduced KIFC1/HSET. In particular, the human zygote without introduced KIFC1/HSET may have at least a 5% lower probability of having a bipolar mitotic spindle compared to the human zygote with introduced KIFC1/HSET, preferably at least 10%, more preferably at least 15%, more preferably at least 20%, more preferably at least 25%, and even more preferably at least 35%. The human zygote with introduced KIFC1/HSET has a lower probability of having a multipolar or disorganized mitotic spindle compared to the human zygote without introduced KIFC1/HSET. In particular, the human zygote with introduced KIFC1/HSET has at least a 5% lower probability of having a multipolar or disorganized mitotic spindle compared to the human zygote without introduced KIFC1/HSET, preferably at least 10%, more preferably at least 15%, more preferably at least 20%, more preferably at least 25%, and even more preferably at least 35%. Vice versa, the human zygote without introduced KIFC1/HSET may have a higher probability of having a multipolar or disorganized mitotic spindle compared to the human zygote with introduced KIFC1/HSET. Especially, the human zygote without introduced KIFC1/HSET may have at least a 5% higher probability of having a multipolar or disorganized mitotic spindle compared to the human zygote with introduced KIFC1/HSET, preferably at least 10%, more preferably at least 15%, more preferably at least 20%, more preferably at least 25%, and even more preferably at least 35%. The human zygote without introduced KIFC1/HSET may have at least a 1.05 fold higher probability of having a multipolar or disorganized mitotic spindle compared to the human zygote with introduced KIFC1/HSET, preferably at least 1.1 fold, more preferably at least 1.15 fold, more preferably at least 1.2 fold, more preferably at least 1.25 fold, and even more preferably at least 1.35 fold.

The skilled person may assess or detect the stabilization of the meiotic spindle by spindle polarity morphology, preferably wherein the spindle polarity morphology of a stabilized meiotic spindle may have higher probability of being a bipolar spindle than a non-stabilized meiotic spindle, more preferably wherein the non-stabilized spindle may be a multipolar or a disorganized spindle.

In **Fig.** 8, KIFC1/HSET is introduced into the human oocyte and wherein the introduction of KIFC1/HSET showed a significantly lower duration of spindle pole instability, lower frequency of misaligned chromosomes and lagging chromosomes. Based on this data, it is expected that KIFC1/HSET shows the same behavior in zygotes. Furthermore, zygotes frequently assembled multipolar spindles, which are not viable and poses a large burden on reproductive medicine. **Fig. 9** demonstrates that the introduction of KIFC1/HSET protein into human multipolar (e.g. 3 pronuclei; 3PN) zygotes significantly reduces multipolar spindle assembly and/or promoted bipolar spindle assembly (**Fig.** 9, B to D). The skilled person is aware that 3PN is an example of a multipolar spindle. Moreover, the introduction of KIFC1/HSET protein increased the frequency of zygotes without lagging chromosomes during anaphase from 40% to 71% (**Fig.** 9F). This corresponds to an increase of 77.5% or 1.775-fold. Thus, introducing exogenous KIFC1 increased the efficiency of bipolar spindle assembly and reduced the risk of aneuploidy in human zygotes. Furthermore, introduction of KIFC1/HSET protein increased the frequency of zygotes with aligned chromosomes at anaphase onset from 20% to 57% (**Fig.** 9E). This corresponds to an increase of 185 % or 2.85-fold. Hence, it is expected and shown that the introduction of KIFC1/HSET leads to a lower duration of spindle pole instability in human zygotes, a higher proportion of human zygotes with aligned chromosomes at anaphase onset, and a higher proportion of human zygotes without lagging chromosomes compared to human zygotes without introduced KIFC1/HSET.

In an instance, the human zygote with introduced KIFC1/HSET has at least a 5% higher probability of aligned chromosomes at anaphase onset in comparison to the zygote without introduced KIFC1/HSET, preferably wherein the probability is at least 10%, more preferably 20%, even more preferably 30%, even more preferably 40%, even more preferably 50%, even more preferably 60%, even more preferably 70%, even more preferably 80%, even more preferably 90%, even more preferably 100%, even more preferably 110%, even more preferably 120%, even more preferably 130%, even more preferably 140%, even more preferably 150%, even more preferably 160%, even more preferably 170%, and even more preferably 180% higher in comparison to the zygote without introduced KIFC1/HSET. Preferably, the probability is at most 800%. It is also contemplated that the human zygote with introduced KIFC1/HSET has at least a 1.05 fold higher probability of aligned chromosomes at anaphase onset in comparison to the zygote without introduced KIFC1/HSET, preferably wherein the probability is at least 1.1 fold, more preferably at least 1.2 fold, even more preferably at least 1.3 fold, even more preferably at least 1.4 fold, even more preferably at least 1.5 fold, even more preferably at least 1.7 fold, even more preferably at least 1.9 fold, even more preferably at least 2.0 fold, even more preferably at least 2.2 fold, even more preferably at least 2.4 fold, even more preferably at least 2.6 fold, even more preferably at least 2.7 fold, and even more preferably at least 2.8 fold higher in comparison to the zygote without introduced KIFC1/HSET.

In another instance, the human zygote with introduced KIFC1/HSET has at least a 5% higher probability of non-lagging chromosomes in comparison to the zygote without introduced KIFC1/HSET, preferably wherein the probability is at least 10%, more preferably 20%, even more preferably 30%, even more preferably 40%, even more preferably 50%, even more preferably 60%, and even more preferably 70% higher in comparison to the zygote without introduced KIFC1/HSET. It is also contemplated that the human zygote with introduced KIFC1/HSET has at least a 1.05 fold higher probability of non-lagging chromosomes in comparison to the zygote without introduced KIFC1/HSET, preferably wherein the probability is at least 1.1 fold, more preferably at least 1.2, even more preferably at least 1.3 fold, even more preferably at least 1.4 fold, even more preferably at least 1.5 fold, even more preferably at least 1.6 fold, and even more preferably at least 1.7 fold higher in comparison to the zygote without introduced KIFC1/HSET.

In another instance, a suitable amount of (i) the human KIFC1/HSET protein or (ii) mRNA encoding the human KIFC1/HSET is introduced into the human zygote. As already disclosed above, the skilled person is able to determine a suitable amount, e.g. by performing a titration experiment. Furthermore, the titration experiment may detect spindle morphology disturbances, when too much KIFC1/HSET is introduced into the oocyte, which can be assessed by fluorescence microscopy or polarized light microscopy. One sign of spindle morphology disturbance is the excessive bundling of spindle microtubules, as for example shown in **Fig.** 3G and as described in Example 2 herein. At the same time, introducing too little KIFC1/HSET does not improve the probability of showing bipolar spindles. Exemplary suitable amount of the human KIFC1/HSET protein ranges between 1-250 pg per zygote, preferably 2-150 pg, more preferably 3-100 pg, more preferably 4-80 pg, more preferably 5-50 pg, even more preferably 6-30 pg, even more preferably 7-20 pg, even more preferably 8-15 pg, and most preferably around 10 pg.

Exemplary suitable amount of mRNA encoding the human KIFC1/HSET may be between 1-4000 fg per zygote, preferably 5-3000 fg, more preferably 20-2500 fg, more preferably 35-2000 fg, more preferably 50-1900 fg, even more preferably 60-1700 fg, even more preferably 70-1500 fg, even more preferably 100-1000 fg, and most preferably 140-750 fg. In addition, a (i) KIFC1/HSET protein or (ii) mRNA encoding the KIFC1/HSET for use in a method suitable for stabilizing the mitotic spindle of a human zygote is disclosed herein. Preferably the KIFC1/HSET is introduced into the human zygote. For example, the and/or the KIFC1/HSET protein may be recombinantly produced KIFC1/HSET protein. Aspects relating to (i) the KIFC1/HSET protein or (ii) mRNA encoding the KIFC1/HSET and/or a suitable amount of KIFC1/HSET protein or (ii) mRNA encoding the KIFC1/HSET are further defined above. Similarly, aspects regarding the introduction of (i) the KIFC1/HSET protein or (ii) mRNA encoding the KIFC1/HSET, the stabilization of the mitotic spindle and/or the human zygote are disclosed herein and apply equally to said part of the disclosure.

Ideally, the method of lowering the probability aneuploidy by introducing (i) KIFC1/HSET protein or (ii) mRNA encoding the KIFC1/HSET into a human zygote is an assisted reproductive technology. Similarly, the method suitable for stabilizing the mitotic spindle of a human zygote by the (i) KIFC1/HSET protein or (ii) mRNA encoding the KIFC1/HSET is an assisted reproductive technology. The skilled person is aware of 'assisted reproductive technology' in general and knows that such technologies comprise medical procedures used primarily to address infertility. Hence, it involves procedures such as in vitro fertilization (IVF), intracytoplasmic sperm injection (ICSI), which are not part of the invention.

The present disclosure also relates to a non-naturally occurring human zygote (which is not being part of the invention), wherein a (i) KIFC1/HSET protein or (ii) KIFC1/HSET mRNA has been introduced into an *in vitro* fertilized human zygote, thereby obtaining the non-naturally occurring zygote. The KIFC1/HSET protein may be a recombinantly produced KIFC1/HSET protein. Ideally, (i) the KIFC1/HSET protein or (ii) mRNA encoding the KIFC1/HSET stabilizes the mitotic spindle of the zygote. Furthermore, aspects regarding the introduction of (i) the KIFC1/HSET protein or (ii) mRNA encoding the KIFC1/HSET, the KIFC1/HSET protein or (ii) mRNA encoding the KIFC1/HSET, the stabilization of the mitotic spindle, and/or a suitable amount of KIFC1/HSET protein or (ii) mRNA encoding the KIFC1/HSET are disclosed herein and apply equally to this disclosure. Furthermore, the disclosure relates to a device for injection comprising (i) KIFC1/HSET protein or (ii) mRNA encoding the KIFC1/HSET suitable for introduction into a human zygote *in vitro.* Ideally, the (i) the KIFC1/HSET protein or (ii) mRNA encoding the KIFC1/HSET stabilizes the mitotic spindle of the zygote. In aspects regarding the introduction of (i) the KIFC1/HSET protein or (ii) mRNA encoding the KIFC1/HSET, the KIFC1/HSET protein or (ii) mRNA encoding the KIFC1/HSET, the stabilization of the mitotic spindle, the human zygote, and/or a suitable amount of KIFC1/HSET protein or (ii) mRNA encoding the KIFC1/HSET are discussed above and apply equally to this part of the disclosure.

Moreover, the present disclosure relates to a device for injection comprising KIFC1/HSET protein or KIFC1/HSET mRNA suitable for stabilizing the mitotic spindle a human zygote *in vitro.* The KIFC1/HSET protein or KIFC1/HSET mRNA is introduced into the human zygote. Aspects regarding the introduction of (i) the KIFC1/HSET protein or (ii) mRNA encoding the KIFC1/HSET, the KIFC1/HSET protein or (ii) mRNA encoding the KIFC1/HSET, the stabilization of the mitotic spindle, the human zygote, and/or a suitable amount of KIFC1/HSET protein or (ii) mRNA encoding the KIFC1/HSET are discussed above and apply equally to this part of the disclosure.

In addition, the present disclosure relates to a complex comprising (i) a KIFC1/HSET protein and (ii) a human meiotic spindle, wherein the KIFC1/HSET protein is introducible into a human oocyte by an *in vitro* method, preferably by microinjection and/or electroporation, more preferably by microinjection; and wherein the complex is detectable by fluorescence microscopy. Additionally, the present disclosure relates to a complex comprising (i) a KIFC1/HSET protein and (ii) a human mitotic spindle, wherein the KIFC1/HSET protein is introducible into a human zygote by an *in vitro* method, preferably by microinjection and/or electroporation, more preferably by microinjection; and wherein the complex is detectable by fluorescence microscopy. Specifically, the present disclosure relates to a complex comprising (i) a KIFC1/HSET protein and (ii) a human meiotic spindle, wherein the KIFC1/HSET protein has been introduced into a human oocyte by an *in vitro* method, in particular wherein the KIFC1/HSET protein has been introduced by microinjection and/or electroporation, preferably by microinjection; wherein the complex is detectable by fluorescence microscopy. Similarly, the present disclosure relates to a complex comprising (i) a KIFC1/HSET protein and (ii) a human mitotic spindle, wherein the KIFC1/HSET protein has been introduced into a human zygote by an *in vitro* method, in particular wherein the KIFC1/HSET protein has been introduced by microinjection and/or electroporation, preferably by microinjection; wherein the complex is detectable by fluorescence microscopy. As disclosed herein, microinjection is preferably performed according to the methods as described herein. Alternatively, electroporation may be performed, e.g. according to the methods as disclosed in (114).

The human meiotic and mitotic spindle are known to the skilled person. The human mitotic spindle has been extensively studied and generally includes the spindle microtubules, associated proteins, which include kinesin and dynein molecular motors, condensed chromosomes, and centrosomes. The more detailed organization of microtubules and associated proteins in the mitotic spindle is known to the skilled person and for example detailed in reference 116. Overall, it is assumed that the meiotic spindle and the mitotic spindle are organized in a similar way. However, while centrosomes are the major sites of microtubule nucleation forming the two poles of the mitotic spindle, human meiotic spindles lack centrioles (115). Furthermore, human oocytes assemble a spindle in a lengthy process that does not involve prominent aMTOCs (acentriolar microtubule organizing centers), which are the major organizing centers in mouse oocytes. The skilled person is aware of these differences (115).

For example, the skilled person is aware that the spindle is detectable by an anti-alpha-tubulin antibody. Furthermore, the skilled person also knows that the KIFC1/HSET is detectable by an anti-HSET-C antibody. The skilled person is aware of various antibodies, which are commercially available to detect the meiotic or mitotic spindle as well as KIFC1/HSET. For example, the spindle is detectable by a rat anti-alpha-tubulin antibody (MCA78G; Bio-Rad) and/or the KIFC1/HSET is detectable by rabbit anti-HSET-C (20790-1-AP; Proteintech), as used herein e.g. in Example 2. It is noted that the formation of the complex is detectable in an oocyte or zygote. However, the formation of the complex is typically not assessed as this would require sacrificing the oocyte or zygote for imaging. Instead, the parthenote or the aMTOC-free and KIFC1/HSET depleted mouse oocytes can be used as mimics and the formation of the complex can be assessed therein.

The complex may be specifically detected by fluorescence microscopy and/or polarized light microscopy. Ideally, the KIFC1/HSET protein is recombinantly produced KIFC1/HSET protein. Methods of recombinantly producing KIFC1/HSET protein are disclosed herein and apply equally to this part of the disclosure. Furthermore, aspects regarding the KIFC1/HSET protein also disclosed herein and also apply equally to this part of the disclosure. Ideally, the KIFC1/HSET protein stabilizes the human meiotic or mitotic spindle. The complex may stabilize meiotic or mitotic spindle. The stabilized spindle may have at least a 5% higher probability of being a bipolar spindle than a multipolar or disorganized spindle, preferably at least 10%, more preferably at least 15%, more preferably at least 20%, more preferably at least 25%, and even more preferably at least 35%. In an instance, the complex stabilizes meiotic or mitotic spindle. In an instance, the stabilized spindle has at least a 1.05 fold higher probability of being a bipolar spindle than a multipolar or disorganized spindle, preferably at least 1.1 fold, more preferably at least 1.15 fold, more preferably at least 1.2 fold, more preferably at least 1.25 fold, and even more preferably at least 1.35 fold.

Complex formation may lead to a meiotic or mitotic spindle having a higher probability of being a bipolar spindle than without complex formation. In other words, upon complex formation the meiotic or mitotic spindle may have an increased stability than without complex formation. An increased stability of the spindle may be assessed by the probability of the formation of a bipolar spindle in comparison to a multipolar or a disorganized spindle. The increased stability of the spindle may be present when the probability of a bipolar spindle increases in comparison to an oocyte or zygote, wherein no complex formation occurred. In an instance, the stabilization of the spindle is evaluated by spindle polarity morphology. A non-stabilized spindle may be a multipolar or a disorganized spindle. As noted above complex formation may be assessable fluorescence microscopy or polarized light microscopy.

Additionally, the present disclosure relates to an *in vitro* method of introducing (i) KIFC1/HSET protein or (ii) mRNA encoding KIFC1/HSET into a human parthenote. As known in the art, a parthenote is an organism produced from an unfertilized oocyte, which is incapable of developing beyond the early embryonic stages upon artificial activation so that the egg cell divides. Hence, parthenotes are powerful research tools. Aspects relating to (i) the KIFC1/HSET protein or (ii) mRNA encoding the KIFC1/HSET disclosed herein apply equally to this part of the disclosure. In one aspect, a suitable amount of (i) the human KIFC1/HSET protein or (ii) mRNA encoding the human KIFC1/HSET may be introduced into the human parthenote. Said suitable amount may assessed by a titration experiment detecting spindle morphology disturbances using fluorescence microscopy or polarized light microscopy. For example, the suitable amount of the human KIFC1/HSET protein ranges between 1-250 pg per parthenote, preferably 2-150 pg, more preferably 3-100 pg, more preferably 4-80 pg, more preferably 5-50 pg, even more preferably 6-30 pg, even more preferably 7-20 pg, even more preferably 8-15 pg, and most preferably around 10 pg. Exemplary suitable amount of mRNA encoding the human KIFC1/HSET may be between 1-4000 fg per parthenote, preferably 5-3000 fg, more preferably 20-2500 fg, more preferably 35-2000 fg, more preferably 50-1900 fg, even more preferably 60-1700 fg, even more preferably 70-1500 fg, even more preferably 100-1000 fg, and most preferably 140-750 fg. Ideally, the introduction of KIFC1/HSET into the parthenote increases the probability of having a bipolar spindle in comparison to a parthenote without introduced KIFC1/HSET. Even more ideally, the KIFC1/HSET stabilizes the mitotic spindle of the human parthenote. In another aspect, the human parthenote with the stabilized mitotic spindle has a higher probability of being a bipolar spindle than a non-stabilized mitotic spindle, preferably wherein the non-stabilized spindle is a multipolar or a disorganized spindle. The skilled person is aware of method detecting the spindle, such as assessing the spindle morphology. For example, the bipolar spindle may be assessed or detected by fluorescence microscopy or polarized light microscopy. The human parthenote with introduced KIFC1/HSET has a higher probability of having a bipolar mitotic spindle compared to the human parthenote without introduced KIFC1/HSET. Specifically, the human parthenote with introduced KIFC1/HSET may have at least a 5% higher probability of having a bipolar mitotic spindle compared to the human parthenote without introduced KIFC1/HSET, preferably at least 10%, more preferably at least 15%, more preferably at least 20%, more preferably at least 25%, and even more preferably at least 35%. It is also contemplated that the human parthenote with introduced KIFC1/HSET has at least a 1.05 fold higher probability of having a bipolar mitotic spindle compared to the human parthenote without introduced KIFC1/HSET, preferably at least 1.1 fold, more preferably at least 1.15 fold, more preferably at least 1.2 fold, more preferably at least 1.25 fold, and even more preferably at least 1.35 fold.

Vice versa, the human parthenote without introduced KIFC1/HSET may have a lower probability of having a bipolar mitotic spindle compared to the human parthenote with introduced KIFC1/HSET. Specifically, the human parthenote without introduced KIFC1/HSET may have at least a 5% lower probability of having a bipolar mitotic spindle compared to the human parthenote with introduced KIFC1/HSET, preferably at least 10%, more preferably at least 15%, more preferably at least 20%, more preferably at least 25%, and even more preferably at least 35%. Moreover, the human parthenote with introduced KIFC1/HSET may have a lower probability of having a multipolar or disorganized mitotic spindle compared to the human parthenote without introduced KIFC1/HSET. Specifically, the human parthenote without introduced KIFC1/HSET may have at least a 5% higher probability of having a multipolar or disorganized mitotic spindle compared to the human parthenote with introduced KIFC1/HSET, preferably at least 10%, more preferably at least 15%, more preferably at least 20%, more preferably at least 25%, and even more preferably at least 35%. Furthermore, the human parthenote without introduced KIFC1/HSET may have at least a 1.05 fold higher probability of having a multipolar or disorganized mitotic spindle compared to the human parthenote with introduced KIFC1/HSET, preferably at least 1.1 fold, more preferably at least 1.15 fold, more preferably at least 1.2 fold, more preferably at least 1.25 fold, and even more preferably at least 1.35 fold. In **Fig.** 8, KIFC1/HSET is introduced into the human oocyte and wherein the introduction of KIFC1/HSET showed a significantly lower duration of pole instability, lower frequency of misaligned chromosomes and lagging chromosomes. Based on this data, it is expected that KIFC1/HSET shows the same behavior in human parthenotes. Hence, it is expected that the introduction of KIFC1/HSET leads to a significantly lower duration of pole instability in human parthenotes. In one aspect, the human parthenote without introduced KIFC1/HSET has at least a 5% higher probability of aligned chromosomes at anaphase onset in comparison to the parthenote without introduced KIFC1/HSET, preferably wherein the probability is at least at least 10%, more preferably at least 20%, even more preferably at least 30%, and even more preferably at least 40% higher in comparison to the parthenote without introduced KIFC1/HSET. It is also contemplated that the human parthenote without introduced KIFC1/HSET has at least a 1.05 fold higher probability of aligned chromosomes at anaphase onset in comparison to the parthenote without introduced KIFC1/HSET, preferably wherein the probability is at least 1.1 fold, more preferably at least 1.2 fold, even more preferably at least 1.3 fold, and even more preferably at least 1.4 fold higher in comparison to the parthenote without introduced KIFC1/HSET. In another aspect, the human parthenote without introduced KIFC1/HSET has at least a 5% higher probability of non-lagging chromosomes in comparison to the parthenote without introduced KIFC1/HSET, preferably wherein the probability is at least 10%, more preferably 20%, even more preferably 30%, and even more preferably 40% higher in comparison to the parthenote without introduced KIFC1/HSET. It is also contemplated that the human parthenote without introduced KIFC1/HSET has at least a 1.05 fold higher probability of non-lagging chromosomes in comparison to the parthenote without introduced KIFC1/HSET, preferably wherein the probability is at least 1.1 fold, more preferably at least 1.2, even more preferably at least 1.3 fold, and even more preferably at least 1.4 fold higher in comparison to the parthenote without introduced KIFC1/HSET.

Finally, the present disclosure relates to a human parthenote, wherein a (i) KIFC1/HSET protein or (ii) KIFC1/HSET mRNA has been introduced into the parthenote. Ideally, the (i) KIFC1/HSET protein or (ii) KIFC1/HSET mRNA has been introduced into the human parthenote *in vitro.* It is further disclosed, that the KIFC1/HSET protein is recombinantly produced KIFC1/HSET protein. For example, the human parthenote may express the KIFC1/HSET at least 2-fold less than a mouse parthenote, preferably at least 4-fold less, more preferably at least 6-fold less, even more preferably at least 10-fold less, and even more preferably at least 20-fold less, even more preferably at least 30-fold less, even more preferably at least 40-fold less, even more preferably at least 50-fold less, and even more preferably at least 60-fold less. It is also contemplated that the human parthenote expresses the KIFC1/HSET at least 2-fold less than HeLa cells, preferably at least 4-fold less, more preferably at least 6-fold less, even more preferably at least 10-fold less, and even more preferably at least 20-fold less. With regard to the expression of KIFC1/HSET as well as methods to detect KIFC1/HSET the information provided herein provide equally to said part of the disclosure. For example, the expression of the KIFC1/HSET may be assessed by the band intensity on Western Blot using the same KIF1/HSET antibody as primary antibody, preferably wherein the band intensity is assessed using a rabbit anti-HSET antibody, more preferably the rabbit anti-HSET-N antibody ab172620 (Abcam). Preferred embodiments with regard to the Western Blot are detailed herein and apply equally.

With regard to all embodiments herein, the KIFC1/HSET protein or the mRNA encoding KIFC1/HSET may have been synthesized *in vitro.* The skilled person is aware of several methods to synthesize proteins and mRNA in vitro. Furthermore, KIFC1/HSET may be ordered from a commercial source or obtained in a laboratory in house. The example section herein provides ample data on how to express and purify KIFC1/HSET to obtain KIFC1/HSET, which has been synthesized in vitro.

### Description of the Figures

**Fig. 1****. Human oocytes focus the spindle poles with dynein-dynactin, but lack an additional mechanism that stabilizes the spindles.** (**A**) Immunofluorescence images of human oocytes fixed at different stages of meiosis. Green, dynactin (P150); magenta, microtubules (a-tubulin); blue, chromosomes (Hoechst). (**B**) Immunofluorescence images of a human MI spindle. Yellow, NUMA; magenta, dynactin (P150); cyan, LIS1. The graph is the fluorescence profile of NUMA, dynactin and LIS1 across the spindle pole along the direction of the yellow arrow. (**C**) Immunofluorescence images of MI spindles from human oocytes treated with BSA or P150-CC1-His (dynein inhibitor). Green, NUMA; magenta, microtubules (a-tubulin); blue, chromosomes (Hoechst). Insets are magnifications of regions marked by dashed line boxes. (**D**) Manual scoring of spindle pole defocusing in control and dynein-inhibited human MI oocytes (Fisher's exact test, ***). (**E**) Immunofluorescence images of MI spindles from NUMA-depleted or dynein-inhibited human and aMTOC-free mouse oocytes. Gray, microtubules (a-tubulin); pseudocolor, directionality. (**F**) Manual scoring of spindle midzone organization in NUMA-depleted or dynein-inhibited human and aMTOC-free mouse MI oocytes (Fisher's exact test, ****). (**G**) Manual scoring of spindle polarity in fixed human and aMTOC-free mouse MI oocytes (Fisher's exact test, ****). (**H**) Manual scoring of spindle polarity in live human oocytes from ref.(*2*) and in aMTOC-free mouse oocytes from this study (Fisher's exact test, ****). The number of analyzed oocytes is specified in italics. Scale bars, 5 µm.

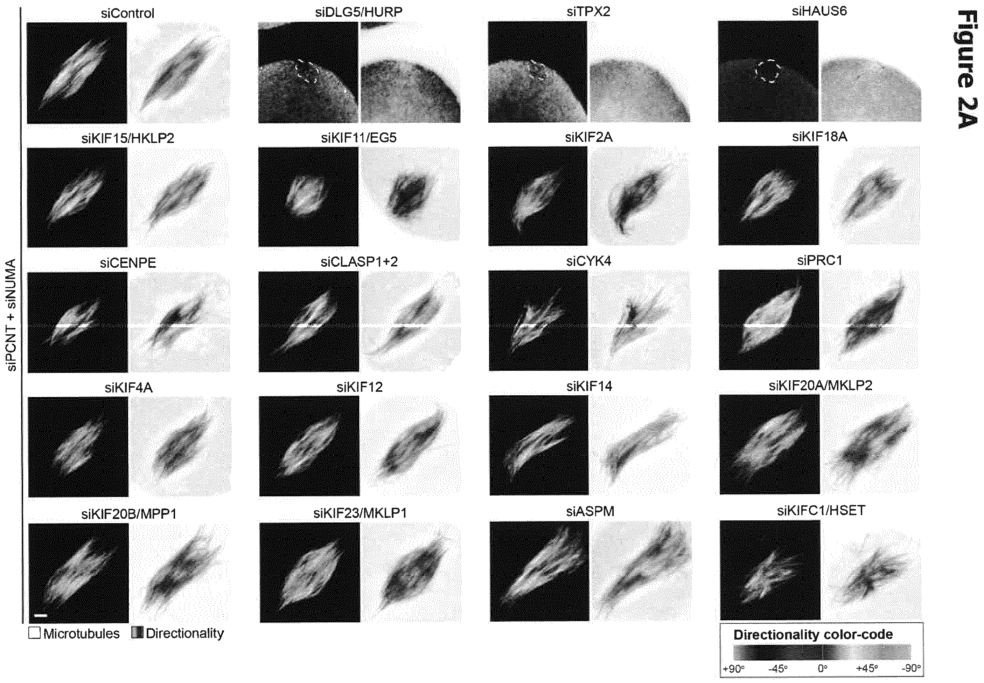

**Fig. 2****. Human oocytes lack the spindle-stabilizing factor kinesin-14 KIFC1/HSET.** (**A**) Immunofluorescence images of MI spindles from aMTOC-free mouse oocytes co-depleted of NUMA and one of the 20 candidate proteins. Gray, microtubules (a-tubulin); pseudocolor, directionality. For oocytes that did not assemble a spindle, chromosomes are outlined by yellow dashed lines. (**B**) On-blot No-Stain protein staining and immunoblots of HeLa cell, human oocyte and mouse oocyte lysates. The amount of HeLa cell lysate or the number of oocytes loaded is indicated above each lane. The corresponding amount of protein loaded is indicated under each lane. Black arrows mark the band corresponding to each protein. (**C**) Immunoblots of control, KIFC1/HSET-depleted and mClover3-HSET-overexpressing mouse oocytes. The number of oocytes loaded is specified in italics. Black arrows mark the band corresponding to each protein. (**D**) Immunofluorescence images of MI spindles from control and KIFC1/HSET-depleted aMTOC-free mouse oocytes. Green, NUMA; magenta, microtubules (a-tubulin); blue, chromosomes (Hoechst). Arrowheads highlight well defined spindle poles. Dashed lines highlight poorly defined spindle poles. (**E** and **F**) Manual scoring (Fisher's exact test, ****) and automated quantification of spindle polarity in control and KIFC1/HSET-depleted aMTOC-free mouse MI oocytes. The number of oocytes analyzed is specified in italics. Scale bars, 5 µm.

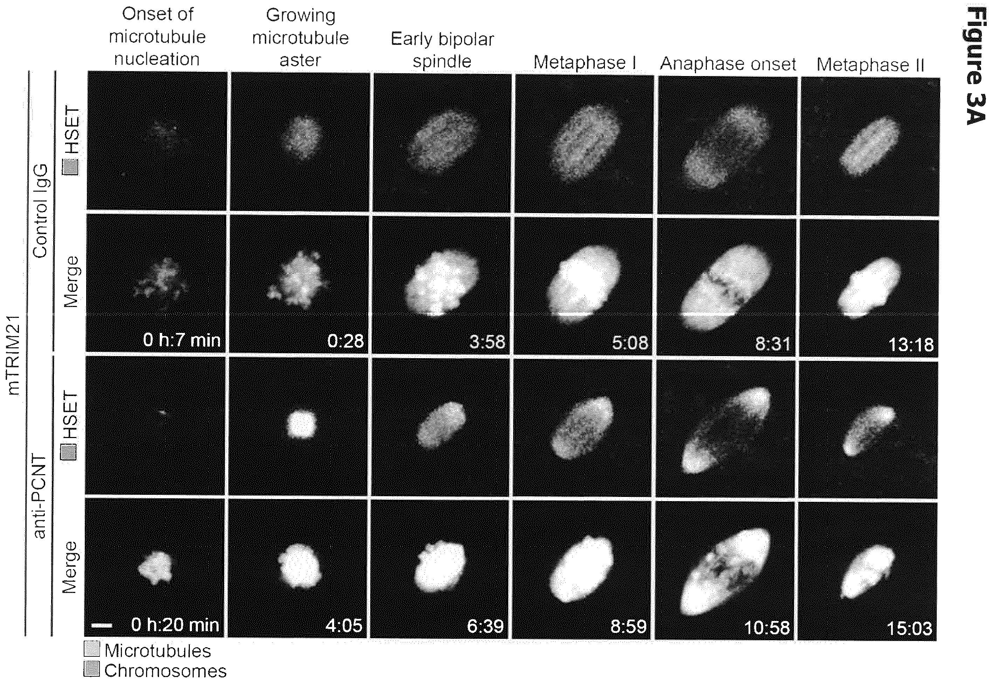

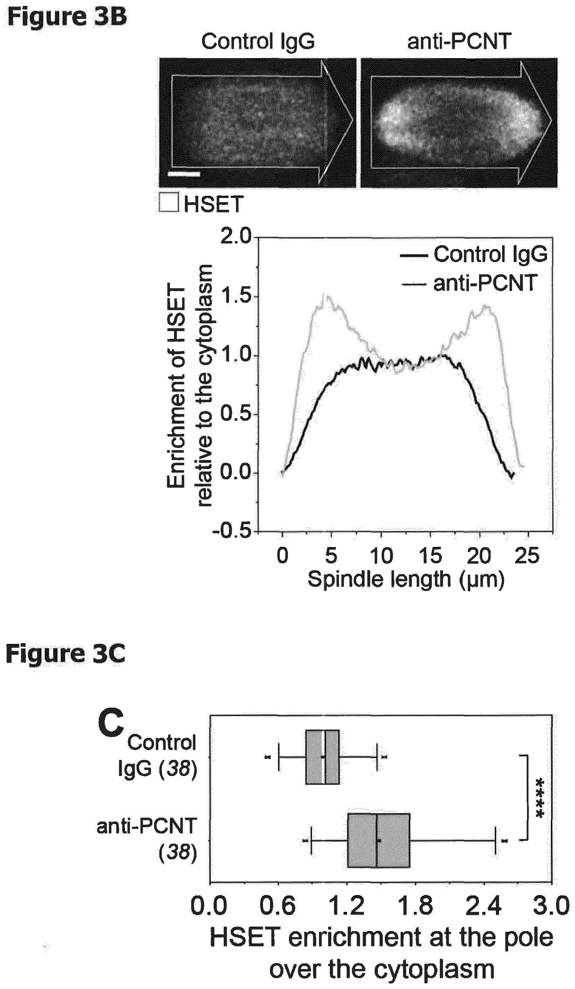

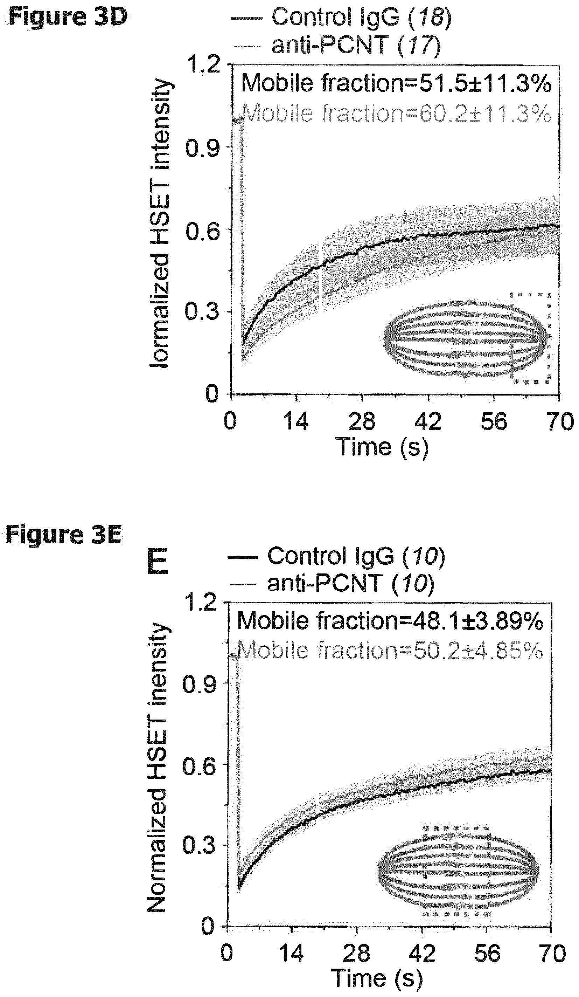

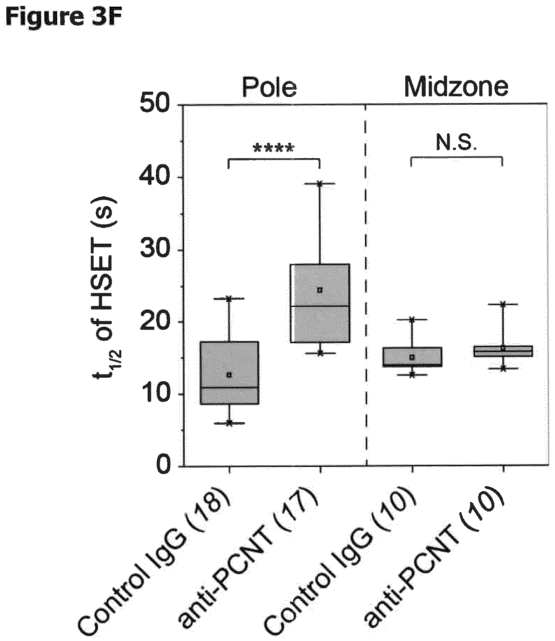

**Fig. 3****. The appropriate level of KIFC1/HSET's crosslinking and sliding activities are required for proper bipolar spindle assembly in aMTOC-free mouse oocytes.** (**A**) Still images from time-lapse movies of wildtype and aMTOC-free mouse oocytes. Green, mClover3-HSET; magenta, microtubules (mScarlet-MAP4-MTBD); blue, chromosomes (H2B-miRFP). Time is given as hours:minutes after NEBD. (**B**) Still images of KIFC1/HSET on MI spindles from wildtype and aMTOC-free mouse oocytes. The graph is the fluorescence profile of KIFC1/HSET across the spindle along the direction of the yellow arrow. (**C**) Quantification of KIFC1/HSET enrichment at the spindle pole over the cytoplasm in wildtype and aMTOC-free mouse MI oocytes. (**D**) Recovery of photobleached KIFC1/HSET at the spindle poles in wildtype and aMTOC-free mouse MI oocytes. (**E**) Recovery of photobleached KIFC1/HSET in the spindle midzone in wildtype and aMTOC-free mouse MI oocytes. (**F**) Half-life of KIFC1/HSET at different regions of MI spindles from wildtype and aMTOC-free mouse oocytes. (**G**) Immunofluorescence images of MI spindles from KIFC1/HSET-depleted aMTOC-free mouse oocytes rescued with HSET-WT or HSET(N593K). Gray, GFP-HSET; green, NUMA; magenta, microtubules (a-tubulin); blue, chromosomes (Hoechst). (**H**) Manual scoring of spindle polarity in aMTOC-free mouse MI oocytes rescued with HSET-WT and HSET(N593K) (Fisher's exact test, ****). (**I**) Quantification of KIFC1/HSET to α-tubulin intensity ratio in bipolar MI spindles from aMTOC-free mouse oocytes rescued with HSET-WT. Error bars (shaded areas) represent SD. The number of analyzed oocytes is specified in italics. Scale bars, 5 µm.

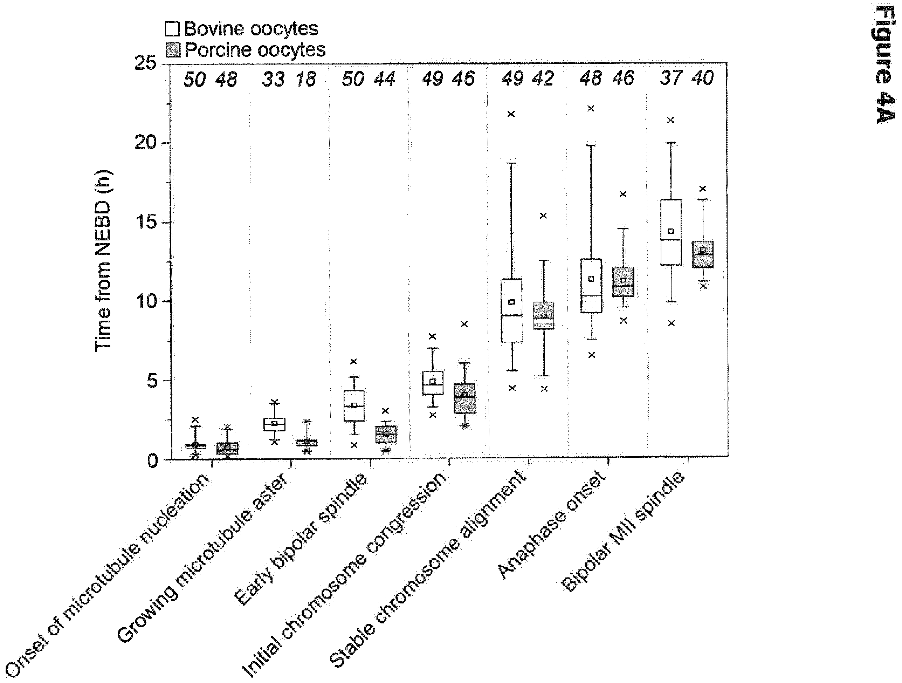

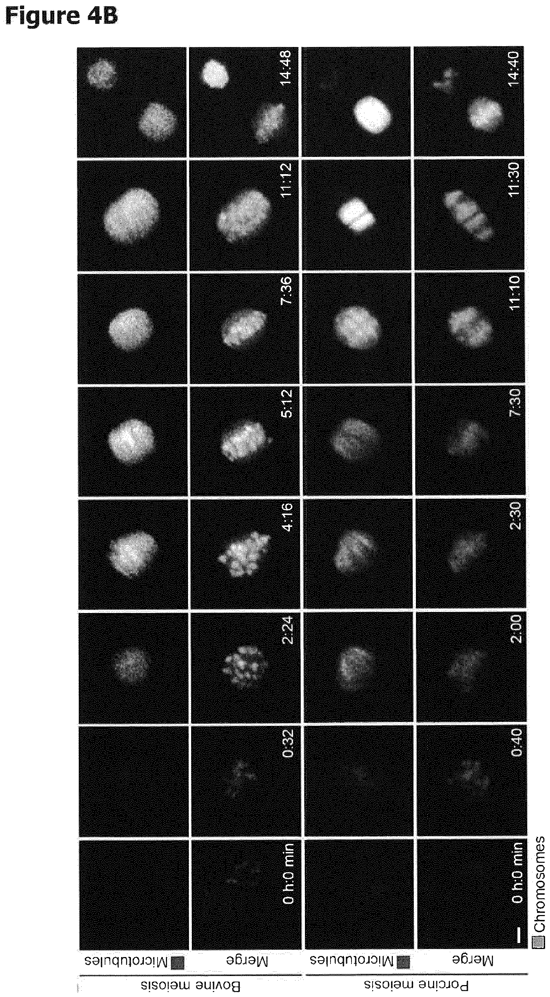

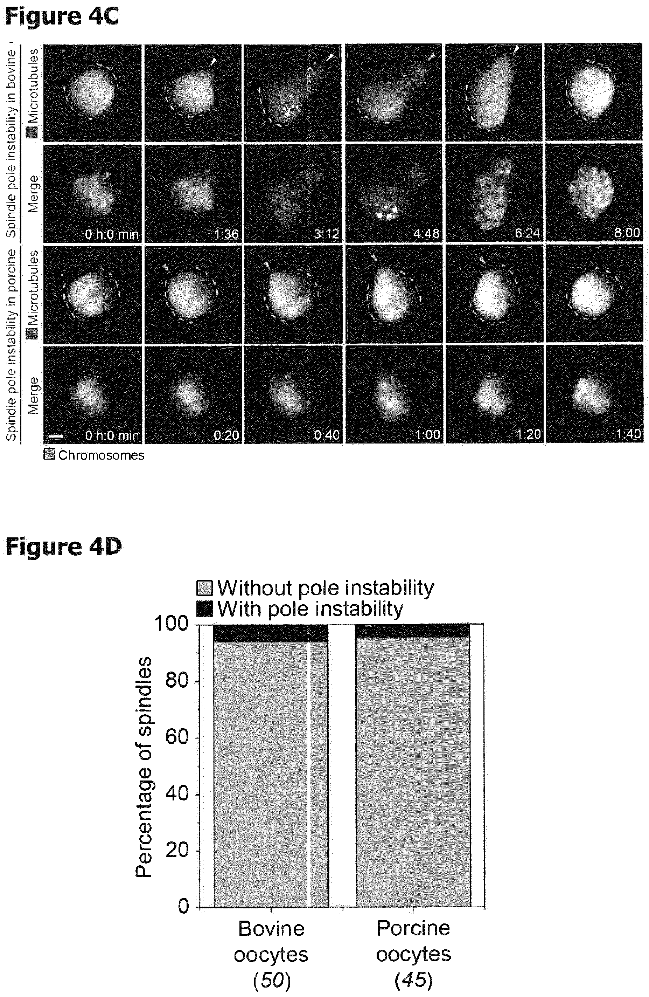

**Fig. 4****. Spindle instability is not a general feature of meiosis I in bovine and porcine oocytes. (A)** Quantification of the timing of different stages of meiosis in bovine and porcine oocytes. (**B**) Still images from time-lapse movies of bovine and porcine oocytes assembling a stable spindle. Green, microtubules (EGFP-MAP4); magenta, chromosomes (H2B-mCherry). Time is indicated as hours:minutes after nuclear envelope breakdown (NEBD). (**C**) Still images from time-lapse movies of bovine and porcine oocytes assembling an unstable spindle. Green, microtubules (EGFP-MAP4); magenta, chromosomes (H2B-mCherry). Time is indicated as hours: minutes after the onset of spindle instability. Dashed lines highlight stable spindle poles. Arrowheads highlight unstable spindle poles. (**D**) Manual scoring of spindle stability in bovine and porcine oocytes. The number of oocytes analyzed is specified in italics. Scale bars, 5 µm.

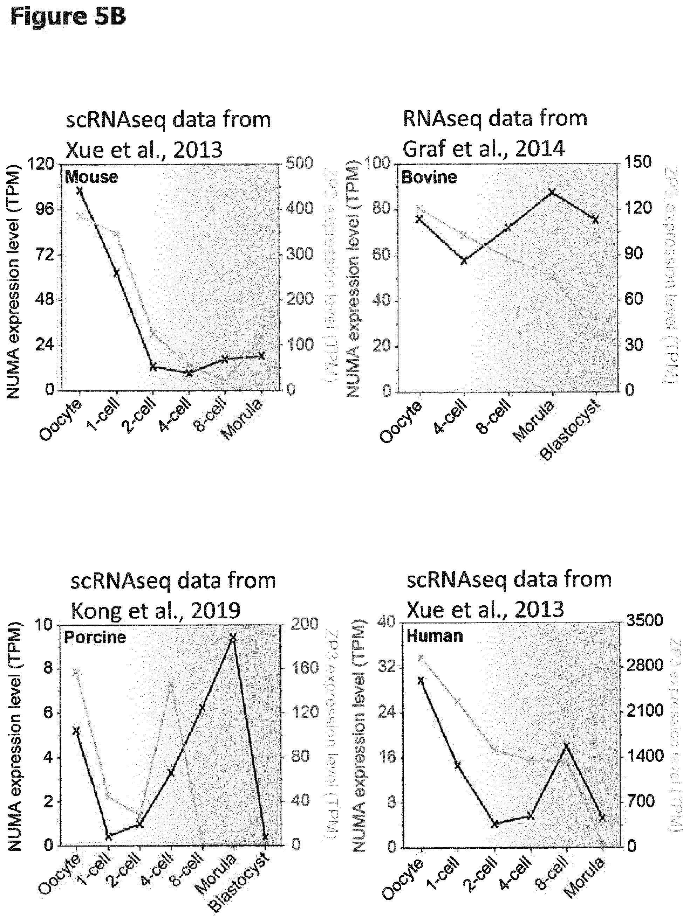

**Fig. 5** **KIFC1/HSET mRNA is barely detectable in human oocytes, but not in oocytes of other mammalian species. (A)** Expression of KIFC1/HSET mRNA in oocytes and preimplantation embryos from mice, cows, pigs and humans. (**B**) Expression of NUMA and ZP3 mRNAs in oocytes and preimplantation embryos from mice, cows, pigs and humans.
**Fig. 6** **Validation of on-blot total protein normalization and the expression of KIFC1/HSET protein in bovine and porcine oocytes. (A)** Immunoblots of HeLa cell, human oocyte and mouse oocyte lysates for canonical housekeeping proteins. The amount of HeLa cell lysate or the number of oocytes loaded is indicated above each lane. Black arrows mark the band corresponding to each protein. (**B**) Quantification of the intensity of canonical housekeeping proteins in different amount of HeLa cell lysate or different number of mouse oocytes. (**C**) Calibration curves for on-blot No-Stain protein staining in **Fig.** 2B. (**D**) On-blot No-Stain protein staining of HeLa cell, mouse oocyte, bovine oocyte and porcine oocyte lysates. 'M.' indicates mouse oocytes; 'B.' indicates bovine oocytes; 'P.' indicates porcine oocytes. The amount of HeLa cell lysate or the number of oocytes loaded is indicated above each lane. The corresponding amount of protein loaded is indicated under each lane. (**E**) Calibration curve for on-blot No-Stain protein staining in (**D**). (**F**) Immunoblots of HeLa cell, mouse oocyte, bovine oocyte and porcine oocyte lysates. 'M.' indicates mouse oocytes; 'B.' indicates bovine oocytes; 'P.' indicates porcine oocytes. The amount of HeLa cell lysate or the number of oocytes loaded is indicated above each lane. Black arrows mark the band corresponding to each protein.

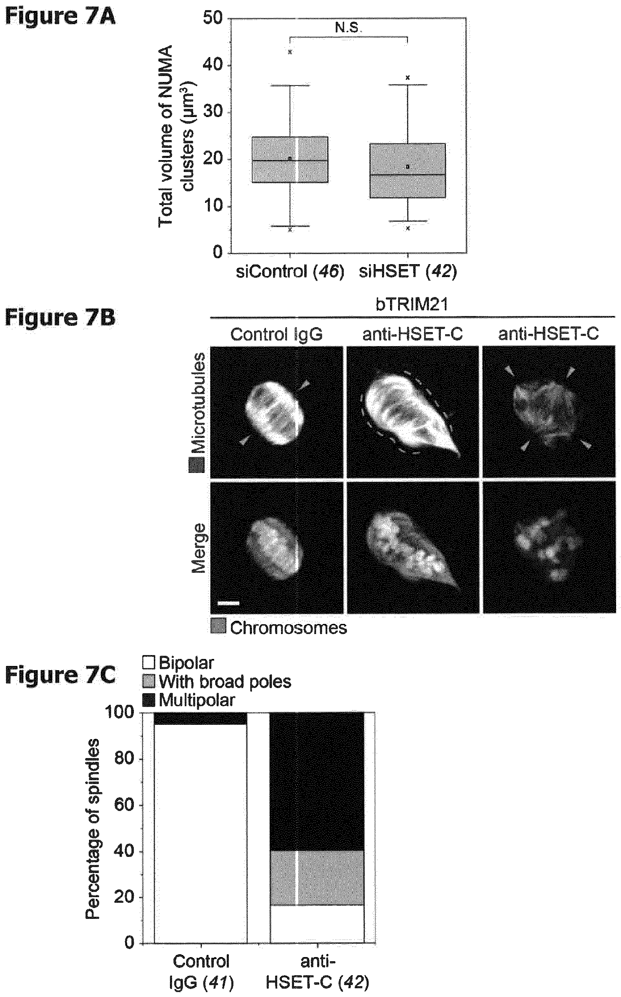

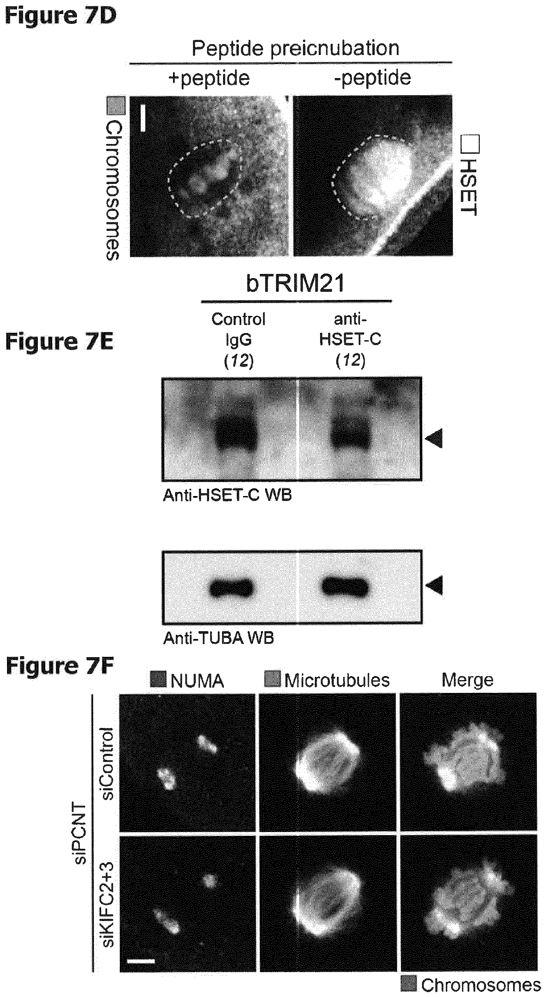

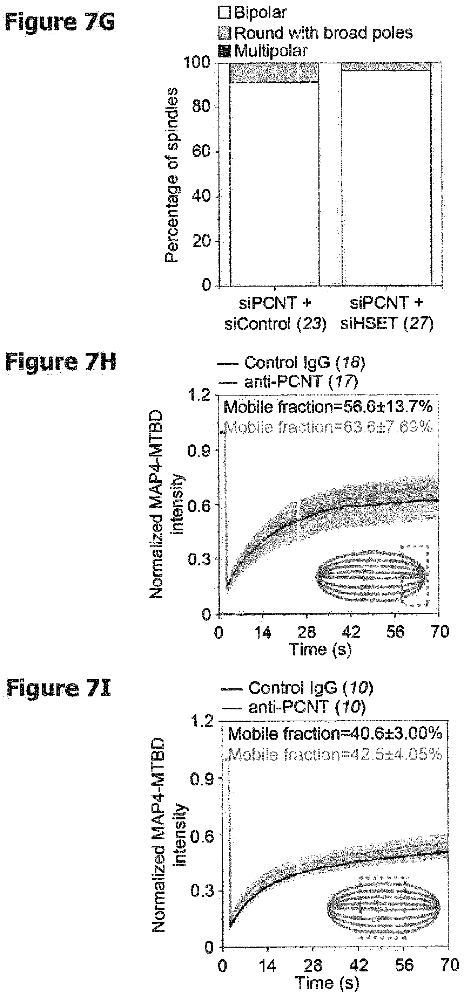

**Fig. 7** **Characterization of KIFC1/HSET in aMTOC-free mouse oocytes and in bovine oocytes. (A)** Quantification of total volume of NUMA clusters in control and KIFC1/HSET-depleted aMTOC-free mouse MI oocytes. (**B**) Immunofluorescence images of MI spindles from control and KIFC1/HSET-depleted bovine oocytes. Green, microtubules (a-tubulin); magenta, chromosomes (Hoechst). Arrowheads highlight spindle poles. (**C**) Manual scoring of spindle polarity in control and KIFC1/HSET-depleted bovine oocytes (Fisher's exact test, ****). Spindles are outlined by yellow dashed lines. (**D**) Immunofluorescence images of MI spindles from bovine oocytes stained with non-preincubated or HSET peptide preincubated HSET-C antibody. Gray, KIFC1/HSET; magenta, chromosomes (Hoechst). (**E**) Immunoblots of control and KIFC1/HSET-depleted bovine oocytes. The number of oocytes loaded is specified in italics. Black arrows mark the band corresponding to each protein. (**F**) Immunofluorescence images of MI spindles from control and KIFC2+3-depleted aMTOC-free mouse oocytes. Green, NUMA; magenta, microtubules (a-tubulin); blue, chromosomes (Hoechst). (**G**) Manual scoring of spindle polarity in control and KIFC2+3-depleted aMTOC-free mouse MI oocytes (Fisher's exact test, N.S.). (H) Recovery of photobleached MAP4-MTBD at the spindle poles in wildtype and aMTOC-free mouse MI oocytes. (**I**) Recovery of photobleached MAP4-MTBD in the spindle midzone in wildtype and aMTOC-free mouse MI oocytes. (**J**) Half-life of α-tubulin and MAP4-MTBD at different regions of MI spindles from wildtype and aMTOC-free mouse oocytes. (**K**) Schematic diagram of different domains in KIFC1/HSET and KIFC1/HSET mutants used in this study. (**L**) Immunofluorescence images of MI spindles from aMTOC-free mouse oocytes expressing HSET(ΔMotor), HSET(ΔTail) or HSET(Motor). Green, GFP-HSET; magenta, NUMA; gray, microtubules (a-tubulin); blue, chromosomes (Hoechst). (M) Mechanistic model for spindle pole organization and stability in the absence of aMTOCs. See text for details. The number of analyzed oocytes is specified in italics. Scale bars, 5 µm.
**Fig. 8** **Microinjection of KIFC1/HSET rescues spindle pole instability in human oocytes. (A)** Still images from time-lapse movies of spindle assembly in human oocytes non-injected and injected with mClover3-HSET. Green, microtubules (5'-SiR-tubulin); magenta, chromosomes (SPY555-DNA). Arrowheads highlight spindle poles. Time is indicated as hours:minutes after microtubule nucleation onset. Scale bar, 5 µm. (**B**) Quantification of the time of early spindle bipolarization in human oocytes non-injected and injected with mClover3-HSET. (**C**) Quantification of the duration of pole instability in human oocytes non-injected and injected with mClover3-HSET. (**D**) Manual scoring of misaligned chromosomes in human oocytes non-injected and injected with mClover3-HSET. (**E**) Manual scoring of lagging chromosomes in human oocytes non-injected and injected with mClover3-HSET.
**Fig. 9** **Introduction of exogenous KIFC1 promotes bipolar spindle assembly and accurate chromosome segregation in human zygotes.** (**A**) *KIFC1* expression levels in human oocytes and preimplantatation embryos. (**B**) Still images from time-lapse movies of non-injected and KIFC1-injected human tripronuclear (3PN) zygotes. Green, microtubules (5-SiR-CTX); magenta, chromosomes (SPY555-DNA). Time is given as hours:minutes after nuclear envelope breakdown. (**C**) Quantification of the number of spindle poles during metaphase in non-injected and KIFC1-injected human 3PN zygotes. **P* < 0.05; P=0.0176 (**D**) Quantification of the number of spindle poles during anaphase in non-injected and KIFC1-injected human 3PN zygotes. ***P* < 0.01; P=0.048 (**E**) Quantification of chromosome misalignment at anaphase onset in non-injected and KIFC1-injected human 3PN zygotes. (**F**) Quantification of chromosome missegregation during anaphase in noninjected and KIFC1-injected human 3PN zygotes. Scale bar is 5 µm.

### Examples

The following examples are intended to illustrate the invention further, but are not limited to it. The examples describe technical features, and the invention also relates to combinations of the technical features presented in this section.

### Example 1

### NUMA is required to focus the spindle poles in human oocytes

To investigate how spindle poles are organized during meiosis I in human oocytes, the inventors selected eight candidate proteins for further study. Spindle pole assembly requires the bundling of parallel microtubules by microtubule crosslinking protein(s) (*36, 37*). In addition, microtubule minus-ends can be anchored to MTOCs and/or stabilized by minus-end-binding protein(s) (*36, 37*)*.* Therefore, the inventors analyzed the localization of four minus-end-binding proteins (γ-tubulin, CAMSAP3, KANSL3 and MCRS1) and four microtubule crosslinking proteins (ASPM, EG5, NUMA and TPX2) in human oocytes, as these proteins have been shown to associate with the spindle poles in mitotic cells (*36, 37*) and oocytes from diverse organisms (*2*, *18-20, 26, 28, 32-35*).

As a general minus-end-cap (*38*), γ-tubulin was enriched at the spindle poles in human oocytes (data not shown), consistent with the enrichment of microtubule minus-ends at the poles. In contrast, CAMSAP3, KANSL3, MCRS1 and ASPM localized to the spindle only weakly, and were not enriched at the poles (data not shown), suggesting that they are unlikely to be important for the organization of spindle poles in human oocytes. Notably, whereas EG5 and TPX2 showed diffuse staining at the spindle poles (data not shown), NUMA staining was confined to the extreme region of the poles, where minus ends of individual microtubule bundles converge (data not shown). Imaging of the spindle in a vertical orientation confirmed that NUMA specifically associates with microtubule minus-ends at the spindle poles (data not shown), making it a good candidate for mediating pole focusing in human oocytes.

The inventors further analyzed NUMA localization at different stages of meiosis and found that it marks the poles early, before spindle bipolarization, until metaphase II arrest (data not shown), suggesting that NUMA is recruited as soon as the poles are forming in human oocytes. Interestingly, whereas two clusters of NUMA were detected in oocytes undergoing bi-directional anaphase I, three clusters of NUMA were detected in an oocyte undergoing tri-directional anaphase I (data not shown). These observations provide further evidence that NUMA is related to the organization of spindle poles in human oocytes.

To test directly if NUMA is required for spindle pole organization, the inventors depleted NUMA protein in human oocytes. Most proteins have already been synthesized and deposited in fully grown oocytes, rendering RNAi inefficient (39). Therefore, the inventors used Trim-Away (40) to trigger acute degradation of NUMA protein. NUMA was enriched in the nucleus before nuclear envelope breakdown (NEBD) in control oocytes, and this enrichment was lost upon Trim-Away of NUMA (data not shown). After NEBD, NUMA-depleted oocytes no longer had NUMA at the spindle poles, and importantly, the poles became fully defocused (data not shown). Thus, NUMA is required for pole focusing in human oocytes.

### Humanization of spindle assembly in mouse oocytes

The inventors also investigated NUMA localization in bovine and porcine oocytes, which like human oocytes, lack aMTOCs. NUMA was similarly enriched at the microtubule minus-ends in these non-rodent mammalian oocytes (data not shown). On the other hand, in mouse oocytes, NUMA was hardly enriched at the spindle poles and its staining largely overlapped with aMTOC staining (data not shown). To confirm that NUMA also labels microtubule minus-ends in mouse oocytes, the inventors enriched kinetochore or interpolar microtubules by cold treatment (*41*) and acute NDC80/HEC1 depletion (*42*), respectively. Both kinetochore and interpolar microtubules ended at NUMA and aMTOCs (data not shown), consistent with NUMA anchoring microtubules to aMTOCs in mouse oocytes (*28*).

aMTOCs enrich several regulatory kinases at the spindle poles in mouse oocytes (26). The inventors considered that AURA, which is an essential kinase for aMTOC functions (43, 44) and can phosphorylate NUMA (*45*), negatively regulates NUMA enrichment at microtubule minus-ends in mouse oocytes. In line with the inventors' hypothesis, constitutive treatment with the AURA inhibitor MLN8237 led to an increased NUMA enrichment at the spindle poles in mouse oocytes (data not shown), as revealed by manual scoring and a significant increase in the standard deviation of NUMA intensity within the spindle (data not shown). A similar enrichment was observed when MLN8237 was acutely added to mouse oocytes at metaphase I (data not shown). Importantly, live imaging of mouse oocytes treated with MLN8237 showed that NUMA becomes enriched before the reduction in spindle volume (data not shown), indicating that this redistribution is not an indirect consequence of changes in spindle dimensions. To confirm that NUMA enrichment is regulated by AURA-dependent phosphorylation, the inventors depleted endogenous NUMA by follicle RNAi (*39*) and re-expressed NUMA with mutations at the AURA phosphorylation site S1969. The S1969A mutation has been shown to phenocopy AURA inhibition in mitotic cells (*46*). The phosphodeficient NUMA(S1969A), but not the phosphomimetic NUMA(S1969D), was significantly enriched at the spindle poles in mouse oocytes (data not shown). Together, the inventors infer that AURA phosphorylation negatively regulates NUMA enrichment at microtubule minus-ends in mouse oocytes.

Immature human oocytes are not available in large quantities, which makes it challenging to gain mechanistic insights into the organization of spindle poles and the cause of spindle instability during meiosis I. "Humanized" mouse oocytes with a human-like spindle assembly process would be a useful model of human oocytes, because mouse oocytes are readily available, progress through meiosis synchronously, and can be manipulated with genetic tools such as follicle RNAi. Given that mouse oocytes treated with an AURA inhibitor phenocopy the NUMA enrichment at spindle poles observed in human oocytes, the inventors reasoned that spindle assembly in mouse oocytes could be humanized by co-ablating aMTOCs and the associated AURA. To this end, the inventors used Trim-Away to deplete the essential scaffolding component of aMTOCs, PCNT (*47*, *48*) (data not shown). PCNT-depleted mouse oocytes displayed cytoplasmic dispersal of aMTOC components, such as CEP192, CDK5RAP2 and γ-tubulin (data not shown), consistent with the successful ablation of aMTOCs. Importantly, aMTOC-associated AURA, but not spindle microtubule-associated AURA, was selectively ablated in PCNT-depleted mouse oocytes (for simplicity, hereafter referred to as aMTOC-free mouse oocytes) (data not shown).

The inventors used complementary approaches to confirm the humanization of spindle assembly in aMTOC-free mouse oocytes. First, spindle assembly in human oocytes is mediated by chromosomes and the small guanosine triphosphatase Ran (*2*). In this pathway, a chromosome-centered gradient of Ran-GTP promotes microtubule nucleation in proximity of chromosomes by locally releasing spindle assembly factors from inhibitory binding to importins (*49*). The inventors found that microtubule nucleation occurred around chromosomes in aMTOC-free mouse oocytes (data not shown), similar to human oocytes (*2*). Furthermore, dominant negative inhibition with RanT24N prevented microtubule nucleation in aMTOC-free mouse oocytes (data not shown). Thus, spindle assembly in aMTOC-free mouse oocytes, like in human oocytes, is dependent on the Ran pathway. Second, the localization patterns of several minus-end-binding proteins and spindle pole-associated proteins were now similar in aMTOC-free mouse oocytes and in human oocytes (data not shown). In particular, NUMA was highly enriched at microtubule minus-ends in aMTOC-free mouse oocytes (data not shown), as confirmed by cold treatment, acute NDC80/HEC1 depletion and immuno-electron microscopy (data not shown). NUMA was enriched at the spindle poles at similar levels in human oocytes and in aMTOC-free mouse oocytes (data not shown), and live imaging demonstrated that NUMA has a similar localization pattern in aMTOC-free mouse oocytes and in human oocytes throughout meiosis (data not shown). Finally, the inventors used Trim-Away to test whether NUMA is required to focus the spindle poles in aMTOC-free mouse oocytes. In wild-type mouse oocytes, perturbation of NUMA results in hyperfocused spindle poles, which coincides with the aggregation of aMTOCs (27, *28*). Instead, depletion of NUMA in aMTOC-free mouse oocytes caused defocused spindle poles similar to in human oocytes (data not shown), as revealed by manual scoring of spindle pole morphologies and a significant decrease in the microtubule packing index (data not shown), which measures how densely microtubules are packed within the spindle. Together, these results strongly support that co-ablation of aMTOCs and the associated AURA kinase humanizes the spindle assembly process in mouse oocytes.

### NUMA forms a microtubule-dependent, stable scaffold in the absence of aMTOCs

NUMA anchors spindle pole microtubules to centrosomes in somatic cells (*9*, *50*) and to aMTOCs in wild-type mouse oocytes (*28*), but these stable scaffolds are not present in human oocytes or in aMTOC-free mouse oocytes. Interestingly, non-phosphorylated NUMA can self-assemble into oligomers *in vitro* (*51, 52*) and the data above suggest that aMTOC-free mouse oocytes contain non-phosphorylated NUMA at their spindle poles (data not shown). Therefore, the inventors hypothesized that non-phosphorylated NUMA oligomerizes to form a stable scaffold at the spindle poles in the absence of aMTOCs.

This hypothesis predicts that NUMA would have a lower turnover in aMTOC-free mouse oocytes compared to wild-type mouse oocytes. To examine the dynamics of NUMA, the inventors photoactivated NUMA at the pole-proximal, pole-distal, or cytoplasmic regions in control and aMTOC-free mouse oocytes, similar to a previous study of γ-tubulin dynamics at the spindle poles in mitotic cells (*53*). Photoactivation of the pole-proximal region revealed a long-lived NUMA population in aMTOC-free mouse oocytes but not in wild-type mouse oocytes (data not shown). In contrast, photoactivation of the pole-distal region revealed a distinct, short-lived NUMA population in both wild-type and aMTOC-free mouse oocytes (data not shown). As NUMA was detected along kinetochore microtubules in both wild-type and aMTOC-free mouse oocytes (data not shown), the inventors propose that this short-lived population corresponds to the transient, dynamic NUMA crosslinks recently reported on kinetochore microtubules (*54, 55*). Interestingly, photoactivated NUMA in the cytoplasm of aMTOC-free mouse oocytes was only incorporated in the pole-distal region (data not shown), providing further support for the low turnover of NUMA at the spindle poles in the absence of aMTOCs.

In mitotic cells, NUMA at the spindle poles forms insoluble aggregates upon microtubule depolymerization (*56-59*). To test whether NUMA forms similar cytoplasmic aggregates in aMTOC-free mouse oocytes, the inventors acutely depolymerized spindle microtubules with nocodazole or prolonged cold treatment. However, NUMA did not persist as cytoplasmic foci upon microtubule depolymerization (data not shown), suggesting that the associations between NUMA at the spindle poles are predominantly microtubule-dependent. Together, the inventors conclude that NUMA forms a microtubule-dependent, stable scaffold at the spindle poles in the absence of aMTOCs.

### NUMA and dynein-dynactin-LIS1 cluster microtubule minus-ends in the absence of aMTOCs

Next, the inventors exploited aMTOC-free mouse oocytes to decipher how the stably associated NUMA organizes the spindle poles. NUMA has been proposed to organize the spindle poles by different mechanisms in different systems. For instance, during spindle assembly on sperm nuclei in *Xenopus* egg extracts, dynein and its cofactor dynactin transport NUMA as a cargo towards the poles. As a result, NUMA accumulates and crosslinks microtubules at the spindle poles (*60*). In addition, NUMA can help dynein-dynactin to capture free microtubules, co-transporting them towards the spindle poles (*61*). On the other hand, NUMA has been shown to associate with microtubules independently of dynein-dynactin during aster assembly in HeLa cell extracts and spindle assembly on chromatin beads in *Xenopus* egg extracts (*17, 62*). This concept is supported by recent laser ablation studies in mitotic cells, which demonstrate that NUMA localizes to newly generated microtubule minus-ends independently of dynein-dynactin (*63*). Instead, NUMA is proposed to serve as a cargo adaptor for microtubule minus-ends, recruiting dynein-dynactin to sort minus-ends towards the spindle poles (*63-65*).

To understand how NUMA drives pole focusing in the absence of aMTOCs, the inventors depleted endogenous NUMA and re-expressed four different NUMA mutants in aMTOC-free mouse oocytes (data not shown). NUMA(ΔN) and NUMA(SpM-4A), which cannot interact with dynein-dynactin (*66*, *67*), bound to microtubule minus-ends but failed to rescue pole focusing (data not shown). NUMA(ΔMTBD1), which cannot bind microtubule tips (*68, 69*), was not enriched at microtubule minus-ends and failed to restore pole focusing (data not shown). However, NUMA(ΔMTBD2), which cannot bundle microtubules (*46*), bound to microtubule minus-ends and partially restored pole focusing (data not shown). These results combined suggest that spindle pole focusing in the absence of aMTOCs depends only partially on NUMA's microtubule crosslinking activity, but predominantly on its binding to microtubule minus-ends and to dynein-dynactin. In line with these observations, the inventors found that NUMA was in proximity to dynein-dynactin at the spindle poles in aMTOC-free mouse oocytes (data not shown).

To examine the roles of dynein-dynactin in spindle pole focusing mediated by NUMA, the inventors performed dominant negative inhibitions. The inventors ectopically expressed the protein fragment P150-CC1, which binds dynein (*70*) and perturbs its interactions with dynactin's ARP1 filament (data not shown), or NUMA-N, which does not oligomerize with endogenous NUMA but perturbs the interactions with dynein-dynactin (*66*). aMTOC-free mouse oocytes expressing P150-CC1 or NUMA-N displayed similar phenotypes: NUMA remained bound to microtubule minus-ends, but spindle poles became defocused (data not shown). In addition, when the inventors acutely injected recombinant P150-CC1 or NUMA-N into metaphase I aMTOC-free mouse oocytes, in which NUMA had already stably associated with microtubule minus-ends (data not shown), spindle poles became defocused (data not shown). Thus, NUMA binds microtubule minus-ends independently of dynein-dynactin, but is insufficient to focus or maintain the spindle poles without dynein-dynactin.

Recent studies have shown that the assembly of fully activated dynein complexes requires an additional cofactor LIS1 (*71*), which is also present in mouse oocytes (*26*). Interestingly, similar to dynein and dynactin, LIS1 localized to the spindle poles in aMTOC-free mouse oocytes (data not shown). To test whether LIS1 is also required for pole focusing, the inventors used Trim-Away (data not shown). Both constitutive and acute depletion of LIS1 phenocopied dynein-dynactin inhibition and led to spindle pole defocusing in aMTOC-free mouse oocytes (data not shown). To determine if dynein, dynactin and LIS1 function as an entity, the inventors interfered with individual components and examined their accumulation at kinetochores by acutely perturbing poleward transport during metaphase I with nocodazole (*72*). P150-CC1-mediated inhibition and LIS1 depletion, but not NUMA depletion, each disrupted the localization of dynein, dynactin and LIS1 in aMTOC-free mouse oocytes (data not shown). Combined, these results suggest that stably associated NUMA, together with a dynein-dynactin-LIS1 complex, clusters microtubule minus-ends in the absence of aMTOCs.

### Example 2

### Dynein-dynactin organizes the spindle poles in human oocytes

To test whether the findings in aMTOC-free mouse oocytes translate to human oocytes, **the inventors** first analyzed the localization of dynactin at different stages of meiosis in human oocytes. Dynactin was not recruited during early spindle assembly, but localized predominantly to kinetochores and spindle microtubules before the metaphase spindle had assembled (**Fig.** 1A). At metaphase I and II, dynactin was most prominently detected at the spindle poles (**Fig.** 1, A and B). Remarkably, dynactin was in proximity to LIS1 and NUMA at the spindle poles (**Fig.** 1B), in line with their localization in aMTOC-free mouse oocytes (data not shown).

The inventors then inhibited dynein-dynactin in human oocytes using P150-CC1. Strikingly, NUMA remained bound to microtubule minus-ends and spindle poles became defocused in dynein-inhibited human oocytes (**Fig.** 1, C and D), recapitulating the observations in aMTOC-free mouse oocytes (data not shown). Thus, the inventors conclude that dynein-dynactin and possibly LIS1 are required for pole focusing in human oocytes.

### Human oocytes lack the spindle-stabilizing factor kinesin-14 KIFC1/HSET

The inventors noticed that around 40% of the defocused spindles in human oocytes with NUMA depletion or dynein inhibition failed to align midzone microtubules (**Fig.** 1, E and F). Intriguingly, a similar fraction of untreated spindles from fixed human oocytes in this study (**Fig.** 1G) and from live human oocytes in the previous study (2) displayed multipolarity (**Fig.** 1H). On the contrary, misalignment of midzone microtubules and spindle instability were never observed in aMTOC-free mouse oocytes (**Fig.** 1, E to H).

To determine whether spindle instability is a general feature of non-rodent mammalian oocytes that lack aMTOCs, the inventors performed live imaging of spindle assembly in bovine and porcine oocytes. These oocytes progressed through similar stages of spindle assembly as human oocytes (**Fig.** 4, A and B). However, the inventors observed unstable spindle poles in only 3.1% of bovine oocytes and 4.4% of porcine oocytes (**Fig.** 4, C and D), compared to in 82% of human oocytes (*2*) (**Fig.** 1G). The inventors infer that firstly, spindle instability in human oocytes is not due to the absence of aMTOCs, and secondly, additional mechanism(s) must stabilize the spindles in other mammalian oocytes. Therefore, the inventors hypothesized that human oocytes lack a protein that is present in mouse, bovine and porcine oocytes, and that the absence of this protein leads to misalignment of midzone microtubules and spindle instability.

The inventors designed an RNAi screen to identify the protein that protects aMTOC-free mouse oocytes from misalignment of midzone microtubules and spindle instability. Specifically, the inventors co-depleted NUMA with one of 20 candidate proteins in aMTOC-free mouse oocytes using follicle RNAi. The candidate proteins included microtubule crosslinking spindle assembly factors (DLG5/HURP and TPX2) (*49*), proteins related to spindle bipolarization (HAUS6, KIF15/HKLP2 and KIF11/EG5) (*73, 74*), proteins related to microtubule dynamics (KIF2A, KIF18A, CENPE, CLASP1 and CLASP2) (*75*), proteins related to bridging fibers and central spindle (CYK4, PRC1, KIF4A, KIF12, KIF14, KIF20A/MKLP2, KIF20B/MPP1 and KIF23/MKLP1) (*76*) and spindle pole-associated proteins that are not related to dynein (ASPM and KIFC1/HSET) (*37*). Whereas oocytes co-depleted of NUMA and DLG5/HURP, TPX2 or HAUS6 did not assemble a spindle, oocytes co-depleted of NUMA and all other proteins except one assembled a spindle with aligned midzone microtubules (**Fig.** 2A). Intriguingly, only oocytes co-depleted of NUMA and kinesin-14 KIFC1/HSET failed to align midzone microtubules, as confirmed by directionality analysis of spindle microtubules (**Fig.** 2A).

To determine whether KIFC1/HSET is expressed in human oocytes, the inventors examined data from previous proteomics studies on mouse and human oocytes (*77, 78*). The inventors noticed that KIFC1/HSET could only be detected in the mouse dataset. However, due to the differences in proteome coverage for these two studies, the inventors additionally analyzed KIFC1/HSET expression using data from previous RNA-seq studies of mammalian oocytes and embryos (*79-82*). Mouse, bovine, and porcine oocytes had a prominent pool of maternal KIFC1/HSET mRNA, which was depleted upon fertilization, and embryonic KIFC1/HSET mRNA was expressed from the 2- to 4-cell stage onwards (**Fig.** 5A). In contrast, KIFC1/HSET mRNA was barely detectable in human oocytes and zygotes, but was readily expressed from the 2- or 4-cell stage onwards in embryos, based on the cited literature (**Fig.** 5A). Such discrepancies in gene expression between oocytes of different mammalian species were not observed for NUMA or for the conserved zona pellucida protein ZP3 (**Fig.** 5B).

The inventors subsequently examined KIFC1/HSET protein levels in oocytes and in asynchronized HeLa cells as a positive control (**Fig.** 2, B and C). To ensure comparable loading of oocyte lysate from different species, the inventors performed on-blot total protein normalization, which outperformed the sensitivity and linearity of all canonical housekeeping proteins (**Fig.** 6, A to E). Although the inventors could readily detect KIFC1/HSET in HeLa cell, mouse oocyte, bovine oocyte and porcine oocyte lysates (**Fig.** 2B, and **Fig.** 6F), the inventors could not detect KIFC1/HSET in comparable amounts of human oocyte lysate, even after overexposure (**Fig.** 2B). Thus, the inventors conclude that human oocytes lack KIFC1/HSET.

### Depletion of KIFC1/HSET in aMTOC-free mouse oocytes and in bovine oocytes fully recapitulates the spindle instability of human oocytes

To mimic the lack of KIFC1/HSET in human oocytes, the inventors depleted KIFC1/HSET in aMTOC-free mouse oocytes and in bovine oocytes using follicle RNAi and Trim-Away, respectively. Strikingly, around 35% of KIFC1/HSET-depleted aMTOC-free mouse oocytes assembled a multipolar spindle (**Fig.** 2, D and E), as confirmed by the significant increase in the number of NUMA clusters per oocyte (**Fig.** 2F). Moreover, around 30% of KIFC1/HSET-depleted aMTOC-free mouse oocytes assembled a round spindle with broad poles (**Fig.** 2, D and E). These spindles closely resembled the "apolar" spindles that were previously observed in live human oocytes (2). Quantification of the total volume of the NUMA clusters revealed no significant difference between control and KIFC1/HSET-depleted oocytes (**Fig.** 7A), suggesting that these pole defects are caused by the failure of NUMA clusters to coalesce rather than by the *de novo* assembly of additional NUMA clusters. Similarly, around 60% and 25% of KIFC1/HSET-depleted bovine oocytes assembled a multipolar spindle or a spindle with broad poles, respectively (**Fig.** 7, B to E). In contrast, co-depletion of other members in the kinesin-14 family, KIFC2 and KIFC3, did not result in multipolar spindles or round spindles with broad poles in aMTOC-free mouse oocytes (**Fig.** 7, F and G). Thus, depletion of KIFC1/HSET specifically recapitulates the spindle instability of human oocytes, strongly suggesting that the lack of KIFC1/HSET is a major contributor to spindle instability in human oocytes.

KIFC1/HSET is dispensable for stable spindle poles in wild-type mouse oocytes during meiosis I (*83, 84*). To understand the functions of KIFC1/HSET in the absence of aMTOCs, the inventors first compared the localization of mClover3-HSET in wild-type and aMTOC-free mouse oocytes. Consistent with a previous study (83), KIFC1/HSET was uniformly localized throughout the spindle in wild-type mouse oocytes (**Fig.** 3, A and B). In contrast, KIFC1/HSET was enriched at the forming spindle before bipolarization, and subsequently at the poles, in aMTOC-free mouse oocytes (**Fig.** 3, A to C). Fluorescence recovery after photobleaching analysis revealed that wild-type and aMTOC-free mouse oocytes have a comparable fraction of mobile KIFC1/HSET, but the turnover of KIFC1/HSET at the spindle poles is two-times slower in the absence of aMTOCs **(****Fig.** 3, D to F). In contrast, loss of aMTOCs did not affect the turnover of KIFC1/HSET in the spindle midzone nor of the microtubule reporter MAP4-MTBD (**Fig.** 7, H to J). Overall, the data suggest that the dynamics of KIFC1/HSET at the spindle poles is specifically altered by the loss of aMTOCs and that KIFC1/HSET accumulation at the poles may stabilize the spindles in the absence of aMTOCs.

Unlike the kinesin-14 family members KIFC2 and KIFC3, KIFC1/HSET uniquely possesses a microtubule-binding domain at its tail (*85*). This domain, together with the conserved, microtubule-binding motor domain, allows KIFC1/HSET to organize microtubules via crosslinking and/or sliding (*86*). To investigate the requirement of different domains in KIFC1/HSET, the inventors expressed a series of truncation mutants in aMTOC-free mouse oocytes (**Fig.** 7K). Notably, the spindle localization of KIFC1/HSET in aMTOC-free mouse oocytes depended predominantly on the tail domain but not on its motor domain (**Fig.** 7L). To examine whether the motor activity is dispensable for the function of KIFC1/HSET in aMTOC-free mouse oocytes, the inventors depleted endogenous KIFC1/HSET by follicle RNAi and restored expression with wild-type HSET or with HSET(N593K), which crosslinks microtubules but cannot slide (*87*). Whereas almost all aMTOC-free mouse oocytes expressing wild-type HSET assembled a bipolar spindle, nearly all oocytes rescued with HSET(N593K) assembled a disorganized spindle (**Fig.** 3, G and H). These disorganized spindles were composed of several weakly associated microtubule bundles (**Fig.** 3G), indicating that KIFC1/HSET's sliding activity is required to align crosslinked microtubules into a bipolar spindle. In addition, the inventors noticed that around 25% of oocytes rescued with wild-type HSET assembled a bipolar spindle but had poorly focused poles that lacked NUMA (**Fig.** 3G); quantification of the HSET to α-tubulin intensity ratio revealed that almost 2-fold more HSET was loaded onto these spindles (**Fig.** 3I). These data suggest that overexpression of KIFC1/HSET could interfere with NUMA-mediated pole focusing. Together, the inventors infer that the appropriate level of KIFC1/HSET's crosslinking and sliding activities are required for proper bipolar spindle assembly in the absence of aMTOCs.

### Microinjection of KIFC1/HSET rescues spindle pole instability in human oocytes

To confirm that the lack of KIFC1/HSET is a major contributor to spindle instability in human oocytes, the inventors introduced KIFC1/HSET into human oocytes in **Fig.** 8. To visualize the dynamics of microtubules and chromosomes using live cell imaging, human oocytes were stained with 5'-SiR-tubulin and SPY555-DNA, respectively. While the introduction of KIFC1/HSET did not significantly alter the time of early spindle bipolarization after onset of microtubule nucleation (2.4 h in non-injected oocytes and 2.65 h in injected oocytes; p=0.7197) (**Fig.** 8B), the introduction of KIFC1/HSET significantly reduced the duration of spindle pole instability (10.3 h in non-injected oocytes and 3.4 h in injected oocytes; p<0.0001) (**Fig.** 8C). The inventors also quantified the frequency of misaligned chromosomes at anaphase onset (**Fig.** 8D) and lagging chromosomes (**Fig.** 8E) in human oocytes non-injected and injected with KIFC1/HSET. Introduction of KIFC1/HSET increased the proportion of human oocytes with aligned chromosomes at anaphase onset from 44.4% to 75%, and the proportion of human oocytes with no lagging chromosome from 33.3% to 62.5%. Together, the inventors infer that the introduction of KIFC1/HSET stabilized the meiotic spindle and reduced the risk of aneuploidy in human oocytes.

### Introducing exogenous KIFC1 promotes bipolar spindle assembly and accurate chromosome segregation in human zygotes

The inventors showed above that human oocytes are naturally deficient in KIFC1, and that the introduction of KIFC1 into human oocytes stabilizes the spindle and reduces spindle multipolarity. Spindles in human fertilized eggs (zygotes) have also been reported to often be multipolar (*6*, *7, 122*). By analyzing published RNA sequencing datasets the inventors found that KIFC1 is only expressed at higher levels in human embryos from the two-cell stage onward (**Fig.** 9A), suggesting that spindle assembly in human zygotes could also be affected by KIFC1 deficiency. The inventors thus tested if the introduction of exogenous KIFC1 increased the fidelity of spindle assembly and chromosome segregation in human zygotes, by using human tripronuclear (3PN) zygotes. Similar to the zygotes in (*6*, *7, 122*), these zygotes frequently assembled multipolar spindles. Introduction of mClover3-KIFC1 protein into human 3PN zygotes significantly reduced multipolar spindle assembly and promoted bipolar spindle assembly (**Fig.** 9, B to D). Furthermore, introduction of mClover3-KIFC1 protein increased the frequency of zygotes with aligned chromosomes at anaphase onset from 20% to 57% (**Fig.** 9E), and the frequency of zygotes without lagging chromosomes during anaphase from 40% to 71% (**Fig.** 9F). Thus, introducing exogenous KIFC1 increased the efficiency of bipolar spindle assembly and reduced the risk of aneuploidy in human zygotes.

### Discussion

The presented data uncover the long-sought cause of spindle instability in human oocytes: Human oocytes lack kinesin-14 KIFC1/HSET, a key spindle-stabilizing factor that is present in oocytes of other species. As shown in this and previous studies (*23, 24, 83, 88-90*), most mammalian and non-mammalian oocytes express KIFC1/HSET to promote proper spindle assembly. The inventors report that, in the absence of aMTOCs, KIFC1/HSET ensures the coalescence of clustered microtubule minus-ends at the two spindle poles in mammalian oocytes (**Fig.** 7M). Spindle stabilization is likely achieved via the formation of static crosslinks along parallel microtubules at the poles and the alignment of antiparallel microtubules in the midzone. As human oocytes do not express KIFC1/HSET, the inventors propose that the absence of these activities renders their spindles unstable.

The data also reveal further striking differences in spindle pole organization in different mammalian oocytes. Whereas aMTOCs dominate the organization of spindle poles in mouse oocytes (*25, 27-30*), an adaptation in NUMA behavior compensates for the absence of aMTOCs in non-rodent mammalian oocytes. In the absence of aMTOCs, NUMA becomes strongly enriched at and stably associated with microtubule minus-ends. Owing to its multi-modular nature, NUMA can crosslink microtubules at the minus-ends, and importantly, engage minus-end-directed dynein complexes. All of these features allow stably associated NUMA to cluster microtubule minus-ends at the spindle poles (**Fig.** 7M), replacing the microtubule anchoring function of aMTOCs. In addition to anchoring microtubules, aMTOCs promote microtubule nucleation (*25, 27, 30*). However, NUMA neither nucleates microtubules nor interacts with microtubule nucleators (data not shown), in line with the crucial requirement of the Ran pathway for microtubule nucleation in non-rodent mammalian oocytes (*2*, *31*)*.*

In *Xenopus* egg extracts, *Drosophila* S2 cells and non-mammalian oocytes, dynein and KIFC1/HSET appear to have similar roles in pole focusing (*23, 24, 89-94*). Interestingly, perturbation of NUMA-dynein and of KIFC1/HSET result in distinct phenotypes in mammalian oocytes that lack aMTOCs, suggesting that they have non-redundant roles at the spindle poles. Although both dynein and KIFC1/HSET are minus-end-directed motors with microtubule crosslinking and sliding activities, their differential roles may be explained by their different distribution on the meiotic spindle. NUMA-dynein is confined at microtubule minus-ends and hence acts more locally, whereas KIFC1/HSET localizes throughout the polar region of the spindle and hence acts more globally.

In normal mitotic cells with two centrosomes, KIFC1/HSET is largely dispensable for bipolar spindle assembly (*87, 95-97*). In contrast, mitotic cells with supernumerary centrosomes require KIFC1/HSET to cluster extra centrosomes into two spindle poles, preventing spindle multipolarity (*95, 96, 98, 99*). Hence, KIFC1/HSET has been proposed as a selective target for treating cancers, where supernumerary centrosomes are frequently observed (100). In this study, the inventors demonstrate that KIFC1/HSET also prevents spindle multipolarity in mammalian oocytes that lack aMTOCs. This suggests that spindle pole organization in these oocytes may be intrinsically error-prone due to the absence of additional mechanisms enforcing spindle bipolarity such as those that regulate the number of centrosomes (*101*) and aMTOCs (*29*). Thus, KIFC1/HSET is additionally required to cluster extra spindle poles formed in cancer cells and in non-rodent mammalian oocytes.

Upon fertilization, non-rodent mammalian eggs reacquire centrosomes from the sperm (*15*). Although non-rodent mammalian zygotes assemble centrosomal spindles, KIFC1/HSET may still be required for bipolar spindle assembly, given that centrosomes are not always tightly associated with the spindle poles (*6*, *102-108*) or there are more than two centrosomes (*109-113*)*.* Human zygotes have a high incidence of multipolar spindles (*5*-*7*), and low levels of KIFC1/HSET mRNA. Interestingly, spindles are mostly bipolar in human 2-cell stage embryos (*7*). This sudden reduction in multipolar spindles coincides with a rise in KIFC1/HSET mRNA from the 2-cell stage onwards. Notably, bovine oocytes express KIFC1/HSET, and multipolar spindles are rare in bovine zygotes (*107, 108*)*.* Thus, differences in maternal KIFC1/HSET expression may also explain why human zygotes are more prone to assemble multipolar spindles than other mammalian zygotes.

Furthermore, the inventors showed that introducing exogenous KIFC1/HSET increased the efficiency of bipolar spindle assembly and reduced the risk of aneuploidy in human zygotes.

Based on the data shown herein, the inventors propose KIFC1/HSET as a therapeutic candidate for counteracting spindle instability in human oocytes and/or zygotes. Re-expression of KIFC1/HSET in aMTOC-free mouse oocytes grown from KIFC1/HSET-depleted follicles suppresses spindle instability, implying that KIFC1/HSET exerts its functions after folliculogenesis. Thus, it is proposed that spindles in fully grown human oocytes and/or zygotes are stabilized by introducing KIFC1/HSET.

### Materials and Methods

### Preparation and culture of mouse oocytes and follicles

All mice were maintained in a specific pathogen-free environment at the Animal Facility of the Max Planck Institute for Biophysical Chemistry according to The Federation of European Laboratory Animal Science Associations guidelines and recommendations.

Oocytes were isolated from ovaries of 8 - 12-week-old FVB/N female mice. Fully grown oocytes of around 75 µm in diameter with a centered nucleus were maintained at prophase arrest in homemade phenol red-free M2 supplemented with 250 µM dibutyryl cyclic AMP (dbcAMP) (Sigma-Aldrich) under paraffin oil (ACROS Organics) at 37°C. To resume meiosis, oocytes were released into dbcAMP-free M2 at 37°C.

Follicles were mechanically isolated from 10 - 12-day-old (C57BL/6J × CBA) F1 female mice in HEPES-buffered MEM with GlutaMAX (Gibco) supplemented with 5% fetal bovine serum (FBS) (Gibco) and 0.1× penicillin G/streptomycin (Sigma-Aldrich). Compact follicles of around 100 µm in diameter with a centered oocyte were cultured in MEM alpha with GlutaMAX and nucleosides (Gibco) supplemented with 5% FBS, 0.03 µg/ml ovine follicle stimulating hormone (National Hormone and Peptide Program), 1× insulin/transferrin/sodium selenite (Sigma-Aldrich) and 0.1× penicillin G/streptomycin on 12 mm Transwell-COL collagen-coated 0.4 µm pore PTFE membrane insert (Corning) at 37°C/5% CO₂. Half of the medium surrounding the insert was replaced every 3 d. After 10 - 12 d of culture, *in vitro* grown oocytes were denuded and matured in modified M2 supplemented with 10% FBS instead of 4 mg/ml bovine serum albumin (BSA) (Sigma-Aldrich).

### Preparation and culture of bovine and porcine oocytes

All ovaries were obtained from local slaughterhouses. Bovine and porcine ovaries were transported in a thermo-flask to the laboratory within 1 - 3 h after retrieval and washed extensively with warm 0.9% NaCl. Cumulus-oocyte complexes (COCs) were recovered by aspiration of antral follicles with a 17-gauge needle affixed to a 1 ml syringe. 140 µl of 5000 IU/ml heparin (Merck Millipore) was additionally added to every 20 ml of aspirates from bovine ovaries. Bovine and porcine COCs were allowed to sediment and then washed extensively with HEPES-buffered Medium 199 (Sigma-Aldrich) and POE-CM (Cosmo Bio), respectively, at 39°C. Only fully grown oocytes with a homogenous cytoplasm and several layers of compact cumulus cells were selected for experiments. To better synchronize meiotic progression, oocytes were maintained in prophase arrest in medium supplemented with 10 µM RO-3306 (Sigma-Aldrich) instead of dbcAMP. To resume meiosis, bovine and porcine oocytes were released into RO-3306-free BO-IVM (IVF Bioscience) and POM (Cosmo Bio), respectively, at 39°C/5% CO₂.

### Preparation and culture of human oocytes

The use of unfertilized human oocytes in this study was approved by the UK's National Research Ethics Service under the REC reference 11/EE/0346 (IRAS Project ID 84952) and the Ärztekammer Niedersachsen (Ethics Committee of Lower Saxony) under the reference 15/2016. Oocytes were collected from patients who underwent ovarian stimulation for intracytoplasmic sperm injection (ICSI) as part of their assisted reproduction treatment at the Bourn Hall Clinic or Kinderwunschzentrum Göttingen or Fertility Center Berlin. Only oocytes that were immature at the time of ICSI and thus unsuitable for the procedure were used in this study. All patients gave informed consent for their surplus oocyte(s) to be used in this study. Oocytes were cultured as previously described (*1*). Briefly, within 3 - 5 h after retrieval from ovaries, oocytes were transferred into G-MOPS (Vitrolife) supplemented with 10% FBS under paraffin oil at 37°C. The timing of nuclear envelope breakdown (NEBD) was monitored using the Primo Vision EVO+ microscope (Vitrolife) installed inside the incubator. Only oocytes that were morphologically normal and underwent NEBD within 24 h after retrieval from ovaries were used in this study.

### Expression constructs, messenger RNA (mRNA) synthesis, recombinant protein expression and purification

To generate constructs for mRNA synthesis, we fused previously published coding sequences with mClover3 (*2*), mPA-GFP (*3*) and mScarlet (*4*), and sub-cloned them into pGEMHE (*5*) to obtain mClover3-HSET (OriGene), mScarlet-MAP4-MTBD (*6*), mClover3-NUMA and mPA-GFP-NUMA (M. Mancini, unpublished). pGEMHE-mClover3-HSET(ΔMotor), pGEMHE-mClover3-HSET(ΔTail), pGEMHE-mClover3-HSET(Motor), pGEMHE-mClover3-HSET(N593K), pGEMHE-mClover3-NUMA(S1955D), pGEMHE-mClover3-NUMA(S1955A), pGEMHE-mClover3-NUMA(ΔN), pGEMHE-mClover3-NUMA(SpM-4A), pGEMHE-mClover3-NUMA(ΔMTBD1), pGEMHE-mClover3-NUMA(ΔMTBD2), pGEMHE-mClover3-NUMA-N, pGEMHE-EGFP-P150(ΔMTBD), pGEMHE-EGFP-P150(ΔABD) and pGEMHE-EGFP-P150-CC1 were constructed from pGEMHE-mClover3-HSET, pGEMHE-mClover3-NUMA and pGEMHE-EGFP-P150 (*6*) using Q5 Site-Directed Mutagenesis Kit (NEB). mClover3-HSET was sub-cloned from pGEMHE-mClover3-HSET into pQE-TriSystem-His-Strep2 (Qiagen). MBP was sub-cloned from pET-21a(+)/MBP-His (M. J. Fox Foundation, unpublished) into pET28a(+). NUMA-N was sub-cloned from pGEMHE-mClover3-NUMA-N into pET28a(+). HLTV-hTRIM21 (*7*), pET28a(+)-P150-CC1-His (*8*), pGEMHE-AURA-meGFP (*6*), pGEMHE-mScarlet-CEP192 (*6*), pGEMHE-H2B-mCherry (*9*), pGEMHE-H2B-miRFP (*6*), pGEMHE-meGFP-MAP4 (*9*), pGEMHE-mClover3-MAP4-MTBD (*6*), pGEMHE-3×CyOFP-MAP4-MTBD (*6*), pGEMHE-bTRIM21 (*6*) and pGEMHE-mTRIM21 (*6*) were also used. All mRNAs were synthesized and quantified as previously described (*7*).

Recombinant His-NUMA-N, P150-CC1-His and hTRIM21 were expressed in and purified from NiCo21(DE3) (NEB) or OverExpress C41(DE3) (Sigma-Aldrich) as previously described (*7*). Briefly, they were first affinity-purified with ÄKTA pure (GE Healthcare) using HisTrap FF (GE Healthcare), followed by size exclusion chromatography using HiLoad 26/600 Superdex 200 pg (GE Healthcare). Recombinant His-Strep2-mClover3-HSET was expressed in and purified from 293 cells (ECACC) as previously described (*10*).

### Short-interfering RNAs (siRNAs)

All siRNAs were purchased from Qiagen. The sequence of siRNAs used in this study were listed in Table S1. AllStars Negative Control (Qiagen) was used as a control.

### Microinjection of RNAs

Immature mouse oocytes were microinjected with 3.5 pl of mRNAs as previously described (*9*). AURA-meGFP mRNA was microinjected at a needle concentration of 69.7 ng/µl, mScarlet-CEP192 mRNA at 165.8 ng/µl, H2B-miRFP mRNA at 28.4 ng/µl, mClover3-HSET mRNA at 111.1 ng/µl, mClover3-HSET(ΔMotor) mRNA at 224 ng/µl, mClover3-HSET(ΔTail) mRNA at 334 ng/µl, mClover3-HSET(Motor) mRNA at 278.8 ng/µl, mClover3-MAP4-MTBD mRNA at 83.5 ng/µl, 3×CyOFP-MAP4-MTBD mRNA at 166.4 ng/µl, mScarlet-MAP4-MTBD mRNA at 83.5 ng/µl, MBP mRNA at 500 ng/µl, mClover3-NUMA mRNA at 165.9 ng/µl, mClover3-NUMA(ΔN) mRNA at 470.4 ng/µl, mPA-GFP-NUMA mRNA at 400 ng/µl, mClover3-NUMA-N mRNA at 500 ng/µl, EGFP-P150(ΔMTBD) mRNA at 720 ng/µl, EGFP-P150(ΔABD) mRNA at 583.8 ng/µl, EGFP-P150-CC1 mRNA at 448.3 ng/µl, P150-CC1 mRNA at 500 ng/µl and mTRIM21 mRNA at 421 ng/µl. Oocytes were allowed to express the mRNAs for 3 - 4 h before release.

Mouse follicles were microinjected with 3.5 pl of siRNAs at a needle concentration of 2 µM as previously described (*11*)*.* For rescue experiments, *in vitro* grown oocytes were microinjected with 3.5 pl of mRNAs. mClover3-HSET mRNA was microinjected at a needle concentration of 275 ng/µl, mClover3-HSET(N593K) mRNA at 275 ng/µl, mClover3-NUMA(S1955D) mRNA at 400 ng/µl, mClover3-NUMA(S1955A) mRNA at 400 ng/µl, mClover3-NUMA(SpM-4A) mRNA at 400 ng/µl, mClover3-NUMA(ΔMTBD1) mRNA at 400 ng/µl and mClover3-NUMA(ΔMTBD2) mRNA at 400 ng/µl. Oocytes were allowed to express the mRNAs for 3 - 4 h before release.

Cumulus cells surrounding immature bovine and porcine oocytes were fully stripped with a 135 µm EZ-Tip (CooperSurgical) before they were microinjected with 2 pl of mRNAs. Bovine oocytes were microinjected with meGFP-MAP4 and H2B-mCherry mRNAs at a needle concentration of 54 ng/µl and 5 ng/µl, respectively. Porcine oocytes were microinjected with meGFP-MAP4 and H2B-mCherry mRNAs at a needle concentration of 30 ng/µl and 3 ng/µl, respectively. Oocytes were allowed to express the mRNAs for 3 h before release.

### Microinjection of proteins

Immature mouse oocytes were microinjected with 12 pl of recombinant His-RanT24N (Cytoskeleton) at a needle concentration of 2 mg/ml. Corresponding amount of BSA was microinjected as a control.

Mouse meiosis I (MI) oocytes were microinjected with 7 pl of recombinant P150-CC1-His or His-NUMA-N at a needle concentration of 50 mg/ml and 15 mg/ml, respectively. Corresponding amount of BSA was microinjected as a control.

Human oocytes were microinjected with 10 - 15 pl of recombinant His-P150-CC1 at a needle concentration of 47.85 mg/ml with 0.03% NP-40 shortly after NEBD. A corresponding amount of BSA was microinjected as a control.

Immature human oocytes were microinjected with 11 pl of recombinant mClover3-HSET at a needle concentration of 0.87 mg/ml with 0.05% NP-40.

### Trim-Away in mouse, bovine and human oocytes

Only affinity purified antibodies were used in this study for Trim-Away-mediated protein depletion (12). Mouse monoclonal anti-HEC1 (sc-515550; Santa Cruz Biotechnology), rabbit anti-HSET-C (20790-1-AP; Proteintech), mouse monoclonal anti-LIS1 (H00005048-M03; Abnova), rabbit polyclonal anti-NUMA (ab97585; Abcam) and mouse monoclonal anti-PCNT (611814; BD Biosciences) were purified as previously described (*7*). The control IgGs used were normal mouse IgG (12-371; Millipore) and normal rabbit IgG (12-370; Millipore).

For constitutive Trim-Away in immature mouse oocytes, 3.5 pl of mRNAs and 3.5 pl of antibodies were co-injected as previously described (*7*). All antibodies were microinjected at a needle concentration of 1 - 2 mg/ml with 0.1% NP-40. Target proteins were allowed to be depleted for 3 - 4 h before the oocytes were released. For acute Trim-Away in mouse MI oocytes, immature oocytes were first microinjected with 3.5 pl of mRNAs and then released after 3 h. At around 6 h after release, oocytes were further microinjected with 3.5 pl of antibodies to acutely deplete the target proteins.

For constitutive Trim-Away in immature bovine oocytes, partially stripped oocytes were first microinjected with 3.5 pl of bTRIM21 mRNA at a needle concentration of 1400 ng/µl and allowed to express the mRNAs for 12 h. Before release, oocytes were fully stripped and further microinjected with 7 pl of antibodies at a needle concentration of 2.5 mg/ml with 0.05% NP-40.

For constitutive Trim-Away in immature human oocytes, oocytes were first microinjected with 10 - 15 pl of recombinant His-Lipoyl-TRIM21 at a needle concentration of 4.125 mg/ml with 0.0375% NP-40. After 3 - 4 h, oocytes were subsequently injected with 12 pl of antibody at a needle concentration of 2.5 mg/ml with 0.03% NP-40.

### Drug addition

All drugs were prepared in DMSO (Sigma-Aldrich) as 1000× stocks. To acutely depolymerize spindle microtubules, nocodazole (Sigma-Aldrich) was added to a final concentration of 10 µM at around 6 h after release. To constitutively inhibit AURA, immature mouse oocytes were released into dbcAMP-free M2 containing 500 nM MLN8237 (Selleckchem). To acutely inhibit AURA, MLN8237 was added to a final concentration of 500 nM at around 6 h after release.

### Immunofluorescence

To obtain mouse MI spindles, oocytes were incubated at 37°C for around 7 h after released into dbcAMP-free medium. To obtain porcine and bovine MI spindles, oocytes were incubated at 39°C/5% CO₂ for around 11 and 12 h, respectively, after released into RO-3306-free medium. To obtain human MI spindles, oocytes were incubated at 37°C for around 14 and 15 h after NEBD. None of the oocytes used for immunofluorescence analyses was subjected to live imaging before fixation.

Oocytes were fixed in 100 mM HEPES (pH 7.0, titrated with KOH), 50 mM EGTA (pH 7.0, titrated with KOH), 10 mM MgSO₄, 2% methanol-free formaldehyde and 0.5% Triton X-100 at 37°C for 15 - 60 min. Fixed oocytes and cells were extracted in phosphate-buffered saline (PBS) with 0.5% triton X-100 (PBT) overnight at 4°C and blocked in PBT with 5% BSA (PBT-BSA) overnight at 4°C. All antibody incubations were performed in PBT-BSA at 10 µg/ml overnight at 4°C (for primary antibodies) or at 20 µg/ml for 1 h at room temperature (for secondary antibodies).

Primary antibodies used were rabbit anti-ASPM (NB100-2278; Novus Biological), rat anti-α-tubulin (MCA78G; Bio-Rad), rabbit anti-CAMSAP3 (13), rabbit anti-CDK5RAP2 (ABE236; Merck Millipore), rabbit anti-CEP192 (18832-1-AP; Proteintech), rabbit anti-DHC (12345-1-AP; Proteintech), rabbit anti-EG5 (NB500-181; Novus Biologicals), goat anti-GFP (600-101-215; Rockland Immunochemicals), rabbit anti-γ-tubulin (T3559; Sigma-Aldrich), mouse anti-HEC1, rabbit anti-HSET-C, rabbit anti-KANSL3 (HPA035018; Sigma-Aldrich), rabbit anti-KIF2A (NB500-180; Novus Biologicals), mouse anti-LIS1, rabbit anti-MCRS1 (HPA039057; Sigma-Aldrich), rabbit anti-NUMA, rabbit anti-NUMA (ab84680; Abcam), mouse anti-P150 (612708; BD Biosciences), mouse anti-PCNT and rabbit anti-TPX2 (NB500-179; Novus Biological).

Secondary antibodies used were Alexa Fluor 488-, 594- or 647-conjugated AffiniPure Fab Fragment anti-goat, anti-rabbit and anti-rat (Jackson ImmunoResearch Europe), Alexa Fluor 568-conjugated Nano-Secondary anti-mouse IgG1 and anti-mouse IgG2b (ChromoTek), and Atto 488-conjugated FluoTag-X2 anti-mouse IgG2a/b (NanoTag Biotechnologies). DNA was stained with Hoechst 33342 (Molecular Probes).

### Cold treatment

To selectively depolymerize non-kinetochore microtubules, mouse MI oocytes were incubated on ice for 15 min and immediately fixed as for routine immunofluorescence. To depolymerize all spindle microtubules, mouse MI oocytes were incubated on ice for 1 h and immediately fixed as for routine immunofluorescence.

### Optical clearing of bovine and porcine oocytes

Oocytes were fixed, extracted and blocked as for routine immunofluorescence. Prior incubation with primary antibodies, lipid droplets in bovine and porcine oocytes were cleared with 4000 U/ml lipase from *Candida rugose* (Sigma-Aldrich) in 400 mM NaCl, 50 mM Tris (pH 7.2), 5 mM CaCl₂ and 0.2% sodium taurocholate supplemented with cOmplete, EDTA-free Protease Inhibitor Cocktail (Roche) at room temperature for 20 - 40 min.

### Peptide pre-incubation assay

Oocytes were fixed, extracted and blocked as for routine immunofluorescence. Mouse monoclonal anti-HEC1, rabbit polyclonal anti-HSET, mouse monoclonal anti-LIS1 and rabbit polyclonal anti-NUMA were pre-incubated with recombinant HEC1 (Proteintech), recombinant His-Strep2-mClover3-HSET (homemade), recombinant LIS1 (Abnova) and recombinant His-NUMA-N (homemade), respectively, before applied to oocytes as previously described (7).

### Confocal and super-resolution microscopy

For confocal imaging, oocytes were imaged in 2 µl of M2 medium (for live mouse oocytes) or PBS with 1% polyvinylpyrrolidone and 0.5 mg/ml BSA (for fixed oocytes) under paraffin oil in a 35 mm dish with a #1.0 coverslip. Images were acquired with LSM780, LSM800, LSM880 or LSM980 confocal laser scanning microscope (Zeiss) equipped with an environmental incubator box and a 40× C-Apochromat 1.2 NA water-immersion objective. A volume of 50 µm × 50 µm × 37.5 µm or 35 µm × 35 µm × 37.5 µm centered around the chromosomes was typically recorded. Automatic 3D tracking was implemented for time-lapse imaging with a temporal resolution of 5 - 15 min using a custom-made macro (Zeiss) on LSM800 or MyPiC (*14*) on LSM880. mClover3, meGFP, mPA-GFP, Alexa Fluor 488 and Atto 488 were excited with a 488 nm laser line and detected at 493 - 571 nm. CyOFP was excited with a 488 nm laser line and detected at 571 - 638 nm. mScarlet, mCherry and Alexa Fluor 568 were excited with a 561 nm laser line and detected at 571 - 638 nm. Alexa Fluor 594 was excited with a 594 nm laser line and detected at 605 - 638 nm. miRFP and Alexa Fluor 647 were excited with a 633 nm laser line and detected at 638 - 700 nm. Images of the control and experimental groups were acquired under identical imaging conditions on the same microscope. For some images, shot noise was reduced with a Gaussian filter. Airyscan images were acquired using the Airyscan module on LSM880 or LSM980 confocal laser scanning microscopes and processed in ZEN (Zeiss) after acquisition. Care was taken that the imaging conditions (laser power, pixel-dwell time and detector gain) did not cause phototoxicity (for live imaging), photobleaching or saturation.

### Photoactivation

For analyses of fluorescence dissipation, oocytes co-expressing mPA-GFP-NUMA with mScarlet-MAP4-MTBD and H2B-miRFP were rotated on stage with an unbroken microinjection needle to obtain meiotic spindles parallel to the imaging plane. Rectangular ROIs were marked and photoactivated using a 405 nm laser line at the maximum power after the 5^{th} time point.

### Fluorescence recovery after photobleaching

For analyses of fluorescence recovery after photobleaching, oocytes co-expressing mClover3-HSET with mScarlet-MAP4-MTBD and H2B-miRFP were rotated on stage with an unbroken microinjection needle to obtain meiotic spindles parallel to the imaging plane. Rectangular ROIs were marked and photobleached using a 488 and a 561 nm laser line at the maximum power after the 5^{th} time point.

### Immuno-electron microscopy and correlative focused ion beam-scanning electron microscopy (FIB-SEM)

For immune-electron microscopy of NUMA, mouse MI oocytes expressing mClover3-NUMA were microinjected with 7 pl of 0.167 mg/ml 2 nm Immunogold (Aurion)-conjugated GFP V_{H}H (Chromotek) with 0.1% NP-40. Before fixation in 100 mM HEPES (pH 7.0, titrated with KOH), 50 mM EGTA (pH 7.0, titrated with KOH), 10 mM MgSO₄, 3% EM-grade glutaraldehyde and 0.5% methanol-free formaldehyde at 37°C for 1 h, oocytes were randomly distributed and attached to a high Grid-500 35 mm µ-Dish (iBidi) using Cell-Tak Cell and Tissue Adhesive (Corning). To pre-select oocytes with an optimally oriented spindle, oocytes were stained with Hoechst 33322 and screened on a confocal laser scanning microscope. Before electron microscopy staining, silver enhancement was performed with R-Gent SE-EM (Aurion).

All following processing steps were performed in a microwave (Ted Pella) and oocytes were washed three times with water for 40 s at 250 W in between every staining step. Oocytes were first stained with 2% osmium tetroxide-1.5% potassium ferrocyanide in 0.1 M phosphate buffer (pH 7.4) for 12 min at 100 W (microwave cycling between on-off every 2 min). Oocytes were then incubated with 1% thiocarbohydrazide (Sigma-Aldrich) for 12 min at 100 W (microwave cycling between on-off every 2 min) and stained with 2% osmium tetroxide in water for 12 min at 100 W (microwave cycling between on-off every 2 min). Oocytes were further stained with 1% uranyl acetate in water for 12 min at 100 W (microwave cycling between on-off every 2 min) and 0.02 M lead nitrate-0.03 M aspartic acid (pH 5.5) for 12 min at 100 W (microwave cycling between on-off every 2 min). Oocytes were subsequently dehydrated in a graded ethanol series (10, 30, 50, 75, 90, 100 and 100%) for 40 s at 250 W and infiltrated in a graded series (25, 50, 75, 90, 100 and 100%) of Durcupan resin (Sigma-Aldrich) in ethanol for 3 min at 250 W.

Infiltrated oocytes were embedded with minimal amount of resin as previously described (15). The polymer coverslip with embedded oocytes on top was cut out of the culture dish using a jigsaw and attached to a SEM stub (Science Services) using silver-filled EPO-TEK EE129-4 adhesive (Electron Microscopy Sciences), and cured overnight at 60°C. Samples were coated with a 10 nm gold layer using the high vacuum sputter coater EM ACE600 (Leica) at 35 mA current. Afterwards, samples were placed in the Crossbeam 540 FIB-SEM (Zeiss). To ensure even milling and to protect the surface, a 500 nm platinum layer was deposited on top of the region of interest at 3 nA current. Atlas 5 (Atlast 3D, Carl Zeiss Microscopy) was used to collect the 3D datasets. Pre-selected oocytes were exposed with a 15 nA current, and a 7 nA current was used to polish the cross-section surface. Images were acquired at 1.5 kV with the ESB detector at a grid voltage of 450 V (5 nm pixel size in x-y) using 700 pA as the milling current (5 nm z-step).

After acquisition, images were first aligned using Linear Stack Alignment with SIFT in Fiji (NIH). Datasets were then cropped, inverted and smoothed with a Gaussian filter of 1 sigma in Fiji. To better visualize microtubules, datasets were further subjected to Local contrast enhancement (CLAHE) in Fiji. Specific parameters used were: 127 for blocksize, 256 for histogram bins and 1.25 for maximum slope. To obtain a spindle parallel to the imaging plane, the resulting image stacks were rotated and resliced with Interactive Stack Rotation in Fiji.

### Immunoblotting

10 - 50 mouse, 12 human, bovine or porcine oocytes per lane were extensively washed in protein-free medium and transferred into 4 µl of protein-free medium. 12 µl of 1.333× NuPAGE LDS sample buffer (Thermo Fisher Scientific) with 100 mM DTT was then added, and the mixture was immediately snap-frozen in liquid nitrogen. 0.125 - 2.5 µg of asychronized HeLa whole cell lysate (NBP2-10274; Novus Biologicals) were used as a positive control.

Samples were thawed at 37°C and snap-frozen in liquid nitrogen twice more before heated at 95°C for 5 min. Samples were resolved on a 17-well NuPAGE 4 - 12% Bis-Tris protein gel of 1.0 mm thickness (Thermo Fisher Scientific) with NuPAGE MOPS SDS Running Buffer (Thermo Fisher Scientific). Proteins were transferred onto a 0.45 µm PVDF membrane with SDS-free Towbin buffer at 200 mA for 2 h on ice. Blots were stained with No-Stain Protein Labeling Reagent (Thermo Fisher Scientific) before blocking. Blocking and antibody incubations were performed in Tris-buffered saline (TBS) with 5% skim milk and 0.1% tween-20. Primary antibodies used were rat anti-α-tubulin, mouse anti-β-actin (ab8226; Abcam), mouse anti-β-tubulin (T8328; Sigma-Aldrich), rabbit anti-γ-tubulin, rabbit anti-GAPDH (ab181602; Abcam), mouse anti-HEC1, rabbit anti-HSET-N (ab172620; Abcam), rabbit anti-HSET-C, mouse anti-LIS1, rabbit anti-NUMA and rabbit anti-PCNT (ab4448; Abcam). Secondary antibodies used were HRP-conjugated anti-mouse (P0447; Dako), anti-rabbit (A9169; Sigma-Aldrich) and anti-rat (sc-2032; Santa Cruz Biotechnology). Blots were developed with SuperSignal West Femto Maximum Sensitivity Substrate (Thermo Fisher Scientific) and documented with Amersham Imager 600 (GE Healthcare). Care was taken that the exposure time did not cause saturation.

### General quantification

Time-lapse movies of live mouse, bovine and porcine oocytes, and fixed human and mouse spindles were analyzed in 3D using Imaris (Bitplane). To determine the stages of meiosis and to score for spindle instability, the timing of meiotic progression was quantified relative to the timing of NEBD. NEBD was defined as the time point when the sharp boundary between the nucleus and cytoplasm disappeared in the differential interference contrast image (for mouse oocytes) or when the nucleus started collapsing in the H2B channel (for bovine and porcine oocytes). Spindle poles were defined as regions of MAP4 or MAP4-MTBD or α-tubulin intensity that prominently protruded from the main microtubule mass. In **Fig.** 1D, oocytes with bipolar spindle, in which ends of all microtubules converged at the poles, were scored as having focused poles. Oocytes with bipolar spindle, in which ends of microtubules failed to converge at the poles, were scored as having unfocused poles. For mouse oocytes, unfocused poles were further classified into mildly unfocused poles (with partially separated microtubule ends) and severely unfocused poles (with fully separated microtubule ends). In **fig.** 4D, bovine and porcine oocytes, in which the spindle maintained a barrel-shaped bipolar morphology and a detectable spindle axis, were scored as having stable spindle poles. Oocytes, in which the spindle lost their initial bipolarity and underwent dynamic remodeling of the pole(s) were scored as having unstable spindle poles. In **Fig.** 1G and 7C, oocytes, in which the spindle had two poles and had a length-to-width ratio larger than 2, were scored as having bipolar spindle. Oocytes, in which the spindle had two poles and had a length-to-width ratio smaller than 2, were scored as having spindle with broad poles. Oocytes, in which the spindle had more than two poles, were scored as having multipolar spindle. In **Fig.** 1H, mouse oocytes, in which the spindle had two well-defined poles, were scored as having bipolar spindle. Mouse oocytes, in which the spindle had two poorly defined poles, were scored as having apolar/ round spindle. Mouse oocytes, in which the spindle had more than two poles, were scored as having multipolar spindle. In **Fig.** 2E, 3H and 7G, mouse oocytes, in which the spindle had a compact NUMA cluster at both poles, were scored as having normal bipolar spindle. Mouse oocytes, in which the spindle had a ribbon-like NUMA cluster at both poles, were scored as having round spindle with broad poles. Mouse oocytes, in which the spindle had a NUMA cluster at more than two poles, were scored as having multipolar spindle. Mouse oocytes, in which the spindle lost its barrel shape but contains several weakly associated microtubule bundles, were scored as having disorganized spindle.

### Quantification of protein enrichment at the spindle pole over the cytoplasm

Line profiles across half spindle were generated along the spindle axis in ZEN, and mean fluorescence intensities were exported into Excel (Microsoft). The intensity of an equivalent ROI in the cytoplasm of the same oocyte was subtracted to correct for cytoplasmic background. Background-corrected data were then normalized by the minimum intensity of each line profile (cytoplasmic intensity). Enrichment at the spindle pole over the cytoplasm was represented by the maximum intensity of each line profile.

### Automatic quantification of standard deviation (SD) of fluorescence intensity within spindle isosurface and microtubule packing index by 3D reconstruction of spindles

To reproducibly reconstruct the spindles (labeled with anti-α-tubulin) from different immunofluorescence experiments, a previously described MATLAB script (6) was used for automatic surface creation in Imaris. Specific parameters used were: 0.1 (for microtubule packing index) or 1.0 (for SD of fluorescence intensity within spindle isosurface) µm for smoothing size, no background subtraction, 500 - 750 and 1000 - 1750 µm³ for minimum and maximum expected total volume of surface object, respectively. SD of fluorescence intensity, volume and volume of the object-oriented minimum bounding box for the spindle isosurface were exported into Excel. Microtubule packing index was calculated by dividing the volume of spindle isosurface by the volume of the object-oriented minimum bounding box.

### Manual quantification of mean fluorescence intensity and volume by 3D reconstruction of spindles

Manual segmentations were performed using the Surface function of Imaris. For acute MLN8237 addition experiments, mClover3-NUMA was smoothed with a surface detail of 1 µm and thresholded after background subtraction with 0.747 µm as the diameter of largest sphere which fits into the object. Spindles (labeled with 3×CyOFP-MAP4-MTBD) were smoothed with a surface detail of 1 µm and thresholded with absolute intensity. For HSET/KIFC1 depletion experiments, NUMA (labeled with anti-NUMA) was smoothed with a surface detail of 0.5 µm and thresholded after background subtraction with 0.5 µm as the diameter of largest sphere which fits into the object. Depending on the cytoplasmic background in each oocyte, a suitable threshold value was selected for the corresponding channel. For time-lapse movies, the threshold value was maintained for the entire time series. Mean fluorescence intensity and volume of isosurfaces were exported into Excel. To normalize individual dataset for time-lapse movies, all mean intensities were divided by the average mean intensity at steady-state.

### Quantification of fluorescence recovery after photobleaching experiments

Minor temporal drift was corrected using Rigid registration in Icy. Mean intensities of photobleached areas over time were exported from Fiji into Excel for further processing. Data were first corrected for background by subtracting the intensity of an area outside the oocytes. Background-corrected data were then normalized to the intensity of pre-bleached time points (F₀). Plots of intensity (F) against time were fitted to single exponential functions [F(t) = c - F_{∞}e^{-t/τ}, where c is the offset, F_{∞} is the amplitude of maximum intensity recovered after equilibrium, and τ is the time constant] in OriginPro (OriginLab). Half-times of maximum recovery (t_{1/2}) and mobile fractions were determined by τ × ln(2) and F_{∞}/(F₀ - F') (where F' is the minimum intensity measured immediately after photobleaching), respectively.

### Quantification of photoactivation experiments

Minor temporal drift was corrected using Rigid registration in Icy. Mean intensities of photoactivated areas over time were exported from Fiji into Excel for further processing. Data were first corrected for cytoplasmic background by subtracting the intensity of the photoactivated area before photoactivation. Background-corrected data were then normalized to the intensity of the first post-activation time point. Plots of intensity against time were fitted to linear functions (y = mx + c, where m is the slope and c is the y-intercept) or one-component exponential functions [y = Ae^{(c-x)/τ}, where c is the offset, A is the fraction of the component and τ is the time constant] in OriginPro. Half-times of decay (t_{1/2}) were calculated by -0.5/m or τ × ln(2).

### Directionality analysis

Directionality of microtubules within spindles were analyzed using OrientationJ in Fiji. Specific parameters used were: 9 pixel for local window, Gaussian for gradient, HSB for color-survey, orientation for Hue, coherency for saturation and original-image for brightness. The output images were inverted for better visualization of the results.

### Re-analysis of previously published RNA-seq data

Previously published bulk and single-cell RNA-seq datasets for oocytes and preimplantation embryos from mice (*16*), cows (*17*), pigs (*18*) and humans (*16, 19*) were downloaded from Gene Expression Omnibus (NIH). To compare gene expression levels between different samples from the same dataset, Reads Per Kilobase of transcript per Million reads mapped (RPKM) or Fragments Per Kilobase of transcript per Million reads mapped (FPKM) was converted into Transcripts Per Million (TPM). TPM has been shown to better represent transcript abundance at the gene level than RPKM and FPKM because it respects the invariance property and is proportional to the average relative RNA molar concentration of the transcript in a sample *(20, 21).*

### Statistical analysis

No statistical methods were used to predetermine sample size. Average (mean) and SD were calculated in Excel. Statistical significance based on unpaired, two-tailed Student's t-test (for absolute values) and two-tailed Fisher's exact test (for categorical values) were calculated in Prism (GraphPad), assuming normal distribution and similar variance. All box plots show median (horizontal black line), mean (small black squares), 25^{th} and 75^{th} percentiles (boxes), 5^{th} and 95^{th} percentiles (whiskers) and 1^{st} and 99^{th} percentiles (crosses). All data are from at least two independent experiments. *P* values are designated as **P*<0.05, ***P<0.01,* ****P*<0.001 and *****P*<0.0001. Nonsignificant values are indicated as N.S.

### Preparation and culture of human zygotes

The use of human 3PN zygotes in this study was approved by the Ärztekammer Niedersachsen under the reference Bo/46/2020. Zygotes were collected from patients who underwent assisted reproduction treatment at Kinderwunschzentrum Göttingen and Fertility Center Berlin. All patients gave informed consent for their 3PN zygotes to be used in this study. For zygotes collected at Kinderwunschzentrum Göttingen, zygotes were cultured in G-1 PLUS medium (Vitrolife) under paraffin oil at 37oC and 5% CO2. For zygotes collected at Fertility Center Berlin, zygotes were vitrified with Cryolock (FUJIFILM Irvine Scientific) using Vit Kit-Freeze (FUJIFILM Irvine Scientific). To maximize the survival and developmental rate of vitrified-thawed zygotes, zygotes were thawed in 1 ml of prewarmed G-MOPS PLUS (Vitrolife) containing 1 M D-(+)-trehalose (Sigma-Aldrich) at 37°C for 1 min. Zygotes were then transferred to 300 µl of G-MOPS PLUS containing 0.5 M D-(+)-trehalose at room temperature for 3 min, 300 µl of G-MOPS PLUS containing 0.25 M D-(+)-trehalose at room temperature for 5 min, and 300 µl of G-MOPS PLUS at room temperature for 2 min. Recovered zygotes were cultured in G1-PLUS supplemented with 10% FBS under paraffin oil at 37°C and 5% CO2. Staining and microinjection of human zygotes were performed as described above for human oocytes.

**Tables**

| Sequences of HSET/KIFC1 | | |
|---|---|---|
| SEQ ID NO | Name | Sequence |
| 1 | Human KIFC1/HSET protein; Q9BW19 Kinesin-like protein KIFC1 | MDPQRSPLLEVKGNIELKRPLIKAPSQLPLSGSRLKRRPDQMEDGLEPEKKRTRGLGATT KITTSHPRVPSLTTVPQTQGQTTAQKVSKKTGPRCSTAIATGLKNQKPVPAVPVQKSGTS GVPPMAGGKKPSKRPAWDLKGQLCDLNAELKRCRERTQTLDQENQQLQDQLRDAQQQVKALGTERT TLEGHLAKVQAQAEQGQQELKNLRACVLELEERLSTQEGLVQELQKKQVELQEERRGLMSQLEEKERRL QTSEAALSSSQAEVASLRQETVAQAALLTEREERLHGLEMERRRLHNQLQELKGNIRVFCRVRPVLPGE PTPPPGLLLFPSGPGGPSDPPTRLSLSRSDERRGTLSGAPAPPTRHDFSFDRVFPPGSGQDEVFEEIAML VQSALDGYPVCIFAYGQTGSGKTFTMEGGPGGDPQLEGLIPRALRHLFSVAQELSGQGWTYSFVASYVE IYNETVRDLLATGTRKGQGGECEIRRAGPGSEELTVTNARYVPVSCEKEVDALLHLARQNRAVARTAQN ERSSRSHSVFQLQISGEHSSRGLQCGAPLSLVDLAGSERLDPGLALGPGERERLRETQAINSSLSTLG LVIMALSNKESHVPYRNSKLTYLLQNSLGGSAKMLMFVNISPLEENVSESLNSLRFASKV NQCVIGTAQANRK |
| 2 | human KIFC1/HSET mRNA; >NM_002263 .4 Homo sapiens kinesin family member C1 (KIFC1), mRNA | ATGGATCCGCAGAGGTCCCCCCTATTGGAAGTAAAGGGGAACATAGAACTGAAGAGACCTCTGATTA AGGCCCCTTCCCAGCTGCCTCTCTCAGGAAGCAGACTCAAGAGGAGGCCTGACCAGATGGAAGATG GCCTGGAGCCTGAGAAGAAACGGACAAGAGGCCTGGGTGCAACGACCAAAATTACCACATCCCACCC AAGAGTTCCATCCCTCACTACAGTGCCACAGACACAAGGCCAGACCACAGCTCAAAAAGTTTCCAAG AAGACAGGACCCCGGTGTTCCACAGCTATTGCCACAGGGTTGAAGAACCAGAAGCCAGTTCCTGCTG TTCCTGTCCAGAAGTCTGGCACATCAGGTGTTCCTCCCATGGCAGGAGGGAAGAAACCCAGCAAACG TCCAGCCTGGGACTTAAAGGGTCAGTTATGTGACCTAAATGCAGAACTAAAACGGTGCCGTGAGAG GACTCAAACGTTGGACCAAGAGAACCAGCAGCTTCAGGACCAGCTCAGAGATGCCCAGCAGCAGGTC AAGGCCCTGGGGACAGAGCGCACAACACTGGAGGGGCATTTAGCCAAGGTACAGGCCCAGGCTGAG CAGGGCCAACAGGAGCTGAAGAACTTGCGTGCTTGTGTCCTGGAGCTGGAAGAGCGGCTGAGCACG CAGGAGGGCTTGGTGCAAGAGCTTCAGAAAAAACAGGTGGAATTGCAGGAAGAACGGAGGGGACTG ATGTCCCAACTAGAGGAGAAGGAGAGGAGGCTGCAGACATCAGAAGCAGCCCTGTCAAGCAGCCAA GCAGAGGTGGCATCTCTGCGGCAGGAGACTGTGGCCCAGGCAGCCTTACTGACTGAGCGGGAAGAA CGTCTTCATGGGCTAGAAATGGAGCGCCGGCGACTGCACAACCAGCTGCAGGAACTCAAGGGCAAC ATCCGTGTATTCTGCCGGGTCCGCCCTGTCCTGCCGGGGGAGCCCACTCCACCCCCTGGCCTCCTCC TGTTTCCCTCTGGCCCTGGTGGGCCCTCTGATCCTCCAACCCGCCTTAGCCTCTCCCGGTCTGACGA GCGGCGTGGGACCCTGAGTGGGGCACCAGCTCCCCCAACTCGCCATGATTTTTCCTTTGACCGGGTA TTCCCACCAGGAAGTGGACAGGATGAAGTGTTTGAAGAGATTGCCATGCTTGTCCAGTCAGCCCTGG ATGGCTATCCAGTATGCATCTTTGCCTATGGCCAGACAGGCAGTGGCAAGACCTTCACAATGGAGGG TGGGCCTGGGGGAGACCCCCAGTTGGAGGGGCTGATCCCTCGGGCCCTGCGGCACCTCTTCTCTGT GGCTCAGGAGCTGAGTGGTCAGGGCTGGACCTACAGCTTTGTAGCAAGCTACGTAGAGATCTACAA TGAGACTGTCCGGGACCTGCTGGCCACTGGAACCCGGAAGGGTCAAGGGGGCGAGTGTGAGATTCG CCGTGCAGGGCCAGGGAGTGAGGAGCTCACTGTCACCAATGCTCGATATGTCCCTGTCTCCTGTGA GAAAGAAGTGGACGCCCTGCTTCATCTGGCCCGCCAGAATCGGGCTGTGGCCCGCACAGCCCAGAA TGAACGGTCATCACGCAGCCACAGTGTATTCCAGCTACAGATTTCTGGGGAGCACTCCAGCCGAGGC CTGCAGTGTGGGGCCCCCCTCAGTCTTGTGGACCTGGCCGGGAGTGAGCGACTTGACCCCGGCTTA GCCCTCGGCCCCGGGGAGCGGGAACGCCTTCGGGAAACACAGGCCATTAACAGCAGCCTGTCCACG CTGGGGCTGGTTATCATGGCCCTGAGCAACAAGGAGTCCCACGTGCCTTACCGGAACAGCAAACTGA CCTACCTGCTGCAGAACTCTCTGGGTGGTAGTGCTAAGATGCTCATGTTTGTGAACATTTCTCCACT GGAAGAGAACGTCTCCGAGTCCCTCAACTCTCTACGCTTTGCCTCCAAGGTGAACCAGTGTGTTATT GGTACTGCTCAGGCCAACAGGAAGTGA |
| 3 | Bovine: Bos taurus kinesin family member C1 (KIFC1), locus NM_0011019 36; translated sequence | MEPQRSPLLEVKGNVELKRPLAKAASRLPLSGRRLKRGPDQMEEALEPEKKRTRGLGTRVTTTHPRAAA LSSAPQTQGQTAVPKAPRKPAPRCSTAVAPVLKTQKPGPVVPVQKPGTTAAPPMVGGKKPKRPAWDLK GQLCDLNAELKCYRERKQVLDQENQQLRDQLQEAQQQALALGAERKTLEEELTRVRAQAEQGQRELG NLSARVLELEERLGTQEGLVQELQKEQLRLQEERRGLAARLGEQERRLQTSEASLSDSQEEVASLRQEA AAREAILAEREDRLHGLEMERRRLHNQLQELKGNIRVFCRVRPVLPGESTPSPGFLQFPSGPCGPSDPPT RLSLSRSDERRGTLSGAPAGPTRHEFSFDRVFPPGSGQDEVFEEISMLVQSALDGYPVCIFAYGQTGSGK TFTMEGGPGGDPQMEGLIPRALRHLFSVAQELSGQGWTYSFVASYVEIYNETVRDLLATGTRKGQGGE CEIRRAGPGSEELTVTNARYVPVSCEREVEALLHLARQNRAVARTAQNERSSRSHSVFQLQISGEHASR GLQCAAPLSLVDLAGSERLDPGLALGPGERERLRETQAINSSLSTLGLVIMALSNKESHVPYRNSKLTYLL QNSLGGSAKMLMFVNISPLEENVSESLNSLRFASKVNQCVIGTAQANKK |
| | | |
| 4 | Porcine: Sus scrofa kinesin family member C1 (KIFC1), XM_0210985 94; translated sequence | METLQRSPLLEVKGNIEVKRPLPKPPSRLPLSGSRLKRGPEQMEEALEPEKKRTRGLGTKIAPSRPRAALL TAVPQTQGQAAARTAPKKTGPRCPTAVATVLKNQKPGPAVPAQKPGTAAPPMVGGKKPSKRPAWDLK GQLCDRMAELKCCRERNQILDQENQQLRDQLREAQQQASTLGAERRTLEEELARVRVQAEQGQQELG RLSAQVLELEERLGTQEGLVQVLQREQLGLQEERRGLATRLEEQERRLQASEAALSDSQAEVASLRQEA AAQAVLLAEREDRLHGLEMERRRLHNQLQELKGNIRVFCRVRPVLPGEPTPNPGFLLFPGGPADPPTRLS LSRSDERRGTLSGAPAPPTRHDFSFDRVFPPGSRQDEVFEEIAMLVQSALDGYPVCIFAYGQTGSGKTFT MEGGPAGDPQVEGLIPRALRHLFSVAQELSGQGWTYSFVASYVEIYNETVRDLLATGTRKGQAGECEIR RAGPGSEELTVTNARYVPVSCEKEVQALLHLARQNRAVARTAQNERSSRSHSVFQLQISGEHAARGLQC IAPLSLVDLAGSERLDSGLALGPGERERLRETQSINSSLSTLGLVIMALSNKEPHVPYRNSKLTYLLQNSL GGSAKMLMFVNISPLEENVSESLNSLRFASKVNQCVIGTAQANKK |
| 5 | Mouse KIFC1/HSET protein Q9QWT9 KIFC1_MOUS E Kinesin-like protein KIFC1 | MDVQAQRPPLLEVKRNVELKAALVKSSSRVPLSASRLKRGPDQMEDALEPAKKRTRVMGAVTKVDTSR PRGPLLSTVSQTQGHTAAQKGPKKTGPRGCSAIGTVLRSQKPVPAAPAQKPGTSTAPVVVGKRAGKRPA WDLKGQLCDLNEELKRYREKTQTLELENRGLREQLREVQEQATTLGTERNTLEGELASVRSRAEQDQQ RLETLSARVLELEECLGTRERLLQELQGERLQLQEERSTLSTQLEEQERRFQATEAALSSSQEEVVCLRQ KTEAQVTLLAEQGDRLYGLEMERRRLHNQLQELKGNIRVFCRVRPVLEGESTPSPGFLVFPPGPAGPSDP PTGLSLSRSDDRRSTLTGAPAPTVRHDFSFDRVFPPGSKQEEVFEEIAMLVQSALDGYPVCIFAYGQTGS GKTFTMEGGPRGDPQLEGLIPRAMRHLFSVAQEMSGQGWTYSFVASYVEIYNETVRDLLATGPRKGQG GECEIRRASPGSEELTVTNARYVPVSCEKEVEALLHLAHQNRAVAHTAQNKRSSRSHSVFQLQISGEHA ARGLQCGAPLNLVDLAGSERLDPGLHLGPGERDRLRETQAINSSLSTLGLVIMALSNKESHVPYRNSKLT YLLQNSLGGSAKMLMFVNISPLEENVSESLNSLRFASKVNQCVIGTAQANKK |
| 6 | Bovine: Bos taurus kinesin family member C1 (KIFC1), mRNA NM_0011019 36.2 | ATGGAGCCGCAGAGGTCCCCGTTGTTGGAAGTGAAGGGGAACGTAGAGCTGAAGAGACCCCTGGCT AAGGCTGCTTCCCGGCTGCCTCTCTCGGGACGCAGGCTGAAGAGGGGGCCTGACCAGATGGAGGAG GCCTTGGAGCCGGAGAAGAAAAGAACACGAGGCCTGGGCACCAGAGTGACCACGACCCACCCCAGA GCAGCAGCCCTCAGCTCCGCGCCACAGACACAAGGCCAGACCGCAGTGCCCAAAGCTCCCAGGAAGC CAGCACCCCGATGTTCCACGGCTGTTGCCCCAGTGCTGAAGACTCAGAAGCCAGGTCCTGTTGTTCC CGTCCAGAAGCCTGGAACGACAGCCGCACCTCCCATGGTGGGAGGGAAGAAACCCAAACGTCCGGC CTGGGACTTAAAGGGGCAGTTATGTGACCTCAATGCAGAGCTGAAATGCTACCGTGAGAGGAAGCA GGTGCTGGACCAGGAGAACCAACAGCTGCGGGACCAGCTCCAGGAGGCCCAGCAACAGGCCTTGGC CCTGGGGGCAGAGCGTAAGACCCTGGAAGAGGAGTTGACCAGGGTGCGGGCCCAGGCTGAGCAGG GCCAGCGGGAGCTGGGGAACCTGAGTGCCCGTGTCCTGGAGCTGGAAGAGCGGCTGGGCACGCAG GAGGGCTTAGTGCAAGAGCTCCAGAAGGAACAGCTGAGATTGCAGGAGGAGCGCAGGGGACTGGCT GCCCGGCTGGGAGAGCAGGAGCGGAGGCTGCAGACCTCAGAAGCTTCTCTGTCGGACAGCCAGGAA GAGGTGGCATCTCTGCGCCAGGAGGCTGCAGCCCGGGAGGCCATACTGGCTGAGCGGGAAGACCGT CTCCACGGGCTCGAGATGGAGCGCAGGCGGTTACACAACCAGCTGCAGGAACTCAAAGGCAACATC CGTGTGTTCTGCCGGGTCCGCCCCGTCCTTCCAGGGGAGTCCACCCCATCCCCTGGCTTCCTCCAGT TTCCCTCTGGCCCCTGTGGACCCTCTGACCCTCCAACCCGCCTCAGCCTCTCGCGGTCTGACGAGCG GCGTGGGACCCTGAGTGGGGCGCCAGCTGGCCCTACCCGCCATGAGTTCTCCTTTGACCGGGTGTT CCCACCAGGGAGTGGACAGGATGAAGTGTTTGAGGAGATTTCCATGCTTGTCCAGTCAGCCCTGGA TGGCTATCCAGTGTGCATCTTTGCTTATGGCCAGACAGGCAGTGGCAAGACCTTCACCATGGAGGGT GGGCCTGGGGGAGACCCCCAGATGGAGGGGCTGATCCCTCGTGCCCTGCGACACCTCTTTTCTGTG GCCCAGGAGCTAAGTGGCCAGGGCTGGACCTACAGCTTTGTGGCAAGTTACGTAGAGATCTACAAC GAGACTGTCCGAGACCTGCTGGCCACTGGGACCCGGAAGGGCCAGGGGGGTGAGTGTGAGATTCG CCGGGCAGGGCCAGGCAGCGAGGAGCTCACTGTCACCAACGCGCGCTATGTTCCGGTCTCCTGTGA GAGGGAGGTGGAAGCCCTGCTCCATCTAGCCCGCCAGAACCGCGCTGTAGCCCGCACAGCCCAGAA TGAGCGCTCGTCGCGTAGTCACAGCGTGTTCCAGCTGCAGATCTCTGGGGAGCACGCTAGCCGAGG CCTGCAGTGTGCGGCCCCCCTCAGCCTGGTGGACTTGGCTGGGAGTGAGCGGCTAGACCCCGGCTT AGCACTCGGCCCTGGGGAGCGGGAACGCCTTCGGGAAACACAGGCCATTAACAGCAGCCTGTCCAC GCTGGGGCTGGTCATCATGGCCTTGAGCAACAAGGAGTCCCATGTGCCTTACCGGAACAGCAAGCT CACCTACCTGCTGCAGAACTCTCTGGGTGGCAGTGCCAAGATGCTCATGTTTGTGAACATTTCTCCC CTAGAAGAGAACGTCTCCGAGTCCCTCAACTCCCTACGCTTCGCCTCCAAGGTGAACCAGTGTGTTA TTGGCACTGCCCAGGCCAACAAGAAATGA |
| 7 | Porcine: Sus scrofa kinesin family member C1 (KIFC1), | ATGGAGACGCTGCAGAGGTCGCCCTTGTTGGAAGTGAAGGGGAACATAGAGGTAAAGAGACCCCTG CCCAAGCCCCCTTCCCGGCTGCCCTTGTCAGGAAGCAGACTTAAGAGGGGGCCTGAGCAGATGGAG GAGGCCTTGGAACCTGAGAAGAAAAGGACGCGAGGCCTGGGCACCAAAATCGCCCCGTCCCGCCCC AGAGCAGCGTTGCTCACCGCAGTGCCTCAGACGCAGGGCCAGGCTGCAGCGCGCACGGCTCCCAAG AAGACAGGACCCCGCTGTCCCACAGCTGTTGCCACAGTGCTGAAGAATCAGAAGCCAGGCCCTGCTG TTCCTGCCCAGAAGCCTGGCACAGCTGCCCCTCCCATGGTGGGAGGGAAGAAGCCCAGCAAACGTC CGGCCTGGGACTTAAAGGGTCAGTTATGTGACCGAATGGCAGAGCTGAAATGCTGCCGTGAGAGGA |
| | mRNA XM_0210985 94.1 | ATCAGATTTTGGATCAGGAGAACCAACAGCTGCGGGACCAGCTTAGGGAGGCCCAGCAGCAGGCCT CGACCCTGGGCGCAGAGCGCCGGACACTGGAAGAGGAGTTGGCCCGGGTTCGGGTCCAGGCTGAG CAGGGCCAGCAGGAGCTGGGGAGGCTGAGTGCCCAGGTTCTGGAGCTGGAGGAGCGGCTGGGCAC GCAGGAGGGCTTGGTGCAAGTGCTTCAGAGAGAGCAGCTGGGGCTGCAGGAGGAGCGGAGGGGGC TGGCCACCCGGCTGGAGGAGCAGGAGCGGAGGCTGCAGGCATCGGAGGCAGCGCTGTCGGACAGC CAGGCGGAGGTGGCATCTCTTCGCCAGGAGGCTGCGGCCCAGGCAGTCTTGCTGGCCGAGCGGGAA GACCGCCTGCACGGGCTGGAGATGGAGCGCCGGCGGCTCCACAACCAGCTTCAGGAGCTCAAAGGC AACATCCGCGTGTTCTGCCGTGTCCGCCCAGTCCTCCCTGGGGAGCCCACCCCGAACCCCGGCTTCC TCCTGTTCCCTGGCGGGCCCGCTGACCCTCCAACACGCCTCAGCCTCTCCCGGTCTGACGAACGGCG TGGGACCCTGAGTGGGGCACCGGCCCCCCCCACGCGACACGACTTCTCCTTTGACCGGGTCTTCCCA CCAGGGAGCAGGCAGGATGAAGTGTTTGAGGAGATTGCCATGCTTGTGCAGTCTGCCCTGGATGGT TACCCAGTGTGCATCTTTGCCTATGGCCAGACCGGCAGTGGCAAGACCTTCACCATGGAGGGTGGG CCTGCGGGAGACCCCCAGGTGGAGGGACTGATCCCCCGGGCCCTGCGGCACCTCTTCTCTGTGGCA CAGGAGCTCAGTGGCCAGGGCTGGACCTACAGCTTTGTGGCCAGTTACGTCGAGATCTACAACGAG ACTGTCCGAGACCTGCTGGCCACTGGAACCCGGAAGGGCCAGGCGGGTGAGTGTGAGATTCGCCGG GCAGGGCCAGGGAGTGAGGAGCTTACTGTCACCAACGCCCGCTACGTTCCTGTCTCCTGTGAGAAA GAGGTACAGGCCCTGCTCCATCTGGCCCGCCAGAACCGGGCTGTGGCCCGCACAGCCCAGAATGAA CGGTCATCACGCAGTCATAGCGTGTTCCAGCTGCAGATCTCTGGGGAACACGCGGCCCGAGGCCTG CAGTGCATCGCCCCGCTCAGTCTTGTGGACCTGGCCGGGAGCGAGCGGCTGGATTCCGGCCTCGCC CTTGGCCCTGGGGAGCGGGAACGCCTTCGGGAAACACAGTCCATTAACAGCAGCTTGTCCACACTG GGGCTGGTCATCATGGCCTTGAGCAACAAGGAGCCCCATGTGCCTTACCGGAACAGCAAGCTCACCT ACCTGCTGCAGAACTCTCTGGGTGGCAGCGCTAAGATGCTCATGTTCGTGAACATCTCCCCCCTGGA AGAGAATGTCTCTGAGTCTCTCAACTCCTTGCGCTTTGCTTCCAAGGTGAACCAATGTGTCATTGGT ACTGCCCAAGCCAACAAGAAATGA |
| 8 | Mouse: kinesin family member C1 (Kifc1), NM_0011952 98.1; Mus musculus | ATGGACGTGCAGGCGCAGAGGCCACCTTTGTTGGAAGTGAAGAGGAACGTAGAACTGAAGGCAGCC CTGGTGAAGTCCTCCTCCCGAGTGCCCCTGTCAGCAAGCAGGCTCAAGAGGGGTCCTGACCAGATG GAGGATGCCTTGGAGCCTGCAAAGAAACGGACACGAGTCATGGGTGCAGTGACCAAAGTTGACACA TCCCGTCCCAGAGGACCACTCCTCAGCACAGTGTCACAGACCCAGGGCCACACTGCAGCTCAGAAAG GCCCTAAGAAGACAGGACCTCGTGGGTGCTCTGCTATTGGTACAGTGCTGAGGAGCCAGAAGCCAG TTCCCGCTGCTCCTGCCCAGAAGCCTGGCACATCCACTGCTCCTGTGGTGGTAGGGAAGAGAGCTG GCAAACGCCCTGCCTGGGACCTGAAGGGCCAGTTGTGTGACCTCAATGAAGAGTTGAAACGCTATC GGGAGAAGACTCAAACGCTGGAACTGGAGAACCGGGGTCTTCGGGAGCAACTCAGAGAGGTCCAGG AGCAGGCCACGACCCTGGGGACAGAGCGGAACACCCTGGAAGGGGAGCTGGCCAGTGTACGCAGCC GAGCTGAGCAGGACCAGCAGAGGCTGGAGACGCTGAGTGCCCGTGTCTTGGAGCTGGAGGAATGTC TGGGTACCAGGGAAAGGCTGCTTCAGGAGCTTCAGGGAGAGCGGCTGCAATTGCAGGAGGAGCGG AGCACACTGAGCACCCAGCTGGAGGAGCAGGAGAGGAGGTTTCAGGCCACAGAAGCAGCTCTGTCA AGCAGCCAAGAAGAGGTGGTGTGTCTTCGGCAGAAGACTGAAGCCCAGGTGACCTTACTGGCTGAG CAAGGAGACCGGCTCTATGGGTTAGAGATGGAGCGGCGACGACTCCACAACCAGCTGCAGGAACTG AAGGGCAATATCCGGGTGTTCTGCCGCGTGCGCCCTGTCCTCGAAGGGGAATCCACTCCATCTCCTG GCTTCCTCGTGTTTCCTCCTGGCCCTGCTGGACCCTCTGATCCCCCGACGGGCCTTAGCCTCTCACG ATCTGATGATCGGCGCTCCACCCTGACTGGGGCCCCGGCCCCCACTGTCCGCCATGATTTCTCCTTT GATCGGGTGTTCCCGCCGGGAAGCAAGCAGGAGGAAGTGTTTGAGGAGATCGCCATGCTTGTCCAG TCAGCACTGGATGGCTACCCTGTGTGCATTTTTGCCTATGGACAGACAGGCAGTGGCAAGACCTTCA CTATGGAAGGAGGGCCTAGGGGAGACCCCCAATTGGAAGGGCTGATCCCTCGGGCCATGCGGCATC TGTTCTCTGTGGCCCAGGAGATGAGCGGCCAGGGCTGGACATACAGTTTTGTGGCGAGTTACGTAG AGATCTACAATGAGACCGTTCGAGACCTGCTAGCTACTGGGCCCCGCAAGGGACAAGGGGGCGAGT GTGAGATCCGTCGGGCAAGCCCAGGAAGTGAGGAGCTTACTGTCACCAATGCCCGCTATGTCCCTG TTTCCTGTGAGAAAGAGGTGGAGGCCCTGCTCCATTTGGCTCATCAGAACCGGGCTGTGGCCCACAC TGCCCAAAATAAGAGATCATCACGCAGTCATAGTGTGTTCCAGCTGCAGATTTCTGGAGAGCATGCA GCTCGGGGCCTGCAGTGTGGCGCTCCGCTCAACCTTGTGGACCTAGCTGGGAGTGAGCGGCTAGAC CCTGGCTTACACCTAGGCCCTGGGGAGCGTGATCGTCTTCGGGAGACACAGGCCATTAACAGCAGT CTGTCTACACTGGGACTGGTCATAATGGCCCTGAGCAATAAGGAGTCCCACGTGCCTTACCGAAACA GCAAGCTCACCTACTTGCTGCAGAACTCTCTGGGTGGCAGTGCCAAGATGCTTATGTTTGTGAATAT TTCTCCTCTGGAAGAGAATGTCTCCGAGTCTCTGAATTCACTACGCTTTGCTTCCAAGGTGAACCAG TGTGTCATTGGTACTGCTCAGGCTAATAAGAAGTGA |
| 9 | Kifc1 I0FRK2, Kinesin-like protein; Macaca mulatta, | MDPQRSPLLEVKGNIELKRPRIKAPSRLPLSGSRLKRRPDQMEDGLEPEKKRTRGLGATTKITTSHPRVP SLTTVPQTQGQTTAQKVSKKTGPRCSTAIATGLKNQKPVPAVPVQKPGTSAVPPMAGGKKPSKRPAWD LKGQLCDLNAELKRCRERTQTLDQENQQLQDQLRDAQQQAKALGTERRTLEGHLAKVQAQAEQGQQE LKNLRACVLELEERLSTQEGLVQELQKKQVELQEERRGLTSQLEEKERRLQTSEAALSSSRAEVASLWQE TAAQAALLAEREERLHGLEMERRRLHNQLQELKGNIRVFCRVRPVLPGEPTPPPGLLLFPSGPGGPSDPP TRLSLSRSDERRGTLSGAPAPPTRHDFSFDRVFPPGSGQDEVFEEIAMLVQSALDGYPVCIFAYGQTGS GKTFTMEGGPGGDPQLEGLIPRALRHLFSVAQELSGQGGTYSFVASYVEIYNETVRDLLATGTRKGQGG ECEIRRAGPGSEELTVTNARYVPVSCEKEVEALLHLARQNRAVARTAQNERSSRSHSVFQLQISGEHSSR GLQCGASLSLVDLAGSERLDPGLALGPGERERLRETQAINSSLSTLGLVIMALSNKESHVPYRNSKLTYLL QNSLGGSAKMLMFVNISPLEENVSESLNSLRFASKVNQCVIGTAQANRK |

### siRNAs used in this study

| **SEQ ID NO** | **Target gene** | **Name** | **Sequence** |
|---|---|---|---|
| 10 | ASPM | Mm_Calmbp1_1 | AAGGTTGAAATCGAAATTGAA |
| 11 | | Mm_Calmbp1_2 | TTGGAATTTCTTATTAGTAAA |
| 12 | | Mm_Calmbp1_3 | CAGGAGGGTTCTAGCATCTTA |
| 13 | | Mm_Calmbp1_4 | CACAAGAAATATCTAACTTTA |
| 14 | CENPE | Mm_Cenpe_2 | CAAGGCTACAATGGTACTATA |
| 15 | | Mm_Cenpe_3 | AAGCATTGGGCTCGTGAATAA |
| 16 | | Mm_Cenpe_6 | CAGCAACTTCTTAGTACACAA |
| 17 | CLASP1+2 | Mm_Clasp1_2 | ACCGAGAGCAGTGTACGGAAA |
| 18 | | Mm_Clasp1_4 | CAGGTTCGAGACGCAGCAATA |
| 19 | | Mm_Clasp1_5 | CTGGTATTGAATGTAACGGAA |
| 20 | | Mm_Clasp2_2 | AAGGAGGATGGTGACACAATA |
| 21 | | Mm_Clasp2_3 | CAGGAAGAGTTCTAACCACAA |
| 22 | | Mm_Clasp2_4 | AACGCCAAGCTTGAAGGTAAA |
| 23 | CYK4 | Mm_Racgap1_2 | AAGATGGATATTGCCAATCTA |
| 24 | | Mm_Racgap1_3 | CCGGATGGAGATTATCAATGA |
| 25 | | Mm_Racgap1_4 | CTGGAGAAATTCAAAGACCTA |
| 26 | DLG5/HURP | Mm_Dlgap5_1 | AGCAAGGATTGGAGTCGCTAA |
| 27 | | Mm_Dlgap5_2 | CCCGAACAGTGTCATCCACTA |
| 28 | | Mm_Dlg7_3 | CTGGACGGATTACAAGATCAA |
| 29 | | Mm_Dlg7_4 | ATGGATTGTTTCTCTGTTGAA |
| 30 | HAUS6 | Mm_6230416J20Rik_1 | TTGAGTGGTATTGGTATATTA |
| 31 | | Mm_6230416J20Rik_2 | CAGAGTGATGTTGATGGGATA |
| 32 | | Mm_6230416J20Rik_3 | CCAGAATTAGATTCTAATTTA |
| 33 | | Mm_6230416J20Rik_4 | TAGACTGAGAATTTAATTCTA |
| 34 | KIF2A | Mm_Kif2a_2 | AAGGAGTGCATCCGAGCCTTA |
| 35 | | Mm_Kif2a_3 | CTGCTGGACCATTCCATCTTA |
| 36 | | Mm_Kif2a_5 | CAGGATGTTGATGCTACAAAT |
| 37 | | Mm_Kif2a_7 | CAGGAATGGCATCCTGTGAAA |
| 38 | KIF4A | Mm_Kif4_2 | AAGAATTGGCTTGGAAATGAA |
| 39 | | Mm_Kif4_3 | TACGATGAAATACATGGTCAA |
| 40 | | Mm_Kif4_4 | CAGGAACTGGAGGGTCAAATA |
| 41 | KIF11/EG5 | Mm_Kif11_2 | AGCAAAGAACATAATGAATAA |
| 42 | | Mm_Kif11_3 | CACAGGAACTTTGCCAGTTAA |
| 43 | | Mm_Kif11_4 | TTCCATCTTGAACATAAATAA |
| 44 | KIF12 | Mm_Kif12_2 | AAGGCATATTAGAAAGGGAAA |
| 45 | | Mm_Kif12_3 | AAGGCCTCTCTTCTTGTTAAA |
| 46 | | Mm_Kif12_4 | AACCTGAGTCTCGGTTCACAA |
| 47 | KIF14 | Mm_Kif14_4 | CTGGCTGGAACTGGGAAATAA |
| 48 | | Mm_Kif14_7 | ATGGATTAAGTTTATGTGAAA |
| 49 | | Mm_Kif14_8 | TCGGCTTGAGGCAGAAATTAA |
| 50 | | Mm_Kif14_9 | AAGCAGCATCTTGAACAGGAA |
| 51 | KIF15/HKLP2 | Mm_Knsl7_1 | AGGCTGGATAATGATATATTA |
| 52 | | Mm_Knsl7_2 | CAGGATTCCTACGATAACTTA |
| 53 | | Mm_Knsl7_3 | CACAATAGAATCAATGGAGAA |
| 54 | | Mm_Knsl7_4 | CACATGGTAGAACTAAACTAA |
| 55 | KIF18A | Mm_Kif_18a_2 | TGCATTGTAAATATTGTTTAA |
| 56 | | Mm_Kif18a_3 | CAGATTTATTTGCGACAACAA |
| 57 | | Mm_Kif18a_4 | TTGGAGGAAACTGTCAAACTA |
| 58 | KIF20A/MLKP2 | Mm_Kif20a_1 | CAGGAAGTTAAAGCTGAACTA |
| 59 | | Mm_Kif20a_2 | CAGCTAGATGAAACAAGTCAA |
| 60 | | Mm_Kif20a_3 | CTCCTTTGCCTTGAAGAGTAA |
| 61 | | Mm_Kif20a_4 | AACGGCAATCCTTACGTGAAA |
| 62 | KIF20B/MPP1 | Mm_Mphospho1_1 | AAGGAGGAAAGTGCTAACAAA |
| 63 | | Mm_Mphospho1_2 | CAGGACTTAGATATGAAACAA |
| 64 | | Mm_Mphospho1_3 | ACGGTAGAAGTAAGTAAAGAA |
| 65 | | Mm_Mphospho1_4 | CAGATAGAAGATTCTGAAATA |
| 66 | KIF23/MKLP1 | Mm_Kif23_1 | AAGGCTGAAGACTATGAAGAA |
| 67 | | Mm_Kif23_2 | TTGTTTGAATATGATCTTTAA |
| 68 | | Mm_Kif23_3 | TACGATCTATGAGGAAGATAA |
| 69 | | Mm_Kif23_4 | CTGAGTCATCTTGCAGAAGAA |
| 70 | KIFC1/HSET | Mm_LOC100502766_1 | TACACTGGGACTGGTCATAAT |
| 71 | | Mm_LOC100502766_2 | GTGAACAATATTTATTATGTA |
| 72 | | Mm_LOC100502766_3 | CAGAGCCTGATTCCCTTGCAA |
| 73 | | Mm_LOC100502766_4 | CTGCGCGGAGGTTGAAATTCA |
| 74 | KIFC2 | Mm_Kifc2_2 | CAGGCATTTGAGAGAGGGCAA |
| 75 | | Mm_Kifc2_3 | CAGTGTCTGCATCTTCACTTA |
| 76 | | Mm_Kifc2_4 | CAGTATGGTGGAGATCTACAA |
| 77 | KIFC3 | Mm_Kifc3_2 | CCGCACCACCGAGTTCACCAA |
| 78 | | Mm_Kifc3_3 | CAGAGGTCTGGGCTATATTTA |
| 79 | | Mm_Kifc3_7 | GGGCATGTATATAATGTTCTA |
| 80 | NUMA | Mm_Numa1_1 | CAGGATAAGAAATGTCTTGAA |
| 81 | | Mm_Numa1_2 | CAGCTGGACACTCTAAAGCAA |
| 82 | | Mm Numal 3 | AATGTTATTACTTGTAAATAA |
| 83 | | Mm_Numa1_4 | CTGGAAGTAGAACTAGATCAA |
| 84 | PCNT | Mm_Pcnt2_2 | AAGGAGATCCATGCAAAGCAA |
| 85 | | Mm_Pcnt2_3 | CCGCCAGATTCTACTCAGAAA |
| 86 | | Mm_Pcnt2_4 | TGGGATGTAATTGATATTATT |
| 87 | PRC1 | Mm_Prc1_1 | AAGGCTAGTTATGATAGTTAA |
| 88 | | Mm_Prc1_2 | CTGGAGAACATTGCAACATTA |
| 89 | | Mm_Prc1_3 | TCCGGGAAATATGGGAACTAA |
| 90 | | Mm_Prc1_4 | CTGGATAGAATGATTGCTGAA |
| 91 | TPX2 | Mm_Tpx2_1 | CACGGGAACTTGATCCTAGAA |
| 92 | | Mm_Tpx2_2 | CAAGGCAAATCCAATACGGAA |
| 93 | | Mm_Tpx2_5 | CAGAGGATGCACTATCATTAA |
| 94 | | Mm_Tpx2_8 | CAGGTTGAAGCCTTCCACAAA |

### References

1. J. R. Gruhn et al., Chromosome errors in human eggs shape natural fertility over reproductive life span. Science 365, 1466-1469 (2019).
2. Z. Holubcova, M. Blayney, K. Elder, M. Schuh, Human oocytes. Error-prone chromosome-mediated spindle assembly favors chromosome segregation defects in human oocytes. Science 348, 1143-1147 (2015).
3. J. Haverfield et al., Tri-directional anaphases as a novel chromosome segregation defect in human oocytes. Hum Reprod 32, 1293-1303 (2017).
4. H. Balakier, Tripronuclear human zygotes: the first cell cycle and subsequent development. Hum Reprod 8, 1892-1897 (1993).
5. Y. F. Gu, G. Lin, C. F. Lu, G. X. Lu, Analysis of the first mitotic spindles in human in vitro fertilized tripronuclear zygotes after pronuclear removal. Reprod Biomed Online 19, 745-754 (2009).
6. Y. Kai, H. Moriwaki, K. Yumoto, K. Iwata, Y. Mio, Assessment of developmental potential of human single pronucleated zygotes derived from conventional in vitro fertilization. J Assist Reprod Gen 35, 1377-1384 (2018).
7. E. Ford et al., The First Mitotic Division of the Human Embryo is Highly Error-prone. bioRxiv, 2020.2007.2017.208744 (2020).
8. T. Gaglio, M. A. Dionne, D. A. Compton, Mitotic spindle poles are organized by structural and motor proteins in addition to centrosomes. J Cell Biol 138, 1055-1066 (1997).
9. A. D. Silk, A. J. Holland, D. W. Cleveland, Requirements for NuMA in maintenance and establishment of mammalian spindle poles. J Cell Biol 184, 677-690 (2009).
10. P. T. Conduit, A. Wainman, J. W. Raff, Centrosome function and assembly in animal cells. Nat Rev Mol Cell Biol 16, 611-624 (2015).
11. P. Meraldi, Centrosomes in spindle organization and chromosome segregation: a mechanistic view. Chromosome Res 24, 19-34 (2016).
12. H. Maiato, E. Logarinho, Mitotic spindle multipolarity without centrosome amplification. Nature Cell Biology 16, 386-U323 (2014).
13. D. Szollosi, P. Calarco, R. P. Donahue, Absence of centrioles in the first and second meiotic spindles of mouse oocytes. J Cell Sci 11, 521-541 (1972).
14. A. H. Sathananthan, Ultrastructural changes during meiotic maturation in mammalian oocytes: unique aspects of the human oocyte. Microsc Res Tech 27, 145-164 (1994).
15. G. Manandhar, H. Schatten, P. Sutovsky, Centrosome reduction during gametogenesis and its significance. Biol Reprod 72, 2-13 (2005).
16. R. Heald et al., Self-organization of microtubules into bipolar spindles around artificial chromosomes in Xenopus egg extracts. Nature 382, 420-425 (1996).
17. R. Heald, R. Tournebize, A. Habermann, E. Karsenti, A. Hyman, Spindle assembly in Xenopus egg extracts: respective roles of centrosomes and microtubule self-organization. J Cell Biol 138, 615-628 (1997).
18. K. P. McNally, F. J. McNally, The spindle assembly function of Caenorhabditis elegans katanin does not require microtubule-severing activity. Mol Biol Cell 22, 1550-1560 (2011).
19. M. van der Voet et al., NuMA-related LIN-5, ASPM-1, calmodulin and dynein promote meiotic spindle rotation independently of cortical LIN-5/GPR/Galpha. Nat Cell Biol 11, 269-277 (2009).
20. A. A. Connolly et al., Caenorhabditis elegans oocyte meiotic spindle pole assembly requires microtubule severing and the calponin homology domain protein ASPM-1. Mol Biol Cell 25, 1298-1311 (2014).
21. J. X. Yu, Z. Guan, H. A. Nash, The mushroom body defect gene product is an essential component of the meiosis II spindle apparatus in Drosophila oocytes. Genetics 173, 243-253 (2006).
22. S. J. Radford, A. M. M. Go, K. S. McKim, Cooperation Between Kinesin Motors Promotes Spindle Symmetry and Chromosome Organization in Oocytes. Genetics 205, 517-527 (2017).
23. M. Hatsumi, S. A. Endow, Mutants of the microtubule motor protein, nonclaret disjunctional, affect spindle structure and chromosome movement in meiosis and mitosis. J Cell Sci 101 ( Pt 3), 547-559 (1992).
24. H. J. Matthies, H. B. McDonald, L. S. Goldstein, W. E. Theurkauf, Anastral meiotic spindle morphogenesis: role of the non-claret disjunctional kinesin-like protein. J Cell Biol 134, 455-464 (1996).
25. M. Schuh, J. Ellenberg, Self-organization of MTOCs replaces centrosome function during acentrosomal spindle assembly in live mouse oocytes. Cell 130, 484-498 (2007).
26. C. So et al., A liquid-like spindle domain promotes acentrosomal spindle assembly in mammalian oocytes. Science 364, (2019).
27. X. Guo, S. Gao, Pins homolog LGN regulates meiotic spindle organization in mouse oocytes. Cell Res 19, 838-848 (2009).
28. A. Kolano, S. Brunet, A. D. Silk, D. W. Cleveland, M. H. Verlhac, Error-prone mammalian female meiosis from silencing the spindle assembly checkpoint without normal interkinetochore tension. Proc Natl Acad Sci U S A 109, E1858-1867 (2012).
29. D. Clift, M. Schuh, A three-step MTOC fragmentation mechanism facilitates bipolar spindle assembly in mouse oocytes. Nat Commun 6, 7217 (2015).
30. A. Z. Balboula et al., Haspin kinase regulates microtubule-organizing center clustering and stability through Aurora kinase C in mouse oocytes. J Cell Sci 129, 3648-3660 (2016).
31. Y. H. Kim, I. W. Lee, Y. J. Jo, N. H. Kim, S. Namgoong, Acentriolar microtubule organization centers and Ran-mediated microtubule formation pathways are both required in porcine oocytes. Mol Reprod Dev 86, 972-983 (2019).
32. J. Lee, T. Miyano, R. M. Moor, Spindle formation and dynamics of gamma-tubulin and nuclear mitotic apparatus protein distribution during meiosis in pig and mouse oocytes. Biol Reprod 62, 1184-1192 (2000).
33. M. R. Shin, N. H. Kim, Maternal gamma (gamma)-tubulin is involved in microtubule reorganization during bovine fertilization and parthenogenesis. Mol Reprod Dev 64, 438-445 (2003).
34. C. Alvarez Sedo, H. Schatten, C. M. Combelles, V. Y. Rawe, The nuclear mitotic apparatus (NuMA) protein: localization and dynamics in human oocytes, fertilization and early embryos. Mol Hum Reprod 17, 392-398 (2011).
35. X. Xu, X. Duan, C. Lu, G. Lin, G. Lu, Dynamic distribution of NuMA and microtubules in human fetal fibroblasts, developing oocytes and somatic cell nuclear transferred embryos. Hum Reprod 26, 1052-1060 (2011).
36. A. Dammermann, A. Desai, K. Oegema, The minus end in sight. Curr Biol 13, R614-624 (2003).
37. M. Martin, A. Akhmanova, Coming into Focus: Mechanisms of Microtubule Minus-End Organization. Trends Cell Biol 28, 574-588 (2018).
38. J. M. Kollman, A. Merdes, L. Mourey, D. A. Agard, Microtubule nucleation by gamma-tubulin complexes. Nat Rev Mol Cell Biol 12, 709-721 (2011).
39. S. Pfender et al., Live imaging RNAi screen reveals genes essential for meiosis in mammalian oocytes. Nature 524, 239-242 (2015).
40. D. Clift et al., A Method for the Acute and Rapid Degradation of Endogenous Proteins. Cell 171, 1692-1706 e1618 (2017).
41. B. R. Brinkley, J. Cartwright, Jr., Cold-labile and cold-stable microtubules in the mitotic spindle of mammalian cells. Ann N Y Acad Sci 253, 428-439 (1975).
42. J. G. DeLuca, B. Moree, J. M. Hickey, J. V. Kilmartin, E. D. Salmon, hNuf2 inhibition blocks stable kinetochore-microtubule attachment and induces mitotic cell death in HeLa cells. J Cell Biol 159, 549-555 (2002).
43. L. Bury et al., Plk4 and Aurora A cooperate in the initiation of acentriolar spindle assembly in mammalian oocytes. J Cell Biol 216, 3571-3590 (2017).
44. C. S. Blengini et al., Aurora kinase A is essential for meiosis in mouse oocytes. bioRxiv, 2021.2001.2008.425851 (2021).
45. A. N. Kettenbach et al., Quantitative phosphoproteomics identifies substrates and functional modules of Aurora and Polo-like kinase activities in mitotic cells. Sci Signal 4, rs5 (2011).
46. S. Gallini et al., NuMA Phosphorylation by Aurora-A Orchestrates Spindle Orientation. Curr Biol 26, 458-469 (2016).
47. W. Ma, M. M. Viveiros, Depletion of pericentrin in mouse oocytes disrupts microtubule organizing center function and meiotic spindle organization. Mol Reprod Dev 81, 1019-1029 (2014).
48. C. Baumann, X. Wang, L. Yang, M. M. Viveiros, Error-prone meiotic division and subfertility in mice with oocyte-conditional knockdown of pericentrin. J Cell Sci 130, 1251-1262 (2017).
49. P. Kalab, R. Heald, The RanGTP gradient - a GPS for the mitotic spindle. J Cell Sci 121, 1577-1586 (2008).
50. T. Gaglio, A. Saredi, D. A. Compton, Numa Is Required for the Organization of Microtubules into Aster-Like Mitotic Arrays. J Cell Biol 131, 693-708 (1995).
51. A. Saredi, L. Howard, D. A. Compton, Phosphorylation regulates the assembly of NuMA in a mammalian mitotic extract. J Cell Sci 110 ( Pt 11), 1287-1297 (1997).
52. J. Harborth, J. Wang, C. Gueth-Hallonet, K. Weber, M. Osborn, Self assembly of NuMA: multiarm oligomers as structural units of a nuclear lattice. EMBOJ 18, 1689-1700 (1999).
53. N. Lecland, J. Luders, The dynamics of microtubule minus ends in the human mitotic spindle. Nat Cell Biol 16, 770-778 (2014).
54. M. W. Elting, M. Prakash, D. B. Udy, S. Dumont, Mapping Load-Bearing in the Mammalian Spindle Reveals Local Kinetochore Fiber Anchorage that Provides Mechanical Isolation and Redundancy. Curr Biol 27, 2112-2122 e2115 (2017).
55. P. Risteski, M. Jagrić, I. M. Tolić, Sliding of kinetochore fibers along bridging fibers helps center the chromosomes on the spindle. bioRxiv, 2020.2012.2030.424837 (2021).
56. M. Kallajoki, K. Weber, M. Osborn, A 210 Kda Nuclear Matrix Protein Is a Functional Part of the Mitotic Spindle - a Microinjection Study Using Spn Monoclonal-Antibodies. EMBO J 10, 3351-3362 (1991).
57. T. Maekawa, R. Leslie, R. Kuriyama, Identification of a minus end-specific microtubule-associated protein located at the mitotic poles in cultured mammalian cells. Eur J Cell Biol 54, 255-267 (1991).
58. M. A. Dionne, L. Howard, D. A. Compton, NuMA is a component of an insoluble matrix at mitotic spindle poles. Cell Motility 42, 189-203 (1999).
59. T. Chinen et al., NuMA assemblies organize microtubule asters to establish spindle bipolarity in acentrosomal human cells. EMBO J 39, e102378 (2020).
60. A. Merdes, K. Ramyar, J. D. Vechio, D. W. Cleveland, A complex of NuMA and cytoplasmic dynein is essential for mitotic spindle assembly. Cell 87, 447-458 (1996).
61. A. Merdes, R. Heald, K. Samejima, W. C. Earnshaw, D. W. Cleveland, Formation of spindle poles by dynein/dynactin-dependent transport of NuMA. J Cell Biol 149, 851-862 (2000).
62. T. Gaglio et al., Opposing motor activities are required for the organization of the mammalian mitotic spindle pole. J Cell Biol 135, 399-414 (1996).
63. C. L. Hueschen, S. J. Kenny, K. Xu, S. Dumont, NuMA recruits dynein activity to microtubule minus-ends at mitosis. Elife 6, (2017).
64. M. W. Elting, C. L. Hueschen, D. B. Udy, S. Dumont, Force on spindle microtubule minus ends moves chromosomes. J Cell Biol 206, 245-256 (2014).
65. N. Taulet et al., IFT88 controls NuMA enrichment at k-fibers minus-ends to facilitate their re-anchoring into mitotic spindles. Sci Rep 9, 10311 (2019).
66. S. Kotak, C. Busso, P. Gonczy, Cortical dynein is critical for proper spindle positioning in human cells. J Cell Biol 199, 97-110 (2012).
67. M. Okumura, T. Natsume, M. T. Kanemaki, T. Kiyomitsu, Dynein-Dynactin-NuMA clusters generate cortical spindle-pulling forces as a multi-arm ensemble. Elife 7, (2018).
68. L. Haren, A. Merdes, Direct binding of NuMA to tubulin is mediated by a novel sequence motif in the tail domain that bundles and stabilizes microtubules. J Cell Sci 115, 1815-1824 (2002).
69. L. Seldin, A. Muroyama, T. Lechler, NuMA-microtubule interactions are critical for spindle orientation and the morphogenesis of diverse epidermal structures. Elife 5, (2016).
70. N. J. Quintyne, T. A. Schroer, Distinct cell cycle-dependent roles for dynactin and dynein at centrosomes. J Cell Biol 159, 245-254 (2002).
71. J. T. Canty, A. Yildiz, Activation and Regulation of Cytoplasmic Dynein. Trends Biochem Sci 45, 440-453 (2020).
72. R. Gassmann et al., Removal of Spindly from microtubule-attached kinetochores controls spindle checkpoint silencing in human cells. Genes Dev 24, 957-971 (2010).
73. R. G. van Heesbeen, M. E. Tanenbaum, R. H. Medema, Balanced activity of three mitotic motors is required for bipolar spindle assembly and chromosome segregation. Cell Rep 8, 948-956 (2014).
74. S. Watanabe, G. Shioi, Y. Furuta, G. Goshima, Intra-spindle Microtubule Assembly Regulates Clustering of Microtubule-Organizing Centers during Early Mouse Development. Cell Rep 15, 54-60 (2016).
75. M. Jagrić, P. Risteski, J. Martinčić, A. Milas, I. M. Tolić, Optogenetic control of PRC1 reveals that bridging fibers promote chromosome alignment by overlap length-dependent forces. bioRxiv, 865394 (2020).
76. K.-Y. Lee, T. Davies, M. Mishima, Cytokinesis microtubule organisers at a glance. J Cell Sci 125, 3495-3500 (2012).
77. B. Wang, M. J. Pfeiffer, H. C. Drexler, G. Fuellen, M. Boiani, Proteomic Analysis of Mouse Oocytes Identifies PRMT7 as a Reprogramming Factor that Replaces SOX2 in the Induction of Pluripotent Stem Cells. J Proteome Res 15, 2407-2421 (2016).
78. I. Virant-Klun, S. Leicht, C. Hughes, J. Krijgsveld, Identification of Maturation-Specific Proteins by Single-Cell Proteomics of Human Oocytes. Mol Cell Proteomics 15, 2616-2627 (2016).
79. Z. Xue et al., Genetic programs in human and mouse early embryos revealed by single-cell RNA sequencing. Nature 500, 593-597 (2013).
80. L. Leng et al., Single-Cell Transcriptome Analysis of Uniparental Embryos Reveals Parent-of-Origin Effects on Human Preimplantation Development. Cell Stem Cell 25, 697-712 e696 (2019).
81. A. Graf et al., Fine mapping of genome activation in bovine embryos by RNA sequencing. Proc Natl Acad Sci U S A 111, 4139-4144 (2014).
82. Q. Kong et al., Lineage specification and pluripotency revealed by transcriptome analysis from oocyte to blastocyst in pig. FASEB J 34, 691-705 (2020).
83. I. Bennabi et al., Shifting meiotic to mitotic spindle assembly in oocytes disrupts chromosome alignment. EMBO Rep 19, 368-381 (2018).
84. V. Mountain et al., The kinesin-related protein, HSET, opposes the activity of Eg5 and crosslinks microtubules in the mammalian mitotic spindle. J Cell Biol 147, 351-366 (1999).
85. Y. Cao et al., Microtubule Minus-End Binding Protein CAMSAP2 and Kinesin-14 Motor KIFC3 Control Dendritic Microtubule Organization. Curr Biol 30, 899-908 e896 (2020).
86. Z. Y. She, W. X. Yang, Molecular mechanisms of kinesin-14 motors in spindle assembly and chromosome segregation. J Cell Sci 130, 2097-2110 (2017).
87. S. Cai, L. N. Weaver, S. C. Ems-McClung, C. E. Walczak, Kinesin-14 family proteins HSET/XCTK2 control spindle length by cross-linking and sliding microtubules. Mol Biol Cell 20, 1348-1359 (2009).
88. C. E. Walczak, S. Verma, T. J. Mitchison, XCTK2: A kinesin-related protein that promotes mitotic spindle assembly in Xenopus laevis egg extracts. Journal of Cell Biology 136, 859-870 (1997).
89. T. J. Mullen, S. M. Wignall, Interplay between microtubule bundling and sorting factors ensures acentriolar spindle stability during C-elegans oocyte meiosis. PLoS Genet 13, (2017).
90. C. H. Chuang, A. J. Schlientz, J. Yang, B. Bowerman, Microtubule assembly and pole coalescence: early steps in C aenorhabditis elegans oocyte meiosis I spindle assembly. Biol Open 9, (2020).
91. C. E. Walczak, I. Vernos, T. J. Mitchison, E. Karsenti, R. Heald, A model for the proposed roles of different microtubule-based motor proteins in establishing spindle bipolarity. Curr Biol 8, 903-913 (1998).
92. S. Morales-Mulia, J. M. Scholey, Spindle pole organization in Drosophila S2 cells by dynein, abnormal spindle protein (Asp), and KLP10A. Mol Biol Cell 16, 3176-3186 (2005).
93. G. Goshima, F. Nedelec, R. D. Vale, Mechanisms for focusing mitotic spindle poles by minus end-directed motor proteins. J Cell Biol 171, 229-240 (2005).
94. J. Baumbach, Z. A. Novak, J. W. Raff, A. Wainman, Dissecting the function and assembly of acentriolar microtubule organizing centers in Drosophila cells in vivo. PLoS Genet 11, e1005261 (2015).
95. M. Kwon et al., Mechanisms to suppress multipolar divisions in cancer cells with extra centrosomes. Genes Dev 22, 2189-2203 (2008).
96. J. Kleylein-Sohn et al., Acentrosomal spindle organization renders cancer cells dependent on the kinesin HSET. J Cell Sci 125, 5391-5402 (2012).
97. N. Kim, K. Song, KIFC1 Is Essential for Bipolar Spindle Formation and Genomic Stability in the Primary Human Fibroblast IMR-90 Cell. Cell Struct Funct 38, 21-30 (2013).
98. P. L. Chavali et al., A CEP215-HSET complex links centrosomes with spindle poles and drives centrosome clustering in cancer. Nat Commun 7, 11005 (2016).
99. B. Vitre et al., IFT proteins interact with HSET to promote supernumerary centrosome clustering in mitosis. EMBO Rep 21, e49234 (2020).
100. E. A. Nigg, Centrosome aberrations: cause or consequence of cancer progression? Nat Rev Cancer 2, 815-825 (2002).
101. J. Fu, I. M. Hagan, D. M. Glover, The centrosome and its duplication cycle. Cold Spring Harb Perspect Biol 7, a015800 (2015).
102. A. H. Sathananthan et al., inheritance of sperm centrioles and centrosomes in bovine embryos. Arch Androl 38, 37-48 (1997).
103. A. H. Sathananthan et al., Centriolar dynamics in the bovine embryo: inheritance and perpetuation of the sperm centrosome during fertilization and development. Protoplasma 206, 263-269 (1999).
104. E. L. Fishman et al., A novel atypical sperm centriole is functional during human fertilization. Nature Communications 9, (2018).
105. C. R. Simerly et al., Fertilization and Cleavage Axes Differ In Primates Conceived By Conventional (IVF) Versus Intracytoplasmic Sperm Injection (ICSI). Sci Rep 9, 15282 (2019).
106. J. Roeles, G. Tsiavaliaris, Actin-microtubule interplay coordinates spindle assembly in human oocytes. Nat Commun 10, 4651 (2019).
107. T. Cavazza et al., Parental genome unification is highly erroneous in mammalian embryos. bioRxiv, 2020.2008.2027.269779 (2020).
108. I. Schneider, M. de Ruijter-Villani, M. J. Hossain, T. A. E. Stout, J. Ellenberg, Non-rodent mammalian zygotes assemble dual spindles despite the presence of paternal centrosomes. bioRxiv, 2020.2010.2016.342154 (2020).
109. A. H. Sathananthan et al., Centrioles in the beginning of human development. Proc Natl Acad Sci U S A 88, 4806-4810 (1991).
110. A. H. Sathanathan et al., The sperm centriole: Its inheritance, replication and perpetuation in early human embryos. Human Reproduction 11, 345-356 (1996).
111. C. Simerly et al., Biparental inheritance of gamma-tubulin during human fertilization: molecular reconstitution of functional zygotic centrosomes in inseminated human oocytes and in cell-free extracts nucleated by human sperm. Mol Biol Cell 10, 2955-2969 (1999).
112. Y. Kai, K. Iwata, Y. Iba, Y. Mio, Diagnosis of abnormal human fertilization status based on pronuclear origin and/or centrosome number. J Assist Reprod Genet 32, 1589-1595 (2015).
113. Y. Kai, H. Kawano, N. Yamashita, First mitotic spindle formation is led by sperm centrosome-dependent MTOCs in humans. Reproduction 161, V19-V22 (2021).
114. Takemoto T. Zygote Electroporation for CRISPR/Cas9 Delivery to Generate Genetically Modified Mice. Methods Mol Biol. (2020).
115. Mogessie B, Scheffler K, Schuh M. Assembly and Positioning of the Oocyte Meiotic Spindle. Annu Rev Cell Dev Biol. (2018).
116. Petry S. Mechanisms of Mitotic Spindle Assembly. Annu Rev Biochem. (2016)*.*
117. Prosser SL, Pelletier L. Mitotic spindle assembly in animal cells: a fine balancing act. Nat Rev Mol Cell Biol. (2017).
118. Hua, K. Jiang, Expression and Purification of Microtubule-Associated Proteins from HEK293T Cells for In Vitro Reconstitution. Methods Mol Biol 2101, 19-26 (2020)
119. Borensztein M, Syx L, Servant N, Heard E. Transcriptome Profiling of Single Mouse Oocytes. Methods Mol Biol. 2018;1818:51-65.
120. Carlton JG, Jones H, Eggert US. Membrane and organelle dynamics during cell division. Nat Rev Mol Cell Biol. 2020 Mar;21(3):151-166
121. Mogessie B, Schuh M. Actin protects mammalian eggs against chromosome segregation errors. Science. 2017 Aug 25;357(6353):eaal1647
122. Y. F. Gu et al., Abnormalities in centrosome number in human embryos and embryonic stem cells. Mol Reprod Dev 83, 392-404 (2016).

## Claims

1. An *in vitro* method for assisted reproductive technology comprising introducing (i) KIFC1/HSET protein or (ii) mRNA encoding KIFC1/HSET into a human oocyte.

2. The method of claim 1, wherein the KIFC1/HSET stabilizes the meiotic spindle of the human oocyte.

3. The method of claims 1 or 2, wherein the KIFC1/HSET protein is recombinantly produced KIFC1/HSET protein.

4. The method of any of the preceding claims, wherein the human oocyte is naturally occurring and/or wherein the human oocyte expresses the KIFC1/HSET at least 2-fold less than a mouse oocyte, preferably at least 4-fold less, more preferably at least 6-fold less, even more preferably at least 10-fold less, even more preferably at least 20-fold less, even more preferably at least 30-fold less, even more preferably at least 40-fold less, even more preferably at least 50-fold less, and even more preferably at least 60-fold less; and/or wherein the human oocyte is naturally occurring and/or wherein the human oocyte expresses the KIFC1/HSET at least 2-fold less than a HeLa cell, preferably at least 4-fold less, more preferably at least 6-fold less, even more preferably at least 10-fold less, and even more preferably at least 20-fold less.

5. The method of any of the preceding claims, wherein the human oocyte with introduced KIFC1/HSET has a higher probability of having a bipolar meiotic spindle compared to the human oocyte without introduced KIFC1/HSET, preferably wherein the human oocyte with introduced KIFC1/HSET has at least a 5% higher probability of having a bipolar meiotic spindle compared to the human oocyte without introduced KIFC1/HSET, preferably at least 10%, more preferably at least 15%, more preferably at least 20%, more preferably at least 25%, and even more preferably at least 35%.

6. The method of any of the preceding claims, wherein (i) the KIFC1/HSET protein or (ii) the mRNA encoding KIFC1/HSET is introduced during germinal vesicle stage, meiosis I or meiosis II.

## Patentansprüche

1. In vitro Verfahren für assistierte Reproduktionstechnologie, aufweisend das Einführen von (i) KIFC1/HSET-Protein oder (ii) mRNA, die für KIFC1/HSET kodiert, in eine menschliche Oozyte.

2. Das Verfahren nach Anspruch 1, wobei das KIFC1/HSET die meiotische Spindel der menschlichen Oozyte stabilisiert.

3. Das Verfahren nach Anspruch 1 oder 2, wobei das KIFC1/HSET-Protein rekombinant hergestelltes KIFC1/HSET-Protein ist.

4. Das Verfahren nach einem der vorhergehenden Ansprüche, wobei die menschliche Oozyte natürlich vorkommend ist und/oder wobei die menschliche Oozyte das KIFC1/HSET wenigstens 2-fach geringer exprimiert als eine Maus-Oozyte, bevorzugt wenigstens 4-fach geringer, stärker bevorzugt wenigstens 6-fach geringer, noch stärker bevorzugt wenigstens 10-fach geringer, noch stärker bevorzugt wenigstens 20-fach geringer, noch stärker bevorzugt wenigstens 30-fach geringer, noch stärker bevorzugt wenigstens 40-fach geringer, noch stärker bevorzugt wenigstens 50-fach geringer, und noch stärker bevorzugt wenigstens 60-fach geringer; und/oder
wobei die menschliche Oozyte natürlich vorkommend ist und/oder wobei die menschliche Oozyte das KIFC1/HSET wenigstens 2-fach geringer exprimiert als eine HeLa-Zelle, bevorzugt wenigstens 4-fach geringer, stärker bevorzugt wenigstens 6-fach geringer, noch stärker bevorzugt wenigstens 10-fach geringer, und noch stärker bevorzugt wenigstens 20-fach geringer.

5. Das Verfahren nach einem der vorhergehenden Ansprüche, wobei die menschliche Oozyte mit eingeführtem KIFC1/HSET eine höhere Wahrscheinlichkeit hat, eine bipolare meiotische Spindel zu haben, verglichen mit der menschlichen Oozyte ohne eingeführtes KIFC1/HSET, bevorzugt wobei die menschliche Oozyte mit eingeführtem KIFC1/HSET wenigstens eine 5% höhere Wahrscheinlichkeit hat, eine bipolare meiotische Spindel zu haben, verglichen mit der menschlichen Oozyte ohne eingeführtes KIFC1/HSET, bevorzugt wenigstens 10%, stärker bevorzugt wenigstens 15%, stärker bevorzugt wenigstens 20%, stärker bevorzugt wenigstens 25%, und noch stärker bevorzugt wenigstens 35%.

6. Das Verfahren nach einem der vorhergehenden Ansprüche, wobei (i) das KIFC1/HSET-Protein oder (ii) die mRNA, die für KIFC1/HSET kodiert, während des Germinalvesikelstadiums, der Meiose I oder der Meiose II eingeführt wird.

## Revendications

1. Méthode *in vitro* destinée à la technologie de reproduction assistée comprenant l'introduction (i) d'une protéine KIFC1/HSET ou (ii) de l'ARNm codant pour la KIFC1/HSET dans un ovocyte humain.

2. Méthode selon la revendication 1, dans laquelle la KIFC1/HSET stabilise le fuseau méiotique de l'ovocyte humain.

3. Méthode selon la revendication 1 ou 2, dans laquelle la protéine KIFC1/HSET est une protéine KIFC1/HSET produite par recombinaison.

4. Méthode selon l'une quelconque des revendications précédentes, dans laquelle l'ovocyte humain est d'origine naturelle et/ou dans laquelle l'ovocyte humain exprime la KIFC1/HSET au moins 2 fois moins qu'un ovocyte de souris, de préférence au moins 4 fois moins, plus préférablement au moins 6 fois moins, encore plus préférablement au moins 10 fois moins, encore plus préférablement au moins 20 fois moins, encore plus préférablement au moins 30 fois moins, encore plus préférablement au moins 40 fois moins, encore plus préférablement au moins 50 fois moins, et encore plus préférablement au moins 60 fois moins ; et/ou
dans laquelle l'ovocyte humain est d'origine naturelle et/ou dans laquelle l'ovocyte humain exprime la KIFC1/HSET au moins 2 fois moins qu'une cellule HeLa, de préférence au moins 4 fois moins, plus préférablement au moins 6 fois moins, encore plus préférablement au moins 10 fois moins, et encore plus préférablement au moins 20 fois moins.

5. Méthode selon l'une quelconque des revendications précédentes, dans laquelle l'ovocyte humain ayant la KIFC1/HSET introduite présente une probabilité plus élevée d'avoir un fuseau méiotique bipolaire par rapport à l'ovocyte humain sans la KIFC1/HSET introduite, de préférence dans laquelle l'ovocyte humain ayant la KIFC1/HSET introduite présente une probabilité plus élevée d'au moins 5 % d'avoir un fuseau méiotique bipolaire par rapport à l'ovocyte humain sans la KIFC1/HSET introduite, de préférence d'au moins 10 %, plus préférablement d'au moins 15 %, plus préférablement d'au moins 20 %, plus préférablement d'au moins 25 %, et encore plus préférablement d'au moins 35 %.

6. Méthode selon l'une quelconque des revendications précédentes, dans laquelle (i) la protéine KIFC1/HSET ou (ii) l'ARNm codant pour la KIFC1/HSET est introduit au stade de la vésicule germinale, de la méiose I ou de la méiose II.
